# EUROPÄISCHE PATENTSCHRIFT

(11) **EP 1 411 932 B1**
(45) Veröffentlichungstag und Bekanntmachung des Hinweises auf die Patenterteilung: **10.02.2010**
(21) Anmeldenummer: 02738154.0
(22) Anmeldetag: 07.06.2002
(51) Int. Cl.: A61K 31/422, A61K 31/435

(54) **KOMBINATIONSTHERAPIE SUBSTITUIERTER OXAZOLIDINONE**
SUBSTITUTED OXAZOLIDINONES FOR COMBINATIONAL THERAPY
COMBINAISONS THERAPEUTIQUES D'OXAZOLIDINONES SUBSTITUEES

(30) Priorität: 20.06.2001 DE 10129725
(43) Veröffentlichungstag der Anmeldung: 28.04.2004
(73) Patentinhaber: Bayer Schering Pharma Aktiengesellschaft, 13353 Berlin (DE)
(72) Erfinder: STRAUB, Alexander, 42117 Wuppertal (DE); LAMPE, Thomas, 40223 Düsseldorf (DE); PERNERSTORFER, Josef, 42103 Wuppertal (DE); PERZBORN, Elisabeth, 42327 Wuppertal (DE); POHLMANN, Jens, 42285 Wuppertal (DE); RÖHRIG, Susanne, 45276 Essen (DE); SCHLEMMER, Karl-Heinz, 42113 Wuppertal (DE)
(86) Internationale Anmeldenummer: PCT/EP2002/006237
(87) Internationale Veröffentlichungsnummer: WO 2003/000256

(56) Entgegenhaltungen:
- EP-A- 0 930 076
- WO-A-01/47919
- WO-A-99/31092
- DE-A- 10 105 989
- US-A- 5 532 255
- US-A- 6 159 997

## Beschreibung

Die vorliegende Erfindung betrifft Kombinationen von A) Oxazolidinonen der Formel (I) mit B) anderen Wirkstoffen, ein Verfahren zur Herstellung dieser Kombinationen und ihre Verwendung als Arzneimittel, insbesondere zur Prophylaxe und/oder Behandlung von thromboembolischen Erkrankungen.

Oxazolidinone der Formel (I) wirken insbesondere als selektive Inhibitoren des Blutgerinnungsfaktors Xa und als Antikoagulantien.

Eine antithrombotische Wirkung von Faktor Xa-Inhibitoren konnte in zahlreichen Tiermodellen (vgl. WO 99/37304; WO 99/06371; J. Hauptmann, J. Stürzebecher, Thrombosis Research 1999, 93, 203; F. Al-Obeidi, J. A. Ostrem, Factor Xa inhibitors, Exp. Opin. Ther. Patents 1999, 9, 931; B.-Y. Zhu, R. M. Scarborough, Curr. Opin. Card. Pulm. Ren. Inv. Drugs 1999, 1 (1), 63, M. Samama, J. M. Walenga, B. Kaiser, J. Fareed, Specific Factor Xa Inhibitors, Cardiovascular Thrombosis: Thrombocardiology and Thromboneurology, Second Edition, edited by M. Verstraete, V. Fuster, E. J. Topol, Lippincott-Raven Publishers, Philadelphia 1998) sowie in klinischen Studien an Patienten (The Ephesus Study, blood, Vol 96, 490a, 2000; The Penthifra Study, blood, Vol 96, 490a, 2000; The Pentamaks Study, blood, Vol 96, 490a-491a, 2000; The Pentathlon 2000 Study, blood, Vol 96, 491a, 2000) nachgewiesen werden. Faktor Xa-Inhibitoren können deshalb bevorzugt eingesetzt werden in Arzneimitteln zur Prophylaxe und/oder Behandlung von thromboembolischen Erkrankungen.

Thromboembolische Gefäßerkrankungen sind die häufigste Ursache von Morbidität und Mortalität in den industrialisierten Ländern (Thiemes Innere Medizin, Georg Thieme Verlag Stuttgart, New York; American Heart Association, 2000 heart and stroke statistical update, Dallas, TX: American Heart Association, 2000). Die antikoagulatorische Therapie hat sich bei der Behandlung von Gefäßerkrankungen, um thrombotische Gefäßverschlüsse zu vermeiden bzw. um thrombotisch verschlossene Gefäße wieder zu eröffnen, bewährt und nimmt einen hohen Stellenwert bei der Prophylaxe und Behandlung von koronaren, peripheren und cerebralen Gefäßerkrankungen ein, sowie bei der Prophylaxe und/oder Behandlung von Venenthrombosen und Lungenembolien.

Ursache für thromboembolische Komplikationen können atherosklerotische Veränderungen der Gefäßwand sein, insbesondere Störungen der Endothelfunktion, die zu akuten thrombotischen Verschlüssen führen können. Die Atherosklerose ist eine multifaktorielle Erkrankung, die von einer Vielzahl kardiovaskulärer Risikofaktoren abhängig ist. Klinische Studien haben gezeigt, dass eine Prophylaxe mit Antikoagulantien den Verlauf der arteriellen Gefäßerkrankung nicht entscheidend beeinflusst. Eine gezielte Behandlung der Risikofaktoren in Verbund mit einer antithrombotischen Therapie ist daher vorteilhaft.

Risikofaktoren für koronare, periphere und cerebrale Gefäßerkrankungen sind beispielsweise: Erhöhte Serumcholesterinspiegel, arterielle Hypertonie, Zigarettenrauchen, Diabetes mellitus (Allgemeine und spezielle Pharmakologie und Toxikologie, W. Forth, D. Henschler, W. Rummel, K. Starke; Spektrum Akademischer Verlag Heidelberg Berlin Oxford; Thiemes Innere Medizin, Georg Thieme Verlag Stuttgart, New York). Präventivmedizinische Prinzipien basieren auf dem Ausschalten dieser Risikofaktoren. Neben der Änderung von Lebensgewohnheiten gehören dazu auch pharmakologische Maßnahmen wie beispielsweise eine antihypertensive Therapie, lipidsenkende Arzneimittel oder Thromboseprophylaxe. Darüber hinaus ist zur Behandlung bei einer bereits bestehenden koronaren Herzerkrankung die Kombination mit Koronartherapeutika geeignet.

Es wurde nun überraschenderweise gefunden, dass Kombinationen von Oxazolidinonen der Formel (I) mit bestimmten anderen Wirkstoffen interessante Eigenschaften besitzen und für die Prophylaxe und/oder Behandlung verschiedener Krankheiten besser geeignet sind als die Einzelwirkstoffe alleine.

Gegenstand der Erfindung sind daher Kombinationen von
A) Oxazolidinonen der Formel (I) mit
B) anderen Wirkstoffen, insbesondere mit Plättchenaggregationshemmern, Antikoagulantien, Fibrinolytika, Lipidsenkern, Koronartherapeutika und/oder Vasodilatatoren.

Unter "Kombinationen" im Sinne der Erfindung werden nicht nur Darreichungsformen, die alle Komponenten enthalten (sog. Fixkombinationen), und Kombinationspackungen, die die Komponenten voneinander getrennt enthalten, verstanden, sondern auch gleichzeitig oder zeitlich versetzt applizierte Komponenten, sofern sie zur Prophylaxe und/oder Behandlung derselben Krankheit eingesetzt werden. Ebenso ist es möglich, zwei oder mehr Wirkstoffe miteinander zu kombinieren, es handelt sich dabei also jeweils um zwei- oder mehrfach-Kombinationen.

Geeignete Oxazolidinone der erfindungsgemäßen Kombination umfassen beispielsweise Verbindungen der Formel (I) 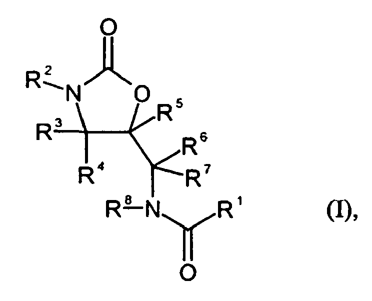 in welcher:
- R¹: für gegebenenfalls benzokondensiertes Thiophen (Thienyl) steht, das gegebe- nenfalls ein- oder mehrfach substituiert sein kann;
- R²: für einen beliebigen organischen Rest steht;
- R³, R⁴, R⁵, R⁶, R⁷ und R⁸: gleich oder verschieden sind und für Wasserstoff oder für (C₁-C₆)-Alkyl stehen
sowie deren pharmazeutisch verträglichen Salze, Hydrate und Prodrugs.

Bevorzugt sind hierbei Verbindungen der Formel (I),
worin
- R¹: für gegebenenfalls benzokondensiertes Thiophen (Thienyl) steht, das gegebe- nenfalls ein- oder mehrfach substituiert sein kann durch einen Rest aus der Gruppe von Halogen; Cyano; Nitro; Amino; Aminomethyl; (C₁-C₈)-Alkyl, das gegebenenfalls seinerseits ein- oder mehrfach durch Halogen substituiert sein kann; (C₃-C₇)-Cycloalkyl; (C₁-C₈)-Alkoxy; Imidazolinyl; -C(=NH)NH₂; Carbamoyl; und Mono- und Di-(C₁-C₄)-alkyl-aminocarbonyl,
- R²: für eine der folgenden Gruppen steht:
A-,
A-M-,
D-M-A-,
B-M-A-,
B-,
B-M-,
B-M-B-,
D-M-B-,
wobei:
der Rest "A" für (C₆-C₁₄)-Aryl, vorzugsweise für (C₆-C₁₀)-Aryl, insbesondere für Phenyl oder Naphthyl, ganz besonders bevorzugt für Phenyl, steht;
der Rest "B" für einen 5- oder 6-gliedrigen aromatischen Heterocyclus steht, der bis zu 3 Heteroatome und/oder Hetero-Kettenglieder, insbesondere bis zu 2 Heteroatome und/oder Hetero-Kettenglieder, aus der Reihe S, N, NO (N-Oxid) und O enthält;
der Rest "D" für einen gesättigten oder teilweise ungesättigten, mono- oder bicyclischen, gegebenenfalls benzokondensierten 4- bis 9-gliedrigen Heterocyclus steht, der bis zu drei Heteroatome und/oder Hetero-Kettenglieder aus der Reihe S, SO, SO₂ N, NO (N-Oxid) und O enthält;
der Rest "M" für -NH-, -CH₂-, -CH₂CH₂-, -O-, -NH-CH₂-, -CH₂-NH-, -OCH₂-, -CH₂O- -CONH-, -NHCO-, -COO-, -OOC-, -S- , -SO₂ oder für eine kovalente Bindung steht;
wobei
die zuvor definierten Gruppen "A", "B" und "D" jeweils gegebenenfalls ein-oder mehrfach substituiert sein können mit einem Rest aus der Gruppe von Halogen; Trifluormethyl; Oxo; Cyano; Nitro; Carbamoyl; Pyridyl; (C₁-C₆)-Alkanoyl; (C₃-C₇)-Cycloalkanoyl; (C₆-C₁₄)-Arylcarbonyl; (C₅-C₁₀)-Heteroarylcarbonyl; (C₁-C₆)-Alkanoyloxymethyloxy; (C₁-C₄)-Hydroxyalkylcarbonyl; -COOR²⁷; -SO₂R²⁷; -C(NR²⁷R²⁸)=NR²⁹_{;} CONR²⁸R²⁹; -SO₂NR²⁸R²⁹; -OR³⁰; -NR³⁰R³¹, (C₁-C₆)-Alkyl und (C₃-C₇)-Cycloalkyl,
wobei (C₁-C₆)-Alkyl und (C₃-C₇)-Cycloalkyl ihrerseits gegebenenfalls substituiert sein können durch einen Rest aus der Gruppe von Cyano; -OR²⁷; -NR²⁸-R²⁹; -CO(NH)ᵥ(NR²⁷R²⁸) und -C(NR²⁷R²⁸)=NR²⁹,
wobei:
- v: entweder 0 oder 1 bedeutet und
- R²⁷, R²⁸ und R²⁹: gleich oder verschieden sind und unabhängig voneinander Wasserstoff, (C₁-C₄)-Alkyl, (C₃-C₇)-Cycloalkyl, (C₁-C₄)-Alkanoyl, Carbamoyl, Trifluormethyl, Phenyl oder Pyridyl bedeuten,
und/oder
- R²⁷ und R²⁸ bzw. R²⁷ und R²⁹: zusammen mit dem Stickstoffatom, an das sie gebunden sind, einen gesättigten oder teilweise ungesättigten 5- bis 7- gliedrigen Heterocyclus mit bis zu drei, vorzugsweise bis zu zwei gleichen oder unterschiedlichen Heteroatomen aus der Gruppe von N, O und S bilden, und
- R³⁰ und R³¹: gleich oder verschieden sind und unabhängig voneinander Wasserstoff, (C₁-C₄)-Alkyl, (C₃-C₇)-Cycloalkyl, (C₁-C₄)-Alkylsulfo- nyl, (C₁-C₄)-Hydroxyalkyl, (C₁-C₄)-Aminoalkyl, Di-(C₁-C₄)-alkyl- amino-(C₁-C₄)-alkyl, -CH₂C(NR²⁷R²⁸)=NR²⁹ oder -COR³³ bedeuten,
wobei
- R³³: (C₁-C₆)-Alkoxy, (C₁-C₄)-Alkoxy-(C₁-C₄)-alkyl, (C₁-C₄)- Alkoxycarbonyl-(C₁-C₄)-alkyl, (C₁-C₄)-Aminoalkyl, (C₁-C₄)- Alkoxycarbonyl, (C₁-C₄)-Alkanoyl-(C₁-C₄)-alkyl, (C₃-C₇)- Cycloalkyl, (C₂-C₆)-Alkenyl, (C₁-C₈)-Alkyl, das gegebenen- falls durch Phenyl oder Acetyl substituiert sein kann, (C₆-C₁₄)- Aryl, (C₅-C₁₀)-Heteroaryl, Trifluormethyl, Tetrahydrofuranyl oder Butyrolacton bedeutet,
- R³, R⁴, R⁵, R⁶, R⁷ und R⁸: gleich oder verschieden sind und für Wasserstoff oder für (C₁-C₆)-Alkyl stehen
und deren pharmazeutisch verträglichen Salze, Hydrate und Prodrugs.

Ebenfalls bevorzugt sind hierbei Verbindungen der allgemeinen Formel (I),
worin
- R¹: für Thiophen (Thienyl), insbesondere 2-Thiophen, steht, das gegebenenfalls ein- oder mehrfach substituiert sein kann durch Halogen, vorzugsweise Chlor oder Brom, Amino, Aminomethyl oder (C₁-C₈)-Alkyl, vorzugsweise Methyl, wobei der (C₁-C₈)-Alkylrest gegebenenfalls seinerseits ein- oder mehrfach durch Halogen, vorzugsweise Fluor, substituiert sein kann,
- R²: für eine der folgenden Gruppen steht:
A-,
A-M-,
D-M-A-,
B-M-A-,
B-,
B-M-,
B-M-B-,
D-M-B-,
wobei:
der Rest "A" für (C₆-C₁₄)-Aryl, vorzugsweise für (C₆-C₁₀)-Aryl, insbesondere für Phenyl oder Naphthyl, ganz besonders bevorzugt für Phenyl, steht;
der Rest "B" für einen 5- oder 6-gliedrigen aromatischen Heterocyclus steht, der bis zu 3 Heteroatome und/oder Hetero-Kettenglieder, insbesondere bis zu 2 Heteroatome und/oder Hetero-Kettenglieder, aus der Reihe S, N, NO (N-Oxid) und O enthält;
der Rest "D" für einen gesättigten oder teilweise ungesättigten 4- bis 7-gliedrigen Heterocyclus steht, der bis zu drei Heteroatome und/oder Hetero-Kettenglieder aus der Reihe S, SO, SO₂ N, NO (N-Oxid) und O enthält;
der Rest "M" für -NH-, -CH₂-, -CH₂CH₂-, -O-, -NH-CH₂-, -CH₂-NH-, -OCH₂-, -CH₂O-, -CONH-, -NHCO-, -COO-, -OOC-, -S- oder für eine kovalente Bindung steht;
wobei
die zuvor definierten Gruppen "A", "B" und "D" jeweils gegebenenfalls ein-oder mehrfach substituiert sein können mit einem Rest aus der Gruppe von Halogen; Trifluormethyl; Oxo; Cyano; Nitro; Carbamoyl; Pyridyl; (C₁-C₆)-Alkanoyl; (C₃-C₇)-Cycloalkanoyl; (C₆-C₁₄)-Arylcarbonyl; (C₅-C₁₀)-Heteroarylcarbonyl; (C₁-C₆)-Alkanoyloxymethyloxy; -COOR²⁷; -SO₂R²⁷; -C(NR²⁷R²⁸)=NR²⁹; -CONR²⁸R²⁹; -SO₂NR²⁸R²⁹; -OR³⁰; -NR³⁰R³¹, (C₁-C₆)-Alkyl und (C₃-C₇)-Cycloalkyl,
wobei (C₁-C₆)-Alkyl und (C₃-C₇)-Cycloalkyl ihrerseits gegebenenfalls substituiert sein können durch einen Rest aus der Gruppe von Cyano; -OR²⁷; -NR²⁸R²⁹; -CO(NH)ᵥ(NR²⁷R²⁸) und -C(NR²⁷R²⁸)=NR²⁹,
wobei:
- v: entweder 0 oder 1 bedeutet und
- R²⁷, R²⁸ und R²⁹: gleich oder verschieden sind und unabhängig voneinander Wasserstoff, (C₁-C₄)-Alkyl oder (C₃-C₇)-Cycloalkyl bedeuten,
und/oder
- R²⁷ und R²⁸ bzw. R²⁷ und R²⁹: zusammen mit dem Stickstoffatom, an das sie gebunden sind, einen gesättigten oder teilweise ungesättigten 5- bis 7- gliedrigen Heterocyclus mit bis zu drei, vorzugsweise bis zu zwei gleichen oder unterschiedlichen Heteroatomen aus der Gruppe von N, O und S bilden, und
- R³⁰ und R³¹: gleich oder verschieden sind und unabhängig voneinander Wasserstoff, (C₁-C₄)-Alkyl, (C₃-C₇)-Cycloalkyl, (C₁-C₄)-Akyl- sulfonyl, (C₁-C₄)-Hydroxyalkyl, (C₁-C₄)-Aminoalkyl, Di-(C₁-C₄)- alkylamino-(C₁-C₄)-alkyl, (C₁-C₄)-Alkanoyl, (C₆-C₁₄)-Arylcarbonyl, (C₅-C₁₀)-Heteroarylcarbonyl, (C₁-C₄)-Alkylaminocarbonyl oder -CH₂C(NR²⁷R²⁸)=NR²⁹ bedeuten,
- R³, R⁴, R⁵, R⁶,: R⁷ und R⁸ gleich oder verschieden sind und für Wasserstoff oder für (C₁-C₆)-Alkyl stehen
und deren pharmazeutisch verträglichen Salze, Hydrate und Prodrugs.

Besonders bevorzugt sind hierbei Verbindungen der allgemeinen Formel (I),
worin
- R¹: für Thiophen (Thienyl), insbesondere 2-Thiophen, steht, das gegebenenfalls ein- oder mehrfach substituiert sein kann durch Halogen, vorzugsweise Chlor oder Brom, oder (C₁-C₈)-Alkyl, vorzugsweise Methyl, wobei der (C₁-C₈)- Alkylrest gegebenenfalls seinerseits ein- oder mehrfach durch Halogen, vor- zugsweise Fluor, substituiert sein kann,
- R²: für eine der folgenden Gruppen steht:
A-,
A-M-,
D-M-A-,
B-M-A-,
B-,
B-M-,
B-M-B-,
D-M-B-,
wobei:
der Rest "A" für Phenyl oder Naphthyl, insbesondere für Phenyl, steht;
der Rest "B" für einen 5- oder 6-gliedrigen aromatischen Heterocyclus steht, der bis zu 2 Heteroatome aus der Reihe S, N, NO (N-Oxid) und O enthält; der Rest "D" für einen gesättigten oder teilweise ungesättigten 5- oder 6-gliedrigen Heterocyclus steht, der bis zu zwei Heteroatome und/oder Hetero-Kettenglieder aus der Reihe S, SO, SO₂ N, NO (N-Oxid) und O enthält;
der Rest "M" für -NH-, -O-, -NH-CH₂-, -CH₂-NH-, -OCH₂-, -CH₂O--CONH-, -NHCO- oder für eine kovalente Bindung steht;
wobei
die zuvor definierten Gruppen "A", "B" und "D" jeweils gegebenenfalls ein-oder mehrfach substituiert sein können mit einem Rest aus der Gruppe von Halogen; Trifluormethyl; Oxo; Cyano; Pyridyl; (C₁₋C₃)-Alkanoyl; (C₆-C₁₀)-Arylcarbonyl; (C₅-C₆)-Heteroarylcarbonyl; (C₁-C₃)-Alkanoyloxymethyloxy; -C(NR²⁷R²⁸)=NR²⁹; -CONR²⁸R²⁹; -SO₂NR²⁸R²⁹; -OH; -NR³⁰R³¹; (C₁-C₄)-Alkyl; und Cyclopropyl, Cyclopentyl oder Cyclohexyl,
wobei (C₁-C₄)-Alkyl und Cyclopropyl, Cyclopentyl oder Cyclohexyl ihrerseits gegebenenfalls substituiert sein können durch einen Rest aus der Gruppe von Cyano; -OH; -OCH₃; -NR²⁸R²⁹; -CO(NH)ᵥ(NR²⁷R²⁸) und -C(NR²⁷R²⁸)=NR²⁹,
wobei:
- v: entweder 0 oder 1, vorzugsweise 0, bedeutet und
- R²⁷, R²⁸ und R²⁹: gleich oder verschieden sind und unabhängig voneinander Wasserstoff, (C₁-C₄)-Alkyl oder aber Cyclopropyl, Cyclopentyl oder Cyclohexyl bedeuten und/oder
- R²⁷ und R²⁸ bzw. R²⁷ und R²⁹: zusammen mit dem Stickstoffatom, an das sie gebunden sind, einen gesättigten oder teilweise ungesättigten 5- bis 7- gliedrigen Heterocyclus mit bis zu zwei gleichen oder unterschied- lichen Heteroatomen aus der Gruppe von N, O und S bilden können, und
- R³⁰ und R³¹: gleich oder verschieden sind und unabhängig voneinander Wasserstoff, (C₁-C₄)-Alkyl, Cyclopropyl, Cyclopentyl, Cyclohexyl, (C₁-C₄)-Alkylsulfonyl, (C₁-C₄)-Hydroxyalkyl, (C₁-C₄)-Aminoalkyl, Di-(C₁-C₄)-alkylamino-(C₁-C₄)-alkyl, (C₁-C₃)-Alkanoyl oder Phenyl- carbonyl bedeuten,
- R³, R⁴, R⁵, R⁶, R⁷ und R⁸: gleich oder verschieden sind und für Wasserstoff oder für (C₁-C₆)-Alkyl stehen
und deren pharmazeutisch verträglichen Salze, Hydrate und Prodrugs.

Insbesondere bevorzugt sind hierbei Verbindungen der allgemeinen Formel (I),
worin
- R¹: für 2-Thiophen, steht, das gegebenenfalls in der 5-Position substituiert sein kann durch einen Rest aus der Gruppe Chlor, Brom, Methyl oder Trifluor- methyl,
- R²: für eine der folgenden Gruppen steht:
A-,
A-M-,
D-M-A-,
B-M-A-,
B-,
B-M-,
B-M-B-,
D-M-B-,
wobei:
der Rest "A" für Phenyl oder Naphthyl, insbesondere für Phenyl, steht;
der Rest "B" für einen 5- oder 6-gliedrigen aromatischen Heterocyclus steht, der bis zu 2 Heteroatome aus der Reihe S, N, NO (N-Oxid) und O enthält;
der Rest "D" für einen gesättigten oder teilweise ungesättigten 5- oder 6-gliedrigen Heterocyclus steht, der ein Stickstoffatom und gegebenenfalls ein weiteres Heteroatom und/oder Hetero-Kettenglied aus der Reihe S, SO, SO₂ und O; oder bis zu zwei Heteroatome und/oder Hetero-Kettenglieder aus der Reihe S, SO, SO₂ und O enthält;
der Rest "M" für -NH-, -O-, -NH-CH₂-, -CH₂-NH-, -OCH₂-, -CH₂O-, -CONH-, -NHCO- oder für eine kovalente Bindung steht;
wobei
die zuvor definierten Gruppen "A", "B" und "D" jeweils gegebenenfalls ein-oder mehrfach substituiert sein können mit einem Rest aus der Gruppe von Halogen; Trifluormethyl; Oxo; Cyano; Pyridyl; (C₁-C₃)-Alkanoyl; (C₆-C₁₀)-Arylcarbonyl; (C₅-C₆)-Heteroarylcarbonyl; (C₁-C₃)-Alkanoyloxymethyloxy; -CONR²⁸R²⁹; -SO₂NR²⁸R²⁹; -OH; -NR³⁰R³¹; (C₁-C₄)-Alkyl; und Cyclopropyl, Cyclopentyl oder Cyclohexyl,
wobei (C₁-C₄)-Alkyl und Cyclopropyl, Cyclopentyl oder Cyclohexyl ihrerseits gegebenenfalls substituiert sein können durch einen Rest aus der Gruppe von Cyano; -OH; -OCH₃; -NR²⁸R²⁹; -CO(NH)ᵥ(NR²⁷R²⁸) und -C(NR²⁷R²⁸)=NR²⁹,
wobei:
- v: entweder 0 oder 1, vorzugsweise 0, bedeutet und
- R²⁷, R²⁸ und R²⁹: gleich oder verschieden sind und unabhängig voneinander Wasserstoff, (C₁-C₄)-Alkyl oder aber Cyclopropyl, Cyclopentyl oder Cyclohexyl bedeuten und/oder
- R²⁷ und R²⁸ bzw. R²⁷ und R²⁹: zusammen mit dem Stickstoffatom, an das sie gebunden sind, einen gesättigten oder teilweise ungesättigten 5- bis 7- gliedrigen Heterocyclus mit bis zu zwei gleichen oder unterschied- lichen Heteroatomen aus der Gruppe von N, O und S bilden können, und
- R³⁰ und R³¹: gleich oder verschieden sind und unabhängig voneinander Wasserstoff, (C₁-C₄)-Alkyl, Cyclopropyl, Cyclopentyl, Cyclohexyl, (C₁-C₄)-Alkylsulfonyl, (C₁-C₄)-Hydroxyalkyl, (C₁-C₄)-Aminoalkyl, Di-(C₁-C₄)-alkylamino-(C₁-C₄)-alkyl, (C₁-C₃)-Alkanoyl oder Phenyl- carbonyl bedeuten,
- R³, R⁴, R⁵, R⁶, R⁷ und R⁸: gleich oder verschieden sind und für Wasserstoff oder für (C₁-C₄)-Alkyl stehen
und deren pharmazeutisch verträglichen Salze, Hydrate und Prodrugs.

Ganz besonders bevorzugt sind hierbei Verbindungen der allgemeinen Formel (I),
worin
- R¹: für 2-Thiophen, steht, das in der 5-Position substituiert ist durch einen Rest aus der Gruppe Chlor, Brom, Methyl oder Trifluormethyl,
- R²: für D-A- steht:
wobei:
der Rest "A" für Phenylen steht;
der Rest "D" für einen gesättigten 5- oder 6-gliedrigen Heterocyclus steht,
der über ein Stickstoffatom mit "A" verknüpft ist,
der in direkter Nachbarschaft zum verknüpfenden Stickstoffatom eine Carbonylgruppe besitzt und
in dem ein Ring-Kohlenstoffglied durch ein Heteroatom aus der Reihe S, N und O ersetzt sein kann;

wobei
die zuvor definierten Gruppe "A" in der meta-Position bezüglich der Ver- knüpfung zum Oxazolidinon gegebenenfalls ein- oder zweifach substituiert sein kann mit einem Rest aus der Gruppe von Fluor, Chlor, Nitro, Amino, Trifluormethyl, Methyl oder Cyano,
- R³, R⁴, R⁵, R⁶, R⁷ und R⁸: für Wasserstoff stehen
und deren pharmazeutisch verträglichen Salze, Hydrate und Prodrugs.

Ebenfalls ganz besonders bevorzugt ist hierbei die Verbindung mit der folgenden Formel 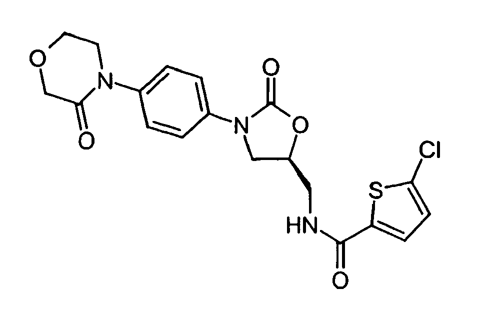 und ihre pharmazeutisch verträglichen Salze, Hydrate und Prodrugs.

Bislang sind Oxazolidinone im wesentlichen nur als Antibiotika, vereinzelt auch als MAO-Hemmer und Fibrinogen-Antagonisten beschrieben (Übersicht: Riedl, B., Endermann, R., Exp. Opin. Ther. Patents 1999, 9 (5), 625), wobei für die antibakterielle Wirkung eine kleine 5-[Acyl-aminomethyl]-gruppe (bevorzugt 5-[Acetyl-aminomethyl]) essentiell zu sein scheint.

Substituierte Aryl- und Heteroarylphenyloxazolidinone, bei denen an das N-Atom des Oxazolidinonrings ein ein- oder mehrfach substituierte Phenylrest gebunden sein kann und die in der 5-Position des Oxazolidinonrings einen unsubstituierten N-Methyl-2-thiophencarboxamid-Rest aufweisen können, sowie ihre Verwendung als antibakteriell wirkende Substanzen sind bekannt aus den U.S.-Patentschriften US-A-5 929 248, US-A-5 801 246, US-A-5 756 732, US-A-5 654 435, US-A-5 654 428 und US-A-5 565 571.

Darüber hinaus sind benzamidinhaltige Oxazolidinone als synthetische Zwischenstufen bei der Synthese von Faktor Xa-Inhibitoren bzw. Fibrinogenantagonisten bekannt (WO-A-99/31092, EP-A-623615).

Die Verbindungen der Formel (I) können in Abhängigkeit von dem Substitutionsmuster in stereoisomeren Formen, die sich entweder wie Bild und Spiegelbild (Enantiomere) oder die sich nicht wie Bild und Spiegelbild (Diastereomere) verhalten, existieren. Umfasst sind sowohl die Enantiomeren oder Diastereomeren als auch deren jeweilige Mischungen. Die Racemformen lassen sich ebenso wie die Diastereomeren in bekannter Weise in die stereoisomer einheitlichen Bestandteile trennen.

Weiterhin können bestimmte Verbindungen der Formel (I) in tautomeren Formen vorliegen. Dies ist dem Fachmann bekannt, und derartige Verbindungen sind ebenfalls umfasst.

Physiologisch unbedenkliche, d.h. pharmazeutisch verträgliche Salze können Salze der erfindungsgemäßen Verbindungen mit anorganischen oder organischen Säuren sein. Bevorzugt werden Salze mit anorganischen Säuren wie beispielsweise Chlorwasserstoffsäure, Bromwasserstoffsäure, Phosphorsäure oder Schwefelsäure, oder Salze mit organischen Carbon- oder Sulfonsäuren wie beispielsweise Essigsäure, Trifluoressigsäure, Propionsäure, Maleinsäure, Fumarsäure, Äpfelsäure, Zitronensäure, Weinsäure, Milchsäure, Benzoesäure, oder Methansulfonsäure, Ethansulfonsäure, Benzolsulfonsäure, Toluolsulfonsäure oder Naphthalindisulfonsäure.

Als pharmazeutisch verträgliche Salze können auch Salze mit üblichen Basen genannt werden, wie beispielsweise Alkalimetallsalze (z.B. Natrium- oder Kaliumsalze), Erdalkalisalze (z.B. Calcium- oder Magnesiumsalze) oder Ammoniumsalze, abgeleitet von Ammoniak oder organischen Aminen wie beispielsweise Diethylamin, Triethylamin, Ethyldiisopropylamin, Prokain, Dibenzylamin, N-Methyhnorpholin, Dihydroabietylamin oder Methylpiperidin.

Als "Hydrate" werden solche Formen der Verbindungen der obigen Formel (I) bezeichnet, welche in festem oder flüssigem Zustand durch Hydratation mit Wasser eine Molekül-Verbindung (Solvat) bilden. In den Hydraten sind die Wassermoleküle nebenvalent durch zwischenmolekulare Kräfte, insbesondere Wasserstoff-Brückenbindungen angelagert. Feste Hydrate enthalten Wasser als sogenanntes Kristall-Wasser in stöchiometrischen Verhältnissen, wobei die Wassermoleküle hinsichtlich ihres Bindungszustands nicht gleichwertig sein müssen. Beispiele für Hydrate sind Sesquihydrate, Monohydrate, Dihydrate oder Trihydrate. Gleichermaßen kommen auch die Hydrate von Salzen der erfindungsgemäßen Verbindungen in Betracht.

Als "Prodrugs" werden solche Formen der Verbindungen der obigen Formel (I) bezeichnet, welche selbst biologisch aktiv oder inaktiv sein können, jedoch in die entsprechende biologisch aktive Form überführt werden können (beispielsweise metabolisch, solvolytisch oder auf andere Weise).

Halogen steht für Fluor, Chlor, Brom und Iod. Bevorzugt sind Chlor oder Fluor.

(C₁-C₈-) Alkyl steht für einen geradkettigen oder verzweigten Alkylrest mit 1 bis 8 Kohlenstoffatomen. Beispielsweise seien genannt: Methyl, Ethyl, n-Propyl, Isopropyl, n-Butyl, Isobutyl, tert.-Butyl, n-Pentyl und n-Hexyl. Aus dieser Definition leiten sich analog die entsprechenden Alkylgruppen mit weniger Kohlenstoffatomen wie z.B. (C₁-C₆)-Alkyl und (C₁-C₄)-Alkyl ab. Im allgemeinen gilt, dass (C₁-C₄)-Alkyl bevorzugt ist.

Aus dieser Definition leitet sich auch die Bedeutung des entsprechenden Bestandteils anderer komplexerer Substituenten ab wie z.B. bei Alkylsulfonyl, Hydroxyalkyl, Hydroxyalkylcarbonyl, Alkoxy-alkyl, Alkoxycarbonyl-alkyl, Alkanoylalkyl, Aminoalkyl oder Alkylaminoalkyl.

(C₃-C₇)-Cycloalkyl steht für einen cyclischen Alkylrest mit 3 bis 7 Kohlenstoffatomen. Beispielsweise seien genannt: Cyclopropyl, Cyclobutyl, Cyclopentyl, Cyclohexyl oder Cycloheptyl. Aus dieser Definition leiten sich analog die entsprechenden Cycloalkylgruppen mit weniger Kohlenstoffatomen wie z.B. (C₃-C₅)-Cycloalkyl ab. Bevorzugt sind Cyclopropyl, Cyclopentyl und Cyclohexyl.

Aus dieser Definition leitet sich auch die Bedeutung des entsprechenden Bestandteils anderer komplexerer Substituenten ab wie z.B. Cycloalkanoyl.

(C₂-C₆)-Alkeny steht für einen geradkettigen oder verzweigten Alkenylrest mit 2 bis 6 Kohlenstoffatomen. Bevorzugt ist ein geradkettiger oder verzweigter Alkenylrest mit 2 bis 4 Kohlenstoffatomen. Beispielsweise seien genannt: Vinyl, Allyl, Isopropenyl und n-But-2-en-1-yl.

(C₁-C₈)-Alkoxy steht für einen geradkettigen oder verzweigten Alkoxyrest mit 1 bis 8 Kohlenstoffatomen. Beispielsweise seien genannt: Methoxy, Ethoxy, n-Propoxy, Isopropoxy, n-Butoxy, Isobutoxy, tert.-Butoxy, n-Pentoxy, n-Hexoxy, n-Heptoxy und n-Oktoxy. Aus dieser Definition leiten sich analog die entsprechenden Alkoxygruppen mit weniger Kohlenstoffatomen wie z.B. (C₁-C₆)-Alkoxy und (C₁-C₄)-Alkoxy ab. Im allgemeinen gilt, dass (C₁-C₄)-Alkoxy bevorzugt ist.

Aus dieser Definition leitet sich auch die Bedeutung des entsprechenden Bestandteils anderer komplexerer Substituenten ab wie z.B. Alkoxy-alkyl, Alkoxycarbonyl-alkyl und Alkoxycarbonyl.

Mono- oder Di-(C₁-C₄)-Akylaminocarbonyl steht für eine Amino-Gruppe, die über eine Carbonylgruppe verknüpft ist und die einen geradkettigen oder verzweigten bzw. zwei gleiche oder verschiedene geradkettige oder verzweigte Alkylsubstituenten mit jeweils 1 bis 4 Kohlenstoffatomen aufweist. Beispielsweise seien genannt: Methylamino, Ethylamino, n-Propylamino, Isopropylamino, t-Butylamino, *N*,*N*-Dimethyl-amino, *N*,*N*-Diethylamino, *N*-Ethyl-*N*-methylamino, *N*-Methyl-*N*-n-propylamino, *N-*Isopropyl-*N*-n-propylamino und *N*-t-Butyl-*N*-methylanüno.

C₁-C₆)-Alkanoyl steht für einen geradkettigen oder verzweigten Alkylrest mit 1 bis 6 Kohlenstoffatomen, der in der 1-Position ein doppelt gebundenes Sauerstoffatom trägt und über die 1-Position verknüpft ist. Beispielsweise seien genannt: Formyl, Acetyl, Propionyl, n-Butyryl, i-Butyryl, Pivaloyl, n-Hexanoyl. Aus dieser Definition leiten sich analog die entsprechenden Alkanoylgruppen mit weniger Kohlenstoffatomen wie z.B. (C₁-C₅)-Alkanoyl, (C₁-C₄)-Alkanoyl und (C₁-C₃)-Alkanoyl ab. Im allgemeinen gilt, dass (C₁-C₃)-Alkanoyl bevorzugt ist.

Aus dieser Definition leitet sich auch die Bedeutung des entsprechenden Bestandteils anderer komplexerer Substituenten ab wie z.B. Cycloalkanoyl und Alkanoylalkyl.

(C₃-C₇)-Cycloalkanoyl steht für einen wie zuvor definierten Cycloalkylrest mit 3 bis 7 Kohlenstoffatomen, der über eine Carbonylgruppe verknüpft ist.

(C₁-C₆)-Alkanoyloxymethyloxy steht für einen geradkettigen oder verzweigten Alkanoyloxymethyloxy-Rest mit 1 bis 6 Kohlenstoffatomen. Beispielsweise seien genannt: Acetoxymethyloxy, Propionoxymethyloxy, n-Butyroxymethyloxy, i-Butyroxymethyloxy, Pivaloyloxymethyloxy, n-Hexanoyloxymethyloxy. Aus dieser Definition leiten sich analog die entsprechenden Alkanoyloxymethyloxy-Gruppen mit weniger Kohlenstoffatomen wie z.B. (C₁-C₃)-Alkanoyloxymethyloxy ab. Im allgemeinen gilt, dass (C₁-C₃)-Alkanoyloxymethyloxy bevorzugt ist.

(C₆-C₁₄)-Aryl steht für einen aromatischen Rest mit 6 bis 14 Kohlenstoffatomen. Beispielsweise seien genannt: Phenyl, Naphthyl, Phenanthrenyl und Anthracenyl. Aus dieser Definition leiten sich analog die entsprechenden Arylgruppen mit weniger Kohlenstoffatomen wie z.B. (C₆-C₁₀)-Aryl ab. Im allgemeinen gilt, dass (C₆-C₁₀)-Aryl bevorzugt ist.

Aus dieser Definition leitet sich auch die Bedeutung des entsprechenden Bestandteils anderer komplexerer Substituenten ab wie z.B. Arylcarbonyl.

(C₅-C₁₀)-Heteroaryl oder ein 5- bis 10-gliedriger aromatischer Heterocyclus mit bis zu 3 Heteroatomen und/oder Heterokettengliedern aus der Reihe S, O, N und/oder NO (N-Oxid) steht für einen mono- oder bicyclischen Heteroaromaten, der über ein Ringkohlenstoffatom des Heteroaromaten, gegebenenfalls auch über ein Ringstickstoffatom des Heteroaromaten, verknüpft ist. Beispielsweise seien genannt: Pyridyl, Pyridyl-N-oxid, Pyrimidyl, Pyridazinyl, Pyrazinyl, Thienyl, Furyl, Pyrrolyl, Pyrazolyl, Imidazolyl, Thiazolyl, Oxazolyl oder Isoxazolyl, Indolizinyl, Indolyl, Benzo[b]thienyl, Benzo[b]furyl, Indazolyl, Chinolyl, Isochinolyl, Naphthyridinyl, Chinazolinyl. Aus dieser Definition leiten sich analog die entsprechenden Heterocyclen mit geringerer Ringgröße wie z.B. 5- oder 6-gliedrige aromatische Heterocyclen ab. Im allgemeinen gilt, dass 5- oder 6-gliedrige aromatische Heterocyclen wie z.B. Pyridyl, Pyridyl-N-oxid, Pyrimidyl, Pyridazinyl, Furyl und Thienyl bevorzugt sind.

Aus dieser Definition leitet sich auch die Bedeutung des entsprechenden Bestandteils anderer komplexerer Substituenten ab wie z.B. (C₅-C₁₀)-Heteroarylcarbonyl.

Ein 3- bis 9-gliedriger gesättigter oder teilweise ungesättigter, mono- oder bicyclischer, gegebenenfalls benzokondensierter Heterocyclus mit bis zu 3 Heteroatomen und/oder Heterokettengliedern aus der Reihe S, SO, SO₂, N, NO (N-Oxid) und/oder O steht für einen Heterocyclus, der eine oder mehrere Doppelbindungen enthalten kann, der mono- oder bicyclisch sein kann, bei dem an zwei benachbarte Ringkohlenstoffatomen ein Benzolring ankondensiert sein kann und der über ein Ringkohlenstoffatom oder ein Ringstickstoftätom verknüpft ist. Beispielsweise seien genannt: Tetrahydrofuryl, Pyrrolidinyl, Pyrrolinyl, Piperidinyl, 1,2-Dihydropyridinyl, 1,4-Dihydropyridinyl, Piperazinyl, Morpholinyl, Morpholinyl-N-oxid, Thiomorpholinyl, Azepinyl, 1,4-Diazepinyl und Cyclohexyl. Bevorzugt sind Piperidinyl, Morpholinyl und Pyrrolidinyl.

Aus dieser Definition leiten sich analog die entsprechenden Cyclen mit geringerer Ringgröße wie z.B. 5- bis 7-gliedrige Cyclen ab.

Die Verbindungen der Formel (I) können hergestellt werden, indem man entweder gemäß einer Verfahrensalternative

### [A] Verbindungen der allgemeinen Formel (II)

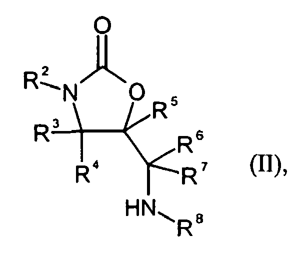

in welcher
die Reste R², R³, R⁴, R⁵, R⁶, R⁷und R⁸ die oben angegebenen Bedeutungen haben,
mit Carbonsäuren der allgemeinen Formel (III) 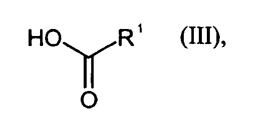 in welcher
der Rest R¹ die oben angegebene Bedeutung hat,
oder aber mit den entsprechenden Carbonsäurehalogeniden, vorzugsweise Carbonsäurechloriden, oder aber mit den entsprechenden symmetrischen oder gemischten Carbonsäureanhydriden der zuvor definierten Carbonsäuren der allgemeinen Formel (III)
in inerten Lösungsmitteln, gegebenenfalls in Gegenwart eines Aktivierungs-oder Kupplungsreagenzes und/oder einer Base, zu Verbindungen der allgemeinen Formel (I) 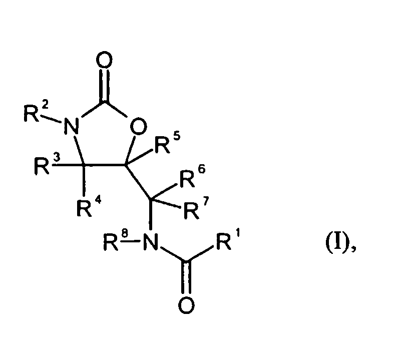 in welcher
die Reste R¹, R², R³, R⁴, R⁵, R⁶, R⁷ und R⁸ die oben angegebenen Bedeutungen haben,
umsetzt,
oder aber gemäß einer Verfahrensalternative

### [B] Verbindungen der allgemeinen Formel (IV)

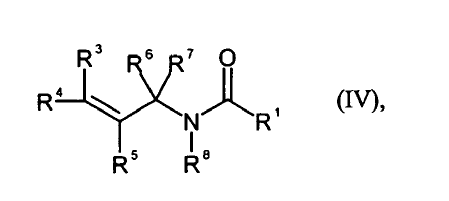

in welcher
die Reste R¹, R³, R⁴, R⁵, R⁶, R⁷ und R⁸ die oben angegebenen Bedeutungen haben,
mit einem geeigneten selektiven Oxidationsmittel in einem inerten Lösungsmittel in das entsprechenden Epoxid der allgemeinen Formel (V) 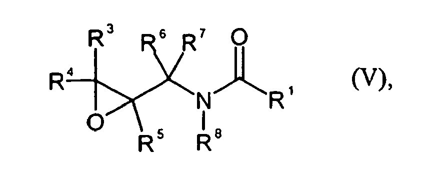 in welcher
die Reste R¹, R³, R⁴, R⁵, R⁶, R⁷ und R⁸ die oben angegebenen Bedeutungen haben,
überführt,
und durch Umsetzung in einem inerten Lösungsmittel gegebenenfalls in Gegenwart eines Katalysators mit einem Amin der allgemeinen Formel (VI)

R² - NH² (VI),

in welcher
der Rest R² die oben angegebene Bedeutung hat,
zunächst die Verbindungen der allgemeinen Formel (VII) 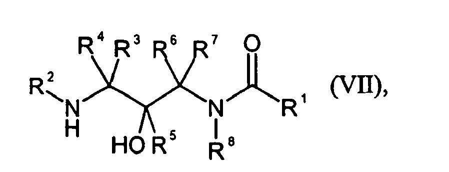 in welcher
die Reste R¹, R², R³, R⁴, R⁵, R⁶, R⁷ und R⁸ die oben angegebenen Bedeutungen haben,
herstellt und
anschließend in inertem Lösungsmittel in Anwesenheit von Phosgen oder Phosgenäquivalenten wie z.B. Carbonyldiimidazol (CDI) zu den Verbindungen der allgemeinen Formel (I) 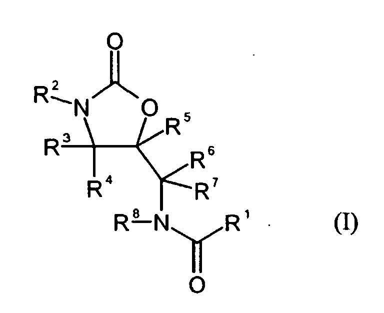 in welcher
die Reste R¹, R², R³, R⁴, R⁵, R⁶, R⁷ und R⁸ die oben angegebenen Bedeutungen haben,
cyclisiert,
wobei sich sowohl für die Verfahrensalternative [A] als auch für die Verfahrensalternative [B] für den Fall, dass R² einen 3- bis 7- gliedrigen gesättigten oder teilweise ungesättigten cyclischen Kohlenwasserstoffrest mit einem oder mehreren gleichen oder verschiedenen Heteroatomen aus der Gruppe von N und S enthält, eine Oxidation mit einem selektiven Oxidationsmittel zum entsprechenden Sulfon, Sulfoxid oder N-Oxid anschließen kann
und/oder
wobei sich sowohl für die Verfahrensalternative [A] als auch für die Verfahrensalternative [B] für den Fall, dass die auf diese Weise hergestellte Verbindung eine Cyanogruppe im Molekül aufweist, eine Amidinierung dieser Cyanogruppe mit den üblichen Methoden anschließen kann
und/oder
wobei sich sowohl für die Verfahrensalternative [A] als auch für die Verfahrensalternative [B] für den Fall, dass die auf diese Weise hergestellte Verbindung eine BOC-Aminoschutzgruppe im Molekül aufweist, eine Abspaltung dieser BOC-Aminoschutzgruppe mit den üblichen Methoden anschließen kann
und/oder
wobei sich sowohl für die Verfahrensalternative [A] als auch für die Verfahrensalternative [B] für den Fall, dass die auf diese Weise hergestellte Verbindung einen Anilin- oder Benzylaminrest im Molekül aufweist, eine Umsetzung dieser Aminogruppe mit verschiedenen Reagenzien wie Carbonsäuren, Carbonsäureanhydriden, Carbonsäurechloriden, Isocyanaten, Sulfonsäurechloriden oder Alkylhalogeniden zu den entsprechenden Derivaten anschließen kann
und/oder
wobei sich sowohl für die Verfahrensalternative [A] als auch für die Verfahrensalternative [B] für den Fall, dass die auf diese Weise hergestellte Verbindung einen Phenylring im Molekül aufweist, eine Reaktion mit Chlorsulfonsäure und anschließende Umsetzung mit Aminen zu den entsprechenden Sulfonamiden anschließen kann.

Die Verfahren können durch folgende Formelschemata beispielhaft erläutert werden:

### [A]

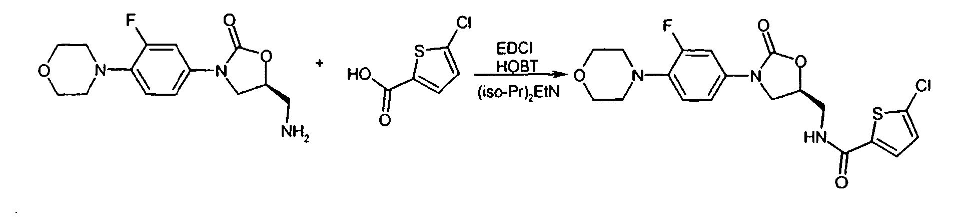

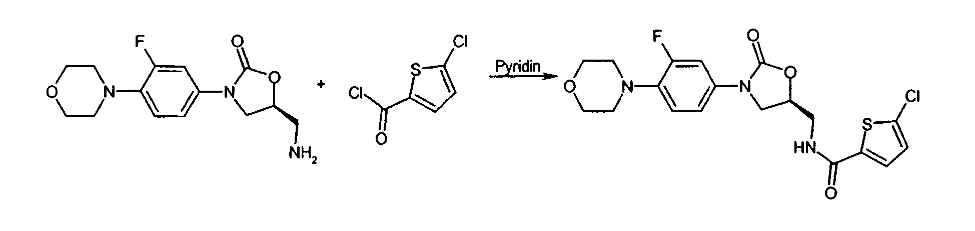

### [B]

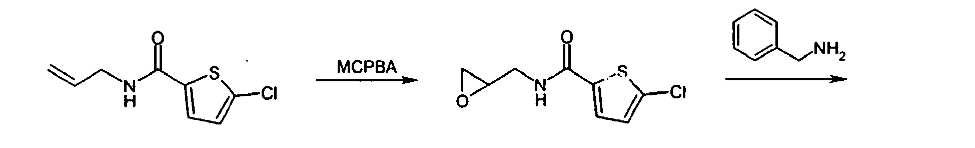

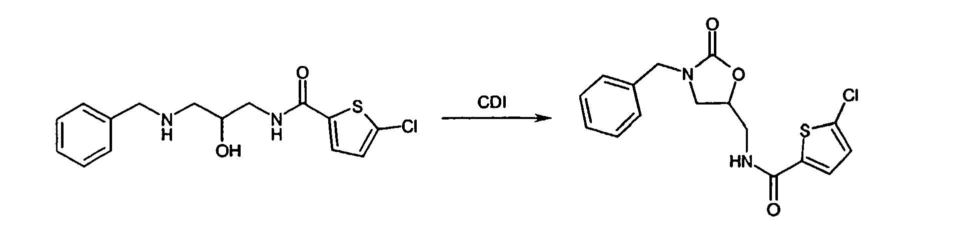

Der zuvor beschriebene, gegebenenfalls erfolgende Oxidationsschritt kann durch folgende Formelschemata beispielhaft erläutert werden:

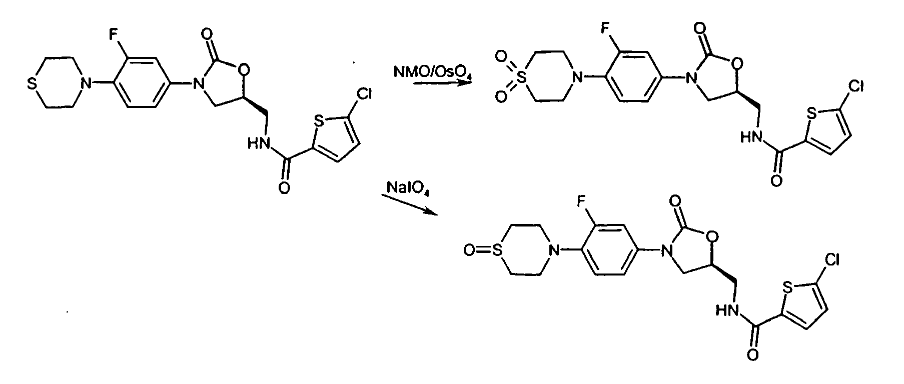

Als Lösemittel für die zuvor beschriebenen Verfahren eignen sich hierbei organische Lösemittel, die unter den Reaktionsbedingungen inert sind. Hierzu gehören Halogenkohlenwasserstoffe wie Dichlormethan, Trichlormethan, Tetrachlormethan, 1,2-Dichlorethan, Trichlorethan, Tetrachlorethan, 1,2-Dichlorethylen oder Trichlorethylen, Ether wie Diethylether, Dioxan, Tetrahydrofuran, Glykoldimethylether oder Diethylenglykoldimethylether, Alkohole wie Methanol, Ethanol, n-Propanol, iso-Propanol, n-Butanol oder tert.-Butanol, Kohlenwasserstoffe wie Benzol, Xylol, Toluol, Hexan oder Cyclohexan, Dimethylformamid, Dimethylsulfoxid, Acetonitril, Pyridin, Hexamethylphosphorsäuretriamid oder Wasser.

Ebenso ist es möglich, Lösemittelgemische der zuvor genannten Lösemittel einzusetzen.

Als Aktivierungs- oder Kupplungsreagenzien für die zuvor beschriebenen Verfahren eignen hierbei die hierfür üblicherweise verwendeten Reagenzien, beispielsweise *N*'-(3-Dimethylaminopropyl)-*N*-ethylcarbodiimid · HCl, *N,N*'-Dicyclohexylcarbodiimid, 1-Hydroxy-1H-benzotriazol · H₂O und dergleichen.

Als Basen eignen sich die üblichen anorganischen oder organischen Basen. Hierzu gehören bevorzugt Alkalihydroxide wie beispielsweise Natrium- oder Kaliumhydroxid oder Alkalicarbonate wie Natrium- oder Kaliumcarbonat oder Natrium- oder Kaliummethanolat oder Natrium- oder Kaliumethanolat oder Kalium-tert.-butylat oder Amide wie Natriumamid, Lithium-bis-(trimethylsilyl)amid oder Lithiumdiisopropylamid oder Amine wie Triethylamin, Diisopropylethylamin, Diisopropylamin, 4-*N*,*N*-Dimethylaminopyridin oder Pyridin.

Die Base kann hierbei in einer Menge von 1 bis 5 Mol, bevorzugt von 1 bis 2 Mol, bezogen auf 1 Mol der Verbindungen der allgemeinen Formel (II), eingesetzt werden.

Die Reaktionen erfolgen im allgemeinen in einem Temperaturbereich von -78°C bis zur Rückflusstemperatur, bevorzugt im Bereich von 0°C bis Rückflusstemperatur.

Die Umsetzungen können bei normalem, erhöhtem oder erniedrigtem Druck durchgeführt werden (z.B. im Bereich von 0,5 bis 5 bar). Im allgemeinen arbeitet man bei Normaldruck.

Als geeignete selektive Oxidationsmittel sowohl für die Herstellung der Epoxide als auch für die gegebenenfalls durchgeführte Oxidation zum Sulfon, Sulfoxid oder N-Oxid kommen beispielsweise m-Chlorperbenzoesäure (MCPBA), Natriummetaperiodat, N-Methylmorpholin-N-oxid (NMO), Monoperoxyphthalsäure oder Osmiumtetroxid in Betracht.

Hinsichtlich der Herstellung der Epoxide werden die hierfür üblichen Herstellungsbedingungen angewandt.

Hinsichtlich der näheren Verfahrensbedingungen für die gegebenenfalls durchgefiihrte Oxidation zum Sulfon, Sulfoxid oder N-Oxid kann verwiesen werden auf die folgende Literatur: M. R. Barbachyn et al., J. Med. Chem. 1996, 39, 680 sowie WO-A-97/10223.

Des weiteren wird auf die im experimentellen Teil aufgeführten Beispiele 14 bis 16 verwiesen.

Die gegebenenfalls durchgeführte Amidinierung erfolgt unter üblichen Bedingungen. Für weitere Einzelheiten kann auf die Beispiele 31 bis 35 und 140 bis 147 verwiesen werden.

Die Verbindungen der Formeln (II), (III), (IV) und (VI) sind dem Fachmann an sich bekannt oder nach üblichen Methoden herstellbar. Für Oxazolidinone, insbesondere die benötigten 5-(Aminomethyl)-2-oxooxazolidine, vgl. WO-A-98/01446; WO-A-93/23384; WO-A-97/03072; J. A. Tucker et al., J. Med. Chem. 1998, 41, 3727; S. J. Brickner et al., J. Med. Chem. 1996, 39, 673; W. A. Gregory et al., J. Med. Chem. 1989, 32, 1673.

Eine bevorzugte Verbindung A) der Formel (I) für den Einsatz in Kombinationen ist 5-Chloro-*N-*({(5*S*)-2-oxo-3-[4-(3-oxo-4-morpholinyl)phenyl]-1,3-oxazolidin-5-yl}methyl)-2-thiophencarboxamid, die Verbindung aus Beispiel **44**.

Die erfindungsgemäßen Kombinationen sind insbesondere zur Prophylaxe und/oder Behandlung von arteriellen Thrombosen und Embolien bei koronaren Herzerkrankungen, cerebrovaskulären Durchblutungsstörungen und peripheren arteriellen Durchblutungsstörungen geeignet. Die Kombinationen von Oxazolidinonen der Formel (I) mit Plättchenaggregationshemmern, Antikoagulantien und/oder Fibrinolytika sind darüber hinaus insbesondere zur Prophylaxe und/oder Behandlung venöser Thrombosen und Lungenembolien geeignet.

Die einzelnen Kombinationswirkstoffe sind literaturbekannt und größtenteils kommerziell erhältlich. Sie können gegebenenfalls, ebenso wie Oxazolidinone der Formel (I), in subtherapeutisch wirksamen Dosen eingesetzt werden.

Zur Prophylaxe und/oder Behandlung arterieller Gefäßerkrankungen ist eine Kombinationstherapie von Oxazolidinonen der Formel (I) mit Lipidsenkern, insbesondere mit HMG-CoA-(3-hydroxy-3-methylglutaryl-Coenzym A)-Reduktase-Inhibitoren wie beispielsweise Cerivastatin (Rivastatin, Baycol; US 5,177,080), Lovastatin (Mevacor; US 4,231,938), Simvastatin (Zocor; US 4,444,784), Pravastatin (Pravachol; US 4,346,227), Fluvastatin (Lescol; US 5,354,772), Atorvastatin (Lipitor; US 5,273,995), oder mit Koronartherapeutika/Vasodilatatoren, insbesondere ACE-(Angiotensin-Converting-Enzyme)-Inhibitoren, wie beispielsweise Captopril, Lisinopril, Enalapril, Ramipril, Cilazapril, Benazepril, Fosinopril, Quinapril, Perindopril; AII-(Angiotensin II)-Rezeptor-Antagonisten wie beispielsweise Embusartan (US 5,863,930), Losartan, Valsartan, Irbesartan, Candesartan, Eprosartan, Temisartan; β-Adrenozeptor-Antagonisten wie beispielsweise Carvedilol, Alprenolol, Bisoprolol, Acebutolol, Atenolol, Betaxolol, Carteolol, Metoprolol, Nadolol, Penbutolol, Pindolol, Propanolol, Timolol; alpha-1-Adrenozeptor-Antagonisten wie beispielsweise Prazosin, Bunazosin, Doxazosin, Terazosin; Diuretika wie beispielsweise Hydrochlorothiazid, Furosemid, Bumetanid, Piretanid, Torasemid, Amilorid; Dihydralazin; Calciumkanalblockern wie beispielsweise Verapamil, Diltiazem oder Dihydropyridin-Derivaten wie beispielsweise Nifedipin (Adalat) oder Nitrendipin (Bayotensin); Substanzen, die eine Erhöhung von cyclischem Guanosinmonophosphat (cGMP) bewirken wie beispielsweise Stimulatoren der löslichen Guanylatcyclase (WO 98/16223, WO 98/16507, WO 98/23619, WO 00/06567, WO 00/06568, WO 00/06569, WO 00/21954, WO 00/66582, WO 01/17998, WO 01/19776, WO 01/19355, WO 01/19780, WO 01/19778), geeignet.

Das pharmakotherapeutische Ziel der Behandlung einer bereits bestehenden koronaren Herzkrankheit ist die Beseitigung des Missverhältnisses zwischen Sauerstoffangebot und Sauerstoffbedarf in den von der Ischämie betroffenen Myokardbezirken. Zur Behandlung bei einer bereits bestehenden koronaren Herzkrankheit ist daher insbesondere eine Kombinationstherapie eines Oxazolidinons der Formel (I) mit Koronartherapeutika, insbesondere mit β-Adrenozeptor-Antagonisten; ACE-(Angiotensin-Converting Enzym)-Inhibitoren; A-II-(Angiotensin II)-Rezeptor-Antagonisten; Nitropräparaten wie beispielsweise Isosorbid-5-mononitrat, Isosorbid-dinitrat, Glyceroltrinitrat; Substanzen, die eine Erhöhung von cyclischem Guanosinmonophosphat (cGMP) bewirken; Calciumkanalblockern geeignet. Die Mehrzahl dieser Verbindungen werden auch zur Therapie des Hochdrucks eingesetzt.

Um thrombotisch verschlossene Gefäße wieder zu öffnen, hat sich die thrombolytische Therapie mit Plasminogen-Aktivatoren (Thrombolytika/Fibrinolytika) wie beispielsweise Gewebsplasminogen-Aktivator (t-PA), Streptokinase, Reteplase oder Urokinase bewährt. Die alleinige Verabreichung von Plasminogen-Aktivatoren verhindert aber nicht ein weiteres Wachstum des Thrombus. Hohe Dosen von Plasminogen-Aktivatoren können zudem ein erhöhtes Blutungsrisiko bedeuten. Die kombinierte Gabe von einem Thrombolytikum mit einem Oxazolidinon der Formel (I) zur Öffnung von thrombotisch verschlossenen Gefäßen bei koronarer Herzerkrankung, transienten ischämischen Attacken, Hirnschlag, peripheren arteriellen Verschlusserkrankungen und Lungenembolien verhindert ein weiteres Wachstum des Thrombus durch die Hemmung der Thrombinbildung und vermindert somit das Risiko eines erneuten Verschlusses. Darüber hinaus kann bei einer Kombinationstherapie mit einem Thrombolytikum und einem Oxazolidinon der Formel (I) die therapeutisch erforderliche Dosis des Thrombolytikums herabgesetzt werden, was zu einer Verringerung der Blutungskomplikationen führt und damit einen erheblichen Vorteil gegenüber der Monotherapie darstellt.

Oxazolidinone der Formel (I) können auch in Kombination mit anderen antikoagulatorisch wirksamen Substanzen (Antikoagulantien) zur Prophylaxe und/oder Behandlung von arteriellen, - intrakardialen und venösen thromboembolischen Erkrankungen gegeben werden. Die Kombinationstherapie von Oxazolidinonen der Formel (I) insbesondere mit Heparin (UFH), niedermolekularen Heparinen (NMH) wie beispielsweise Tinzaparin, Certoparin, Parnaparin, Nadroparin, Ardeparin, Enoxaparin, Reviparin, Dalteparin oder direkten Thrombin-Inhibitoren wie beispielsweise Hirudin führt zu einer verstärkten antithrombotischen Wirkung. Oxazolidinone der Formel (I) können darüber hinaus auch in Kombination mit plättchenaggregationshemmenden Substanzen (Plättchenaggregationshemmer, Thrombozytenaggregationshemmer) zur Prophylaxe und/oder Behandlung von arteriellen, intrakardialen und venösen thromboembolischen Erkrankungen gegeben werden. Bei einer Endothelläsion kommt es zur Wandhaftung und Aktivierung von Blutplättchen und zur gleichzeitigen Stimulierung der Blutgerinnung. Dies führt zur Entstehung von plättchen- und fibrinhaltigen Thromben wobei die Plättchen zur Stabilisierung des Fibringerüstes beitragen (J. Hirsh, E. W. Salzman, V. J. Marder, R. W. Colman, Overview of the Thrombotic Process and its Therapy, Seite 1151-1163 in Hemostasis and Thrombosis: Basic Principles and Clinical Practice, Third Edition, edited by R. W. Colman, J. Hirsh, V. J. Marder, E. W. Salzman. J. B. Lippincott Company, Philadelphia, 1994). Die gleichzeitige Hemmung der Blutgerinnung und der Plättchenaggregation führt daher zu einer verstärkten antithrombotischen Wirkung. Für die Kombinationstherapie geeignet sind insbesondere Kombinationen eines Oxazolidinons der Formel (I) mit Plättchenaggregationshemmern wie beispielsweise Aspirin, Ticlopidin (Ticlid), Clopidogrel (Plavix); Fibrinogen-Rezeptor-Antagonisten; (Glycoprotein-IIb/IIIa-Antagonisten) wie beispielsweise Abciximab, Eptifibatide, Tirofiban, Lamifiban, Lefradafiban.

Für die Applikation der erfindungsgemäßen Kombinationen kommen alle üblichen Applikationsformen in Betracht. Vorzugsweise erfolgt die Applikation oral, lingual, sublingual, bukkal, rektal, topical oder parenteral (d.h. unter Umgehung des Intestinaltraktes, also intravenös, intraarteriell, intrakardial, intrakutan, subkutan, transdermal, intraperitoneal oder intramuskulär).

Zur vorliegenden Erfindung gehören pharmazeutische Zubereitungen, die neben nicht-toxischen, inerten pharmazeutisch geeigneten Hilfs- und/oder Trägerstoffen eine oder mehrere erfindungsgemäße Kombinationen enthalten oder die aus einer erfindungsgemäßen Kombination bestehen, sowie Verfahren zur Herstellung dieser Zubereitungen.

Die erfindungsgemäßen Kombinationen sollen in den oben aufgeführten pharmazeutischen Zubereitungen in einer Konzentration von etwa 0,1 bis 99,5, vorzugsweise etwa 0,5 bis 95 Gew.-% der Gesamtmischung vorhanden sein.

Die oben aufgeführten pharmazeutischen Zubereitungen können außer den erfindungsgemäßen Kombinationen auch weitere pharmazeutische Wirkstoffe enthalten.

Die Herstellung der oben aufgeführten pharmazeutischen Zubereitungen kann in üblicher Weise nach bekannten Methoden erfolgen, z.B. durch Mischen des Wirkstoffes oder der Wirkstoffe mit dem oder den Trägerstoffen.

Im allgemeinen hat es sich als vorteilhaft erwiesen, die erfindungsgemäßen Kombinationen in Gesamtmengen von etwa 0,001 bis 100 mg/kg, vorzugsweise etwa 0,01 bis 100 mg/kg, insbesondere etwa 0,1 bis 10 mg/kg Körpergewicht je 24 Stunden, gegebenenfalls in Form mehrerer Einzelgaben, zur Erzielung der gewünschten Ergebnisse zu verabreichen.

Trotzdem kann es gegebenenfalls erforderlich sein, von den zuvor genannten Mengen abzuweichen, und zwar in Abhängigkeit vom Körpergewicht, von der Art des Applikationsweges, der Art und Schwere der Erkrankung, vom individuellen Verhalten gegenüber dem Medikament, von der Art der Formulierung und von dem Zeitpunkt bzw. Intervall, zu welchem die Verabreichung erfolgt. So kann es in einigen Fällen ausreichend sein, mit weniger als der vorgenannten Mindestmenge auszukommen, während in anderen Fällen die genannte obere Grenze überschritten werden muss. Es kann beispielsweise im Falle der Applikation größerer Mengen empfehlenswert sein, diese über den Tag zu verteilen, und zwar entweder in mehreren Einzelgaben oder als Dauerinfusion.

Weiterer Gegenstand der Erfindung sind daher die oben definierten Kombinationen zur Prophylaxe und/oder Behandlung von Erkrankungen.

Weiterer Gegenstand der Erfindung sind Arzneimittel, enthaltend mindestens eine der oben definierten Kombinationen und gegebenenfalls weitere pharmazeutische Wirkstoffe.

Weiterer Gegenstand der Erfindung ist die Verwendung der oben definierten Kombinationen zur Herstellung von Arzneimitteln zur Prophylaxe und/oder Behandlung der oben beschriebenen Erkrankungen, vorzugsweise von thromboembolischen Erkrankungen, insbesondere von Herzinfarkt, Angina Pectoris (eingeschlossen instabile Angina), plötzlichem Herztod, Reokklusionen und Restenosen nach einer Angioplastie oder aortokoronarem Bypass, Hirnschlag, transitorischen ischämischen Attacken, peripheren arteriellen Verschlusskrankheiten, Lungenembolien oder tiefen venösen Thrombosen.

Die Prozentangaben der nachfolgenden Beispiele beziehen sich jeweils auf das Gewicht; Teile sind Gewichtsteile.

### Beispiele

### A Bewertung der physiologischen Wirksamkeit

### 1. Physiologische Wirksamkeit der Verbindungen der Formel (I)

Die Verbindungen der Formel (I) wirken insbesondere als selektive Inhibitoren des Blutgerinnungsfaktors Xa und hemmen nicht oder erst bei deutlich höheren Konzentrationen auch andere Serinproteasen wie Thrombin, Plasmin oder Trypsin.

Als "selektiv" werden solche Inhibitoren des Blutgerinnungsfaktors Xa bezeichnet, bei denen die IC₅₀-Werte für die Faktor Xa-Inhibierung gegenüber den IC₅₀-Werten für die Inhibierung anderer Serinproteasen, insbesondere Thrombin, Plasmin und Trypsin, um das 100-fache, vorzugsweise um das 500-fache, insbesondere um das 1.000-fache, kleiner sind, wobei bezüglich der Testmethoden für die Selektivität Bezug genommen wird auf die im folgenden beschriebenen Testmethoden der Beispiele A-1) a.1) und a.2).

Die besonders vorteilhaften biologischen Eigenschaften der Verbindungen der Formel (I) können durch folgende Methoden festgestellt werden.

### a) Testbeschreibung (in vitro)

### a.1) Messung der Faktor Xa-Hemmung

Die enzymatische Aktivität von humanem Faktor Xa (FXa) wurde über die Umsetzung eines für den FXa-spezifischen chromogenen Substrats gemessen. Dabei spaltet der Faktor Xa aus dem chromogenen Substrat p-Nitroanilin ab. Die Bestimmungen wurden wie folgt in Mikrotiterplatten durchgeführt.

Die Prüfsubstanzen wurden in unterschiedlichen Konzentrationen in DMSO gelöst und für 10 Minuten mit humanem FXa (0,5 nmol/l gelöst in 50 mmol/l Tris-Puffer [C,C,C-Tris(hydroxymethyl)-aminomethan], 150 mmol/l NaCl, 0,1 % BSA (bovine serum albumine), pH = 8,3) bei 25°C inkubiert. Als Kontrolle dient reines DMSO. Anschließend wurde das chromogene Substrat (150 µmol/l Pefachrome^{®} FXa von der Firma Pentapharm) hinzugefügt. Nach 20 Minuten Inkubationsdauer bei 25°C wurde die Extinktion bei 405 nm bestimmt. Die Extinktionen der Testansätze mit Prüfsubstanz wurden mit den Kontrollansätzen ohne Prüfsubstanz verglichen und daraus die IC₅₀-Werte berechnet.

### a.2) Bestimmung der Selektivität

Zum Nachweis der selektiven FXa-Inhibition wurden die Prüfsubstanzen auf ihre Hemmung anderer humaner Serinproteasen wie Thrombin, Trypsin, Plasmin hin untersucht. Zur Bestimmung der enzymatischen Aktivität von Thrombin (75 mU/ml), Trypsin (500 mU/ml) und Plasmin (3,2 nmol/l) wurden diese Enzyme in Tris-Puffer (100 mmol/l, 20 mmol/l CaCl₂, pH = 8,0) gelöst und für 10 Minuten mit Prüfsubstanz oder Lösungsmittel inkubiert. Anschließend wurde durch Zugabe der entsprechenden spezifischen chromogenen Substrate (Chromozym Thrombin^{®} von der Firma Boehringer Mannheim, Chromozym Trypsin^{®} von der Firma Boehringer Mannheim, Chromozym Plasmin^{®} von der Firma Boehringer Mannheim) die enzymatische Reaktion gestartet und die Extinktion nach 20 Minuten bei 405 nm bestimmt. Alle Bestimmungen wurden bei 37°C durchgeführt. Die Extinktionen der Testansätze mit Prüfsubstanz wurden mit den Kontrollproben ohne Prüfsubstanz verglichen und daraus die IC₅₀-Werte berechnet.

### a.3) Bestimmung der antikoagulatorischen Wirkung

Die antikoagulatorische Wirkung der Prüfsubstanzen wurde in vitro in Humanplasma bestimmt. Dazu wurde Humanblut unter Verwendung einer 0,11 molaren Natriumcitrat-Lösung als Vorlage in einem Mischungsverhältnis Natriumcitrat/Blut 1/9 abgenommen. Das Blut wurde unmittelbar nach der Abnahme gut gemischt und 10 Minuten bei ca. 2000 g zentrifugiert. Der Überstand wurde abpipettiert. Die Prothrombinzeit (PT, Synonyme: Thromboplastinzeit, Quick-Test) wurde in Gegenwart variierender Konzentrationen an Prüfsubstanz oder dem entsprechenden Lösungsmittel mit einem handelsüblichen Testkit (Neoplastin^{®} von der Firma Boehringer Mannheim) bestimmt. Die Testverbindungen wurden 10 Minuten bei 37°C mit dem Plasma inkubiert. Anschließend wurde durch Zugabe von Thromboplastin die Gerinnung ausgelöst und der Zeitpunkt des Gerinnungseintritts bestimmt. Es wurde die Konzentration an Prüfsubstanz ermittelt, die eine Verdoppelung der Prothrombinzeit bewirkt.

### b) Bestimmung der antithrombotischen Wirkung (in vivo)

### b.1) Arteriovenöses Shunt-Modell (Ratte)

Nüchterne männliche Ratten (Stamm: HSD CPB:WU) mit einem Gewicht von 200-250 g wurden mit einer Rompun/Ketavet Lösung narkotisiert (12 mg/kg/50 mg/kg). Die Thrombusbildung wurde in einem arteriovenösen Shunt in Anlehnung an die von Christopher N. Berry et al., Br. J. Pharmacol. (1994), 113, 1209-1214 beschriebene Methode ausgelöst. Dazu wurden die linke Vena jugularis und die rechte Arteria carotis freipräpariert. Ein extracorporaler Shunt wurde mittels eines 10 cm langen Polyethylenschlauchs (PE 60) zwischen den beiden Gefäßen gelegt. Dieser Polyethylenschlauch war in der Mitte in einen weiteren 3 cm langen Polyethylenschlauch (PE 160), der zur Erzeugung einer thrombogenen Oberfläche einen aufgerauhten und zu einer Schlinge gelegten Nylonfaden enthielt, eingebunden. Der extrakorporale Kreislauf wurde 15 Minuten lang aufrechterhalten. Dann wurde der Shunt entfernt und der Nylonfaden mit dem Thrombus sofort gewogen. Das Leergewicht des Nylonfadens war vor Versuchsbeginn ermittelt worden. Die Prüfsubstanzen wurden vor Anlegung des extrakorporalen Kreislaufs entweder intravenös über die Schwanzvene oder oral mittels Schlundsonde wachen Tieren verabreicht. Die Ergebnisse sind in Tabelle 1 gezeigt:

**Tabelle 1: Antithrombotische Wirkung im arteriovenösern Shunt Modell (Ratte) nach oraler oder intravenöser Gabe**

| **Beispiel** | **ED₅₀ [mg/kg] p.o.** | **ED₅₀ [mg/kg] i.v.** |
|---|---|---|
| 1 | | 10 |
| 17 | | 6 |
| 44 | 3 | |
| 95 | | 3 |
| 114 | | 3 |
| 115 | | 3 |
| 123 | 3 | |
| 162 | | 3 |

### b.2) Arterielles Thrombose-Modell (Ratte)

Männliche nüchterne Ratten (Stamm: HSD CPB: WU) wurden wie oben beschrieben narkotisiert. Die Ratten waren im Mittel etwa 200 g schwer. Die linke Arteria carotis wurde freipräpariert (ca. 2 cm). Die Bildung eines arteriellen Thrombus wurde durch eine mechanische Gefäßverletzung in Anlehnung an die von K. Meng et al., Naunyn-Schmiedeberg's Arch. Pharmacol. (1977), 301, 115-119 beschriebene Methode induziert. Dazu wurde die freipräparierte Arteria carotis vom Blutfluss abgeklemmt, für 2 Minuten in einer Metallrinne auf -12°C abgekühlt und zur Standardisierung der Thrombengröße gleichzeitig mit einem Gewicht von 200 g komprimiert. Anschließend wurde der Blutfluss durch einen um die Arteria carotis distal von dem verletzten Gefäßabschnitt gelegten Clip zusätzlich reduziert. Die proximale Klemme wurde entfernt, die Wunde verschlossen und nach 4 Stunden wieder geöffnet, um den verletzten Gefäßabschnitt zu entnehmen. Der Gefäßabschnitt wurde longitudinal geöffnet und der Thrombus von dem verletzten Gefäßabschnitt entfernt. Das Feuchtgewicht der Thromben wurde sofort ermittelt. Die Prüfsubstanzen wurden zu Versuchsbeginn entweder intravenös über die Schwanzvene oder oral mittels Schlundsonde wachen Tieren verabreicht.

### b.3) Venöses Thrombose-Modell (Ratte)

Männliche nüchterne Ratten (Stamm: HSD CPB: WU) wurden wie oben beschrieben narkotisiert. Die Ratten waren im Mittel etwa 200 g schwer. Die linke Vena jugularis wurde freipräpariert (ca. 2 cm). Die Bildung eines venösen Thrombus wurde durch eine mechanische Gefäßverletzung in Anlehnung an die von K. Meng et al., Naunyn-Schmiedeberg's Arch. Pharmacol. (1977), 301, 115-119 beschriebene Methode induziert. Dazu wurde die Vena jugularis vom Blutfluss abgeklemmt, für 2 Minuten in einer Metallrinne auf-12°C abgekühlt und zur Standardisierung der Thrombengröße gleichzeitig mit einem Gewicht von 200 g komprimiert. Der Blutfluss wurde wieder eröffnet und die Wunde verschlossen. Nach 4 Stunden wurde die Wunde wieder geöffnet, um die Thromben von den verletzten Gefäßabschnitten zu entfernen. Das Feuchtgewicht der Thromben wurde sofort ermittelt. Die Prüfsubstanzen wurden zu Versuchsbeginn entweder intravenös über die Schwanzvene oder oral mittels Schlundsonde wachen Tieren verabreicht.

### 2. Physiologische Wirksamkeit der Kombinationen von Verbindungen der Formel (I)

### a) In vivo Untersuchungen in einem Ratten-Thrombosemodell

Unter Narkose wurde bei Ratten (HSD CPB:WU, Harlan Winkelmann) die Arteria carotis freipräpariert. Ein mit einer wässrigen 10%igen FeCl₃-Lösung (gelöst in 1N wässrige Salzsäure) getränktes Stück Filterpapier wurde vorsichtig unter das freipräparierte Gefäß geschoben, entsprechend der von Kurz et al. (Rat Model of Arterial Thrombosis Induced by Ferric Chloride, Thrombosis Research **60**, 269-280, 1990) beschriebenen Methode. Nach 3 Minuten wurde das Stück Filterpapier entfernt. Die Arteria carotis wurde nach 15 Minuten entnommen, der Thrombus abgelöst und sofort gewogen. Die Tiere (10 Ratten pro Gruppe) waren mit 1 mg/kg jeweils der einzelnen Wirkstoffe (Oxazolidinon der Formel (I) bzw. Kombinationswirkstoff) oder mit der Kombination von 1 mg/kg Oxazolidinon der Formel (I) und 1 mg/kg Kombinationswirkstoff vorbehandelt worden. Die Tiere der Kontrollgruppe waren mit dem entsprechendem Lösungsmittel behandelt worden. Die statistische Signifikanz wurde mittels Student's t-Test berechnet. Als statistisch signifikante Wirkung werden Werte mit p< 0.05 angesehen (Medical Statistics, MJ Campbell, D.Machin, Second Edition, John Wiley & Sons). Die Ergebnisse sind in Tabelle 2 gezeigt:

**Tabelle 2: Synergistische antithrombotische Wirkung der Kombination eines Oxazolidinons der Formel (I) mit einem Plättchenaggregationshemmer**

| Verminderung des Thrombusgewichts nach oraler Behandlung mit | | |
|---|---|---|
| Verbindung aus Beispiel 44 [1mg/kg] | Clopidogrel [1mg/kg] | Kombination der Verbindung aus Beispiel 44 [1mg/kg] mit Clopidogrel [1mg/kg] |
| 22% | 28% | 39% |
| kein Effekt (p > 0.05) | kein Effekt (p > 0.05) | Wirkung (p < 0.05) |

Wie in Tabelle 2 gezeigt, wird mit der Kombination eines Oxazolidinons der Formel (I) wie der Verbindung aus Beispiel 44 mit einem Plättchenaggregationshemmer wie Clopidogrel eine synergistische Wirkung erzielt, d.h. beide Komponenten verstärken sich gegenseitig in ihrer Wirkung. In der Einzeldosierung waren beide Verbindungen bei der untersuchten Dosis unwirksam. Die Kombination beider Verbindungen führte dagegen zu einer signifikanten Verminderung des Thrombusgewichts. Durch die Kombination von Oxazolidinonen der Formel (I) mit einer plättchenaggregationshemmenden Substanz kann daher die antithrombotische Therapie erheblich verbessert werden.

### B Herstellungbeispiele

### Ausgangsverbindungen

Die Darstellung von 3-Morpholinon wird in US 5 349 045 beschrieben.

Die Darstellung von N-(2,3-Epoxypropyl)phthalimid wird in J.-W. Chern et al. Tetrahedron Lett. 1998,39,8483 beschrieben.

Die substituierten Aniline können erhalten werden, indem man z.B. 4-Fluornitrobenzol, 2,4-Difluornitrobenzol oder 4-Chlornitrobenzol mit den entsprechenden Aminen oder Amiden in Gegenwart einer Base umsetzt. Dies kann auch unter Verwendung von Pd-Katalysatoren wie Pd(OAc)₂/DPPF/NaOt-Bu (Tetrahedron Lett. 1999,40,2035) oder Kupfer (Renger, Synthesis 1985,856; Aebischer et al., Heterocycles 1998,48,2225) geschehen. Genauso können Halogenaromaten ohne Nitrogruppe zunächst in die entsprechenden Amide umgewandelt werden, um sie anschließend in 4-Stellung zu nitrieren (US3279880).

### I. 4-(4-Morpholin-3-onyl)nitrobenzol

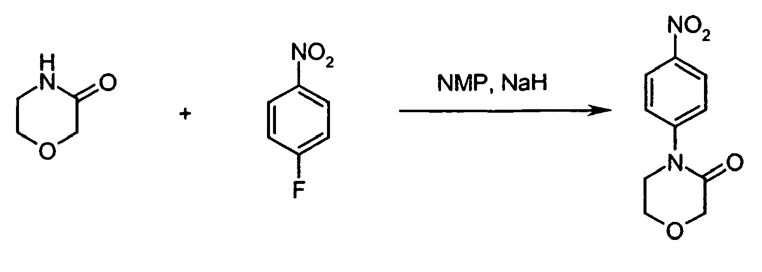

In 2 1 N-Methylpyrrolidon (NMP) werden 2 mol (202 g) Morpholin-3-on (E. Pfeil, U. Harder, Angew. Chem. 79, 1967, 188) gelöst. Über einen Zeitraum von 2 h erfolgt nun portionsweise die Zugabe von 88 g (2,2 mol) Natriumhydrid (60% in Paraffin). Nach Beendigung der Wasserstoffentwicklung werden unter Kühlung bei Raumtemperatur 282 g (2 mol) 4-Fluornitrobenzol innerhalb von 1 h zugetropft und das Reaktionsgemisch über Nacht nachgerührt. Im Anschluss werden bei 12 mbar und 76°C 1,71 des Flüssigkeitsvolumens abdestilliert, der Rückstand auf 2 1 Wasser gegossen und dieses Gemisch zweimal mit je 11 Ethylacetat extrahiert. Nach dem Waschen der vereinigten organischen Phasen mit Wasser wird über Natriumsulfat getrocknet und das Lösemittel im Vakuum abdestilliert. Die Reinigung erfolgt durch Chromatographie an Kieselgel mit Hexan/Ethylacetat (1:1) und nachfolgende Kristallisation aus Ethylacetat. Das Produkt fällt mit 78 g als farbloser bis bräunlicher Feststoff in 17,6 % d. Th. an.
¹H-NMR (300 MHz, CDCl₃): 3,86 (m, 2 H, CH₂CH₂), 4,08 (m, 2 H, CH₂C*H*₂), 4,49 (s, 2 H, C*H*₂CO), 7,61 (d, 2 H, ³*J*=8,95 Hz, C*H*CH), 8,28 (d, 2 H, ³*J*=8,95 Hz, CHC*H*)
MS (r.I.%) = 222 (74, M⁺), 193 (100), 164 (28), 150 (21), 136 (61), 117 (22), 106 (24), 90 (37), 76 (38), 63 (32), 50 (25)

Analog wurden folgende Verbindungen synthetisiert:
3-Fluor-4-(4-morpholin-3-onyl)nitrobenzol
4-(N-Piperidonyl)nitrobenzol
3-Fluor-4-(N-piperidonyl)nitrobenzol
4-(N-Pyrrolidonyl)nitrobenzol
3-Fluor-4-(N-pyrrolidonyl)nitrobenzol

### II. 4-(4-Morpholin-3-onyl)anilin

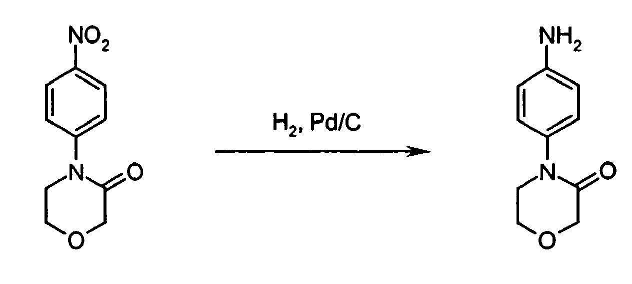

In einem Autoklaven werden 63 g (0,275 mol) 4-(4-Morpholin-3-onyl)nitrobenzol in 200 ml Tetrahydrofuran gelöst, mit 3,1 g Pd/C (5 %ig) versetzt und 8 h bei 70°C und einem Wasserstoffdruck von 50 bar hydriert. Nach Filtration des Katalysators wird das Lösemittel im Vakuum abdestilliert und das Produkt durch Kristallisation aus Ethylacetat gereinigt. Das Produkt fällt mit 20 g als farbloser bis bläulicher Feststoff in 37,6 % d. Th. an.

Die Reinigung kann auch durch Chromatographie an Kieselgel mit Hexan/Ethylacetat erfolgen.
¹H-NMR (300 MHz, CDCl₃): 3,67 (m, 2 H, C*H*₂CH₂), 3,99 (m, 2 H, CH₂C*H*₂), 4,27 (s, 2 H, C*H*₂CO), 6,68 (d, 2 H, ³*J*=8,71 Hz, C*H*CH), 7,03 (d, 2 H, ³*J*=8,71 Hz, CHC*H*)
MS (r.I.%) = 192 (100, M⁺), 163 (48), 133 (26), 119 (76), 106 (49), 92 (38), 67 (27), 65 (45), 52 (22), 28 (22)

Analog wurden folgende Verbindungen synthetisiert:
3-Fluor-4-(4-morpholin-3-onyl)anilin
4-(N-Piperidonyl)anilin
3-Fluor-4-(N-piperidonyl)anilin
4-(N-Pyrrolidonyl)anilin
3-Fluor-4-(N-pyrrolidonyl)anilin

### Allgemeine Methode zur Darstellung von 4-substituierten Anilinen durch Umsetzung von 1-Fluor-4-nitrobenzolen und 1-Chlor-4-nitrobenzolen mit primären oder sekundären Aminen und anschließender Reduktion

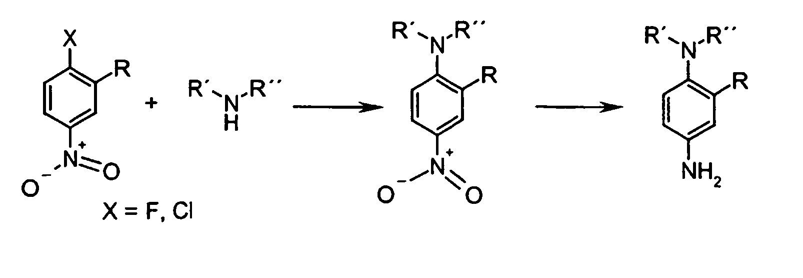

Äquimolare Mengen des Fluornitrobenzols bzw. Chlornitrobenzols und des Amins werden in Dimethylsulfoxid oder Acetonitril gelöst (0.1 M bis 1 M Lösung) und über Nacht bei 100°C gerührt. Nach Abkühlen auf RT wird das Reaktionsgemisch mit Ether verdünnt und mit Wasser gewaschen. Die organische Phase wird über MgSO₄ getrocknet, filtriert und eingeengt. Fällt im Reaktionsgemisch ein Niederschlag an, so wird dieser abfiltriert und mit Ether oder Acetonitril gewaschen. Ist auch in der Mutterlauge Produkt zu finden, wird diese wie beschrieben mit Ether und Wasser aufgearbeitet. Die Rohprodukte können durch Chromatographie an Kieselgel (Dichlormethan/Cyclohexan- und Dichlormethan/Ethanol-Gemische) gereinigt werden.

Zur anschließenden Reduktion wird die Nitroverbindung in Methanol, Ethanol oder Ethanol/Dichlormethan-Gemischen gelöst (0.01 M bis 0.5 M Lösung), mit Palladium auf Kohle (10%) versetzt und über Nacht unter Wasserstoff Normaldruck gerührt. Dann wird filtriert und eingeengt. Das Rohprodukt kann durch Chromatographie an Kieselgel (Dichlormethan/Ethanol-Gemische) oder präparative reversed-phase HPLC (Acetonitril/Wasser-Gemische) gereinigt werden.

Alternativ kann als Reduktionsmittel auch Eisenpulver verwendet werden. Dazu wird die Nitroverbindung in Essigsäure gelöst (0.1 M bis 0.5 M Lösung) und bei 90°C werden sechs Äquivalente Eisenpulver und Wasser (0.3- bis 0.5-faches Volumen der Essigsäure) portionsweise innerhalb von 10-15 min hinzugegeben. Nach weiteren 30 min bei 90°C wird filtriert und das Filtrat wird eingeengt. Der Rückstand wird mit Essigester und 2N Natronlauge extraktiv aufgearbeitet. Die organische Phase wird über Magnesiumsulfat getrocknet, filtriert und eingeengt. Das Rohprodukt kann durch Chromatographie an Kieselgel (Dichlormethan/Ethanol-Gemische) oder präparative reversed-phase HPLC (Acetonitril/Wasser-Gemische) gereinigt werden.

Auf analoge Weise wurden folgende Ausgangsverbindungen hergestellt:

### III-1. Tert.-butyl-1-(4-aminophenyl)-L-prolinat

MS (ESI): m/z (%) = 304 (M+H+MeCN, 100), 263 (M+H, 20);
HPLC (Methode 4): rt = 2.79 min.

### III-2. 1-(4-Aminophenyl)-3-piperidincarboxamid

MS (ESI): m/z (%) = 220 (M+H, 100);
HPLC (Methode 4): rt = 0.59 min.

### III-3. 1-(4-Aminophenyl)-4-piperidincarboxamid

MS (ESI): m/z (%) = 220 (M+H, 100);
HPLC (Methode 4): rt = 0.57 min.

### III-4. 1-(4-Aminophenyl)-4-piperidinon

MS (ESI): m/z (%) = 191 (M+H, 100);
HPLC (Methode 4): rt = 0.64 min.

### III-5. 1-(4-Aminophenyl)-L-prolinamid

MS (ESI): m/z (%) = 206 (M+H, 100);
HPLC (Methode 4): rt = 0.72 min.

### III-6. [1-(4-Aminophenyl)-3-piperidinyl]methanol

MS (ESI): m/z (%) = 207 (M+H, 100);
HPLC (Methode 4): rt = 0.60 min.

### III-7. [1-(4-Aminophenyl)-2-piperidinyl]methanol

MS (ESI): m/z (%) = 207 (M+H, 100);
HPLC (Methode 4): rt = 0.59 min.

### III-8. Ethyl-1-(4-aminophenyl)-2-piperidincarboxylat

MS (ESI): m/z (%) = 249 (M+H, 35), 175 (100);
HPLC (Methode 4): rt = 2.43 min.

### III-9. [1-(4-Aminophenyl)-2-pyrrolidinyl]methanol

MS (ESI): m/z (%) = 193 (M+H, 45);
HPLC (Methode 4): rt = 0.79 min.

### III-10. 4-(2-Methylhexahydro-5H-pyrrolo[3,4-d]isoxazol-5-yl)phenylamin

ausgehend von 2-Methylhexahydro-2H-pyrrolo[3,4-d]isoxazol (Ziegler, Carl B., et al.; J. Heterocycl. Chem.; 25; 2; 1988; 719-723)
MS (ESI): m/z (%) = 220 (M+H, 50), 171 (100);
HPLC (Methode 4): rt = 0.54 min.

### III-11. 4-(1-Pyrrolidinyl)-3-(trifluoromethyl)anilin

MS (ESI): m/z (%) = 231 (M+H, 100);
HPLC (Methode 7): rt = 3.40 min.

### III-12. 3-Chloro-4-(1-pyrrolidinyl)anilin

MS (ESI): m/z (%) = 197 (M+H, 100);
HPLC (Methode 4): rt = 0.78 min.

### III.-13. 5-Amino-2-(4-morpholinyl)benzamid

MS (ESI): m/z (%) = 222 (M+H, 100);
HPLC (Methode 4): rt = 0.77 min.

### III-14. 3-Methoxy-4-(4-morpholinyl)anilin

MS (ESI): m/z (%) = 209 (M+H, 100);
HPLC (Methode 4): rt = 0.67 min.

### III-15. 1-[5-Amino-2-(4-morpholinyl)phenyl]ethanon

MS (ESI): m/z (%) = 221 (M+H, 100);
HPLC (Methode 4): rt = 0.77 min.

### Allgemeine Methode zur Darstellung von 4-substituierten Anilinen durch Umsetzung von 1-Fluor-4-nitrobenzolen mit Amiden und anschließender Reduktion

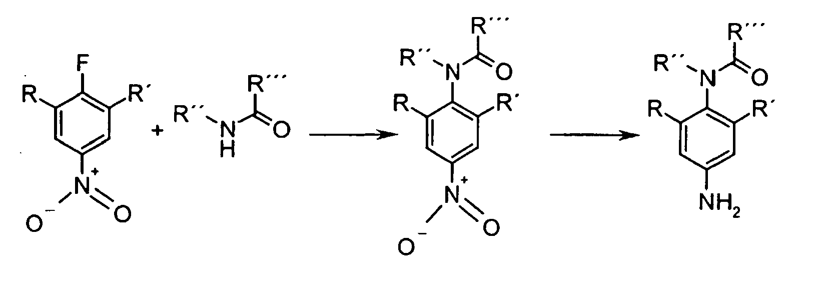

Das Amid wird in DMF gelöst und mit 1.5 Äquivalenten Kalium-tert.-butylat versetzt. Das Gemisch wird 1h bei RT gerührt, dann werden 1.2 Äquivalente des 1-Fluor-4-nitrobenzols portionsweise zugegeben. Das Reaktionsgemisch wird über Nacht bei RT gerührt, mit Ether oder Essigester verdünnt und mit ges. wässr. Natriumhydrogencarbonatlösung gewaschen. Die organische Phase wird über Magnesiumsulfat getrocknet, filtriert und eingeengt. Das Rohprodukt kann durch Chromatographie an Kieselgel (Dichlormethan/Ethanol-Gemische) gereinigt werden.

Zur anschließenden Reduktion wird die Nitroverbindung in Ethanol gelöst (0.01 M bis 0.5 M Lösung), mit Palladium auf Kohle (10%) versetzt und über Nacht unter Wasserstoff Normaldruck gerührt. Dann wird filtriert und eingeengt. Das Rohprodukt kann durch Chromatographie an Kieselgel (Dichlormethan/Ethanol-Gemische) oder präparative reversed-phase HPLC (Acetonitril/Wasser-Gemische) gereinigt werden.

Alternativ kann als Reduktionsmittel auch Eisenpulver verwendet werden. Dazu wird die Nitroverbindung in Essigsäure gelöst (0.1 M bis 0.5 M Lösung) und bei 90°C werden sechs Äquivalente Eisenpulver und Wasser (0.3- bis 0.5-faches Volumen der Essigsäure) portionsweise innerhalb von 10-15 min hinzugegeben. Nach weiteren 30 min bei 90°C wird filtriert und das Filtrat wird eingeengt. Der Rückstand wird mit Essigester und 2N Natronlauge extraktiv aufgearbeitet. Die organische Phase wird über Magnesiumsulfat getrocknet, filtriert und eingeengt. Das Rohprodukt kann durch Chromatographie an Kieselgel (Dichlormethan/Ethanol-Gemische) oder präparative reversed-phase HPLC (Acetonitril/Wasser-Gemische) gereinigt werden.

Auf analoge Weise wurden folgende Ausgangsverbindungen hergestellt:

### IV-1. 1.[4-Amino-2-(trifluoromethyl)phenyl]-2-pyrrolidinon

MS (ESI): m/z (%) = 245 (M+H, 100);
HPLC (Methode 4): rt = 2.98 min

### IV-2. 4-[4-Amino-2-(trifluoromethyl)phenyl]-3-morpholinon

MS (ESI): m/z (%) = 261 (M+H, 100);
HPLC (Methode 4): rt = 2.54 min.

### IV-3. 4-(4-Amino-2-chlorophenyl)-3-morpholinon

MS (ESI): m/z (%) = 227 (M+H, 100);
HPLC (Methode 4): rt = 1.96 min.

### IV-4. 4-(4-Amino-2-methylphenyl)-3-morpholinon

MS (ESI): m/z (%) = 207 (M+H, 100);
HPLC (Methode 4): rt = 0.71 min.

### IV-5. 5-Amino-2-(3-oxo-4-morpholinyl)benzonitril

MS (ESI): m/z (%) = 218 (M+H, 100);
HPLC (Methode 4): rt = 1.85 min.

### IV-6. 1-(4-Amino-2-chlorophenyl)-2-pyrrolidinon

MS (ESI): m/z (%) = 211 (M+H, 100);
HPLC (Methode 4): rt = 2.27 min.

### IV-7. 4-(4-Amino-2,6-dimethylphenyl)-3-morpholinon

ausgehend von 2-Fluoro-1,3-dimethyl-5-nitrobenzol (Bartoli et al., J. Org. Chem. 1975, 40, 872):
MS (ESI): m/z (%) = 221 (M+H, 100);
HPLC (Methode 4): rt = 0.77 min.

### IV-8. 4-(2,4-Diaminophenyl)-3-morpholinon

ausgehend von 1-Fluoro-2,4-dinitrobenzol:
MS (ESI): m/z (%) = 208 (M+H, 100);
HPLC (Methode 4): rt = 0.60 min.

### IV-9. 4-(4-Amino-2-chlorophenyl)-2-methyl-3-morpholinon

ausgehend von 2-Methyl-3-morpholinon (Pfeil, E.; Harder, U.; Angew. Chem. 1967, 79, 188):
MS (ESI): m/z (%) = 241 (M+H, 100);
HPLC (Methode 4): rt = 2.27 min.

### IV-10. 4-(4-Amino-2-chlorophenyl)-6-methyl-3-morpholinon

ausgehend von 6-Methyl-3-morpholinon (EP 350 002):
MS (ESI): m/z (%) = 241 (M+H, 100);
HPLC (Methode 4): rt = 2.43 min.

### Synthesebeispiele

Die folgenden Beispiele 1 bis 13, 17 bis 19 und 36 bis 57 beziehen sich auf die Verfahrensvariante [A].

### Beispiel 1

### Herstellung von 5-Chloro-N-{[(5S)-3-(3-fluoro-4-morpholinophenyl)-2-oxo-1,3-oxazolidin-5-yl]methyl}-2-thiophencarboxamid

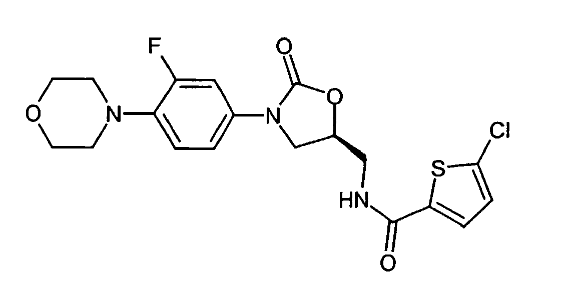

(5S)-5-(Aminomethyl)-3-(3-fluoro-4-morpholinophenyl)-1,3-oxazolidin-2-on (Herstellung siehe S. J. Brickner et al., J. Med. Chem. 1996, 39, 673) (0.45 g, 1.52 mmol), 5-Chlorthiophen-2-carbonsäure (0.25 g, 1.52 mmol) und 1-Hydroxy-1H-benzotriazol Hydrat (HOBT) (0.3 g, 1.3 Äquivalente) werden in 9.9 ml DMF gelöst. Man gibt 0.31 g (1.98 mmol, 1.3 Äquivalente) N'-(3-Dimethylaminopropyl)-N-ethylcarbodiimid (EDCI) hinzu und tropft bei Raumtemperatur 0.39 g (0.53 ml, 3.05 mmol, 2 Äquivalente) Diisopropylethylamin (DIEA) hinzu. Man rührt über Nacht bei Raumtemperatur. Man gibt 2 g Kieselgel hinzu und dampft den Ansatz im Vakuum bis zur Trockene ein. Der Rückstand wird auf Kieselgel mit einem Toluol-Essigester-Gradienten chromatographiert. Man erhält 0.412 g (61.5 % d. Th.) der Zielverbindung mit einem Schmelzpunkt (Smp.) von 197°C.
R_{f} (SiO₂, Toluol/Essigester 1:1) = 0.29 (Edukt = 0.0);
MS (DCI) 440.2 (M+H), Cl-Muster;
¹H-NMR (d₆-DMSO, 300 MHz) 2.95 (m, 4H), 3.6 (t, 2H), 3.72 (m, 4H), 3.8 (dd, 1H), 4.12 (t, 1H), 4.75-4.85 (m, 1H), 7.05 (t, 1H), 7.15-7.2 (m, 3H), 7.45 (dd, 1H), 7.68 (d, 1H), 8.95 (t, 1H).

### Beispiel 2

### 5-Chloro-N-{[(5S)-3-(4-morpholinophenyl)-2-oxo-1,3-oxazolidin-5-yl]methyl}-2-thiophencarboxamid

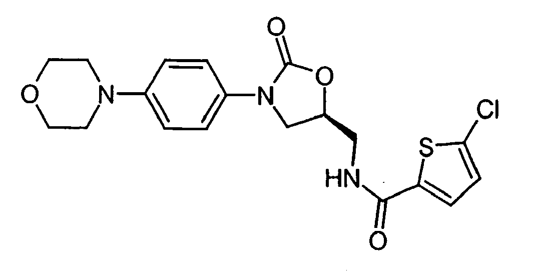

wird analog aus Benzyl-4-morpholinophenylcarbamat über die Stufe des (5S)-5-(Aminomethyl)-3-(3-fluoro-4-morpholinophenyl)-1,3-oxazolidin-2-ons (siehe Beispiel 1) erhalten.
Smp.: 198°C;
IC₅₀-Wert = 43 nM;
R_{f} (SiO₂, Toluol/Essigester 1: 1) = 0.24.

### Beispiel 3

### 5-Chloro-N-({(5S)-3-[3-fluoro-4-(1,4-thiazinan-4-yl)phenyl]-2-oxo-1,3-oxazolidin-5-yl}methyl)-2-thiophencarboxamid

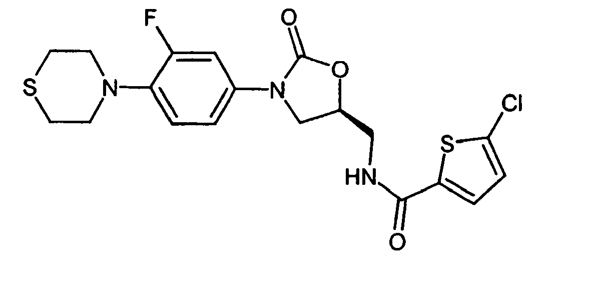

wird analog aus (5S)-5-(Aminomethyl)-3-[3-fluoro-4-(1,4-thiazinan-4-yl)phenyl]-1,3-oxazolidin-2-on (Herstellung siehe M. R. Barbachyn et al., J. Med. Chem. 1996, 39, 680) erhalten.
Smp.: 193°C;
Ausbeute: 82 %;
R_{f} (SiO₂, Toluol/Essigester 1:1) = 0.47 (Edukt = 0.0).

### Beispiel 4

### 5-Brom-N-({(5S)-3-[3-fluoro-4-(1,4-thiazinan-4-yl)phenyl]-2-oxo-1,3-oxazolidin-5-yl}methyl)-2-thiophencarboxamid

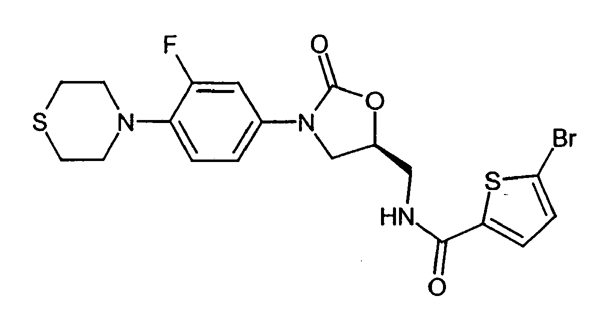

wird analog aus 5-Bromthiophen-2-carbonsäure erhalten.
Smp.: 200°C.

### Beispiel 5

### N-({(5S)-3-[3-Fluoro-4-(1,4-thiazinan-4-yl)phenyl]-2-oxo-1,3-oxazolidin-5-yl}methyl)-5-methyl-2-thiophencarboxamid

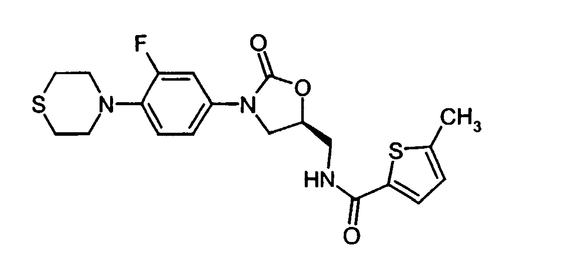

wird analog aus 5-Methylthiophen-2-carbonsäure erhalten.
Smp.: 167°C.

### Beispiel 6

### 5-Chloro-N-{[(5S)-3-(6-methylthieno[2,3-b]pyridin-2-yl)-2-oxo-1,3-oxazolidin-5-yl]methyl}-2-thiophencarboxamid

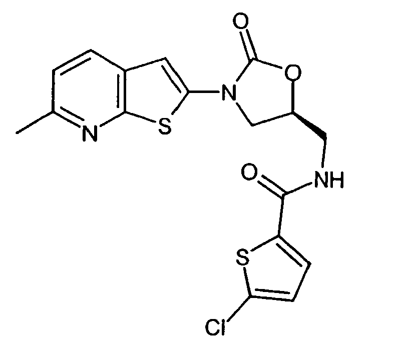

wird analog aus (5S)-5-(Aminomethyl)-3-(6-methylthieno[2,3-b]pyridin-2-yl)-1,3-oxazolidin-2-on (Herstellung siehe EP-A-785 200) erhalten.
Smp.: 247°C.

### Beispiel 7

### 5-Chloro-N-{[(5S)-3-(3-methyl-2-oxo-2,3-dihydro-1,3-benzothiazol-6-yl)-2-oxo-1,3-oxazolidin-5-yl]methyl}-2-thiophencarboxamid

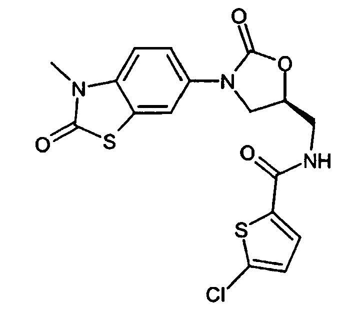

wird analog aus 6-[(5S)-5-(Aminomethyl)-2-oxo-1,3-oxazolidin-3-yl]-3-methyl-1,3-benzothiazol-2(3H)-on (Herstellung siehe EP-A-738 726) erhalten.
Smp.: 217°C.

### Beispiel 8

### 5-Chloro-N-[((5S)-3-{3-fluoro-4-[4-(4-pyridinyl)piperazino]phenyl}-2-oxo-1,3-oxazolidin-5-yl)methyl]-2-thiophencarboxamid

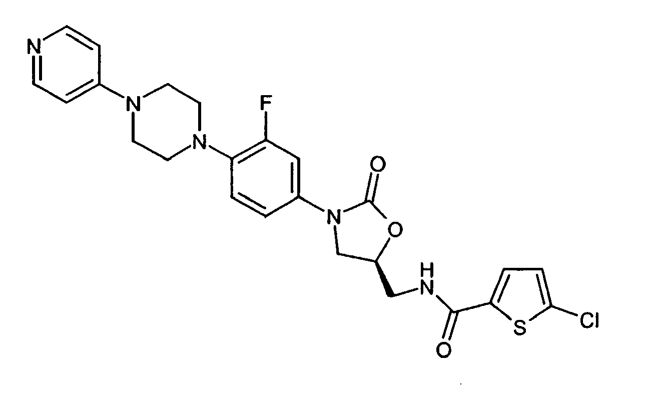

wird analog aus (5S)-5-(Aminomethyl)-3-{3-fluoro-4-[4-(4-pyridinyl)piperazino] phenyl}-1,3-oxazolidin-2-on (Herstellung analog J. A. Tucker et al., J. Med. Chem. 1998, 41, 3727) erhalten.
MS (ESI) 516 (M+H),Cl-Muster.

### Beispiel 9

### 5-Chloro-N-({(5S)-3-[3-fluoro-4-(4-methylpiperazino)phenyl]-2-oxo-1,3-oxazolidin-5-yl}methyl)-2-thiophencarboxamid

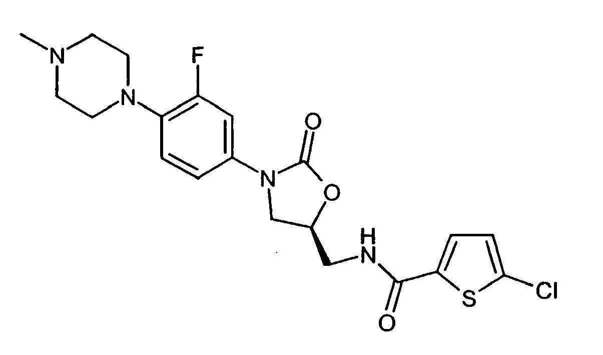

wird analog aus (5S)-5-(Aminomethyl)-3-[3-fluoro-4-(4-methylpiperazino)phenyl]-1,3-oxazolidin-2-on erhalten.

### Beispiel 10

### 5-Chloro-N-({(5S)-3-[3-fluoro-4-(4-tert-butoxycarbonylpiperazin-1-yl)phenyl]-2-oxo-1,3-oxazolidin-5-yl}methyl)-2-thiophencarboxamid

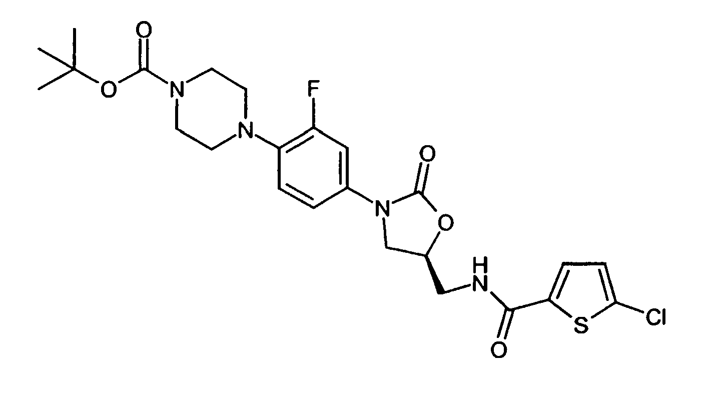

wird analog aus (5S)-5-(Aminomethyl)-3-[3-fluoro-4-(4-tert-butoxycarbonylpiperazin-1-yl)phenyl]-1,3-oxazolidin-2-on (Herstellung siehe bereits zitierte WO-A-93/23384) erhalten.
Smp.: 184°C;
R_{f} (SiO₂, Toluol/Essigester 1:1) = 0.42.

### Beispiel 11

### 5-Chloro-N-({(5S)-3-[3-fluoro-4-(piperazin-1-yl)phenyl]-2-oxo-1,3-oxazolidin-5-yl}methyl)-2-thiophencarboxamid

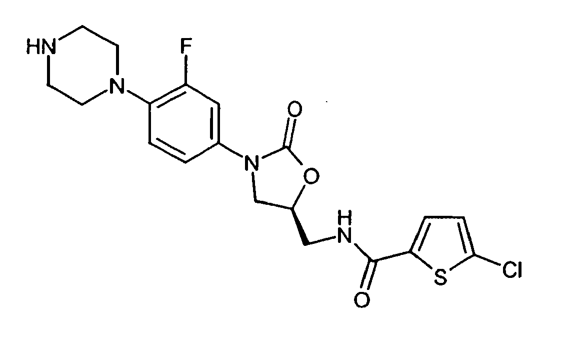

wird durch Umsetzung von Beispiel 12 mit Trifluoressigsäure in Methylenchlorid erhalten.
IC₅₀-Wert = 140 nM;
¹H-NMR [d₆-DMSO]: 3.01-3.25 (m, 8H), 3.5-3.65 (m, 2H), 3.7-3.9 (m, 1H), 4.05-4.2 (m, 1H), 4.75-4.9 (m, 1H), 7.05-7.25 (m, 3H), 7.5 (dd, 1H), 7.7 (d, 1H), 8.4 (broad s, 1H), 9.0 (t, 1H).

### Beispiel 12

### 5-Chloro-N-[((5S)-3-(2,4-bipyridinyl-5-yl)-2-oxo-1,3-oxazolidin-5-yl)methyl]-2-thiophencarboxamid

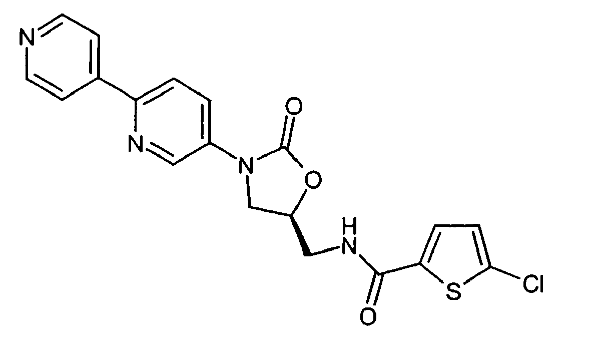

wird analog aus (5S)-5-Aminomethyl-3-(2,4'-bipyridinyl-5-yl)-2-oxo-1,3-oxazolidin-2-on (Herstellung siehe EP-A-789 026) erhalten.
R_{f} (SiO₂, Essigester/Ethanol 1:2) = 0.6;
MS (ESI) 515 (M+H), Cl-Muster.

### Beispiel 13

### 5-Chloro-N-{[(5S)-2-oxo-3-(4-piperidinophenyl)-1,3-oxazolidin-5-yl]methyl}-2-thiophencarboxamid

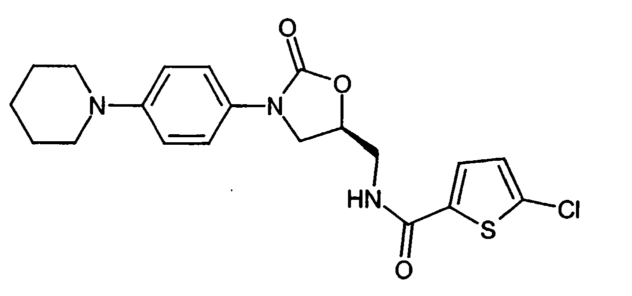

wird aus 5-(Hydroxymethyl)-3-(4-piperidinophenyl)-1,3-oxazolidin-2-on (Herstellung siehe DE 2708236) nach Mesylierung, Umsetzung mit Phthalimidkalium, Hydrazinolyse und Reaktion mit 5-Chlorthiophen-2-carbonsäure erhalten.
R_{f} (SiO₂, Essigester/Toluol 1:1) = 0.31;
Smp. 205°C.

### Beispiel 17

### 5-Chloro-N-({(5S)-2-oxo-3-[4-(2-oxo-1-pyrrolidinyl)phenyl]-1,3-oxazolidin-5-yl}methyl)-2-thiophencarboxamid

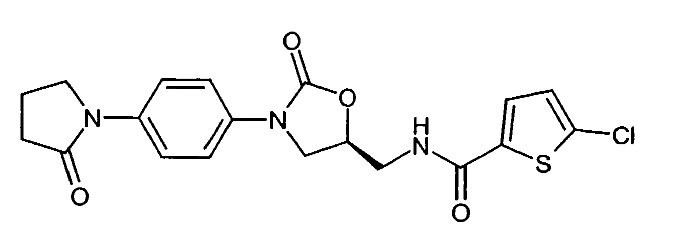

Aus 1-(4-Aminophenyl)pyrrolidin-2-on (Herstellung siehe Reppe et al., Justus Liebigs Ann. Chem.; 596; 1955; 209) erhält man in Analogie zu dem bekannten Syntheseschema (siehe S.J. Brickner et al., J. Med. Chem. 1996, 39, 673) nach Umsetzung mit Benzyloxycarbonylchlorid, anschließender Reaktion mit *R*-Glycidylbutyrat, Mesylierung, Umsetzung mit Phthalimidkalium, Hydrazinolyse in Methanol und Reaktion mit 5-Chlorthiophen-2-carbonsäure schließlich das 5-Chloro-N-({(5S)-2-oxo-3-[4-(2-oxo-1-pyrrolidinyl)phenyl]-1,3-oxazolidin-5-yl}methyl)-2-thiophencarboxamid. Das auf diese Weise erhaltene 5-Chloro-N-({(5S)-2-oxo-3-[4-(2-oxo-1-pyrrolidinyl)phenyl]-1,3-oxazolidin-5-yl}methyl)-2-thiophencarboxamid weist einen Wert IC₅₀= 4 nM auf (Testmethode für den IC₅₀-Wert gemäß zuvor beschriebenem Beispiel A-1. a.1) "Messung der Faktor Xa-Hemmung").
Smp.: 229°C;
R_{f}-Wert (SiO₂, Toluol/Essigester 1:1) = 0.05 (Edukt: = 0.0);
MS (ESI): 442.0 (21%, M+Na, Cl-Muster), 420.0 (72%, M+H, Cl-Muster), 302.3 (12%), 215(52%), 145 (100%);
¹H-NMR (d₆-DMSO, 300 MHz): 2.05 (m,2H), 2.45 (m,2H), 3.6 (t,2H), 3.77-3.85 (m,3H), 4.15(t,1H), 4.75-4.85 (m,1H), 7.2 (d,1H), 7.5 (d,2H), 7.65 (d,2H), 7.69 (d,1H), 8.96 (t,1H).

Die einzelnen Stufen der zuvor beschriebenen Synthese von Beispiel 17 mit den jeweiligen Vorstufen sind wie folgt:

4 g (22.7 mmol) 1-(4-Aminophenyl)pyrrolidin-2-on und 3.6 ml (28.4 mmol) N,N-Dimethylanilin werden in 107 ml Tetrahydrofuran bei -20°C langsam mit 4.27 g (25.03 mmol) Chlorameisensäurebenzylester versetzt. Man rührt 30 Minuten bei -20°C und lässt das Ganze anschließend auf Raumtemperatur kommen. Man gibt 0.5 1 Essigester hinzu und wäscht die organische Phase mit 0.5 1 gesättigter NaCl-Lösung. Man trocknet die abgetrennte organische Phase mit MgSO₄ und verdampft das Lösungsmittel im Vakuum. Der Rückstand wird mit Diethylether verrieben und abgesaugt. Man erhält 5.2 g (73.8 % d.Th.) Benzyl-4-(2-oxo-1-pyrrolidinyl)phenyl-carbamat als helle beige Kristalle mit einem Schmelzpunkt von 174°C.

Man versetzt 1.47 g (16.66 mmol) Isoamylalkohol in 200 ml Tetrahydrofuran unter Argon bei -10°C tropfenweise mit 7.27 ml einer 2.5 M Lösung von n-Butyllithium (BuLi) in Hexan, wobei weitere 8 ml der BuLi-Lösung bis zum Umschlag des hinzugesetzten Indikators N-Benzylidenbenzylamin notwendig waren. Man rührt 10 Minuten bei -10°C, kühlt auf -78°C ab und gibt langsam eine Lösung von 4.7 g (15.14 mmol) Benzyl-4-(2-oxo-1-pyrrolidinyl)phenylcarbamat hinzu. Anschließend gibt man nochmals bis zum Farbumschlag des Indikators nach rosa 4 ml n-BuLi-Lösung hinzu. Man rührt 10 Minuten bei -78°C und gibt 2.62 g (18.17 mmol) R-Glycidylbutyrat hinzu und rührt 30 Minuten bei -78°C nach.

Man lässt das Ganze über Nacht auf Raumtemperatur kommen, gibt zu dem Ansatz 200 ml Wasser und verdampft den THF-Anteil im Vakuum. Der wässrige Rückstand wird mit Essigester extrahiert, die organische Phase mit MgSO₄ getrocknet und im Vakuum eingedampft. Man verreibt den Rückstand mit 500 ml Diethylether und saugt die ausgefallenen Kristalle im Vakuum ab.

Man erhält 3.76 g (90 % d.Th.) (5R)-5-(Hydroxymethyl)-3-[4-(2-oxo-1-pyrrolidinyl)phenyl]-1,3-oxazolidin-2-on mit einem Schmelzpunkt von 148°C und einem R_{f}-Wert (SiO₂, Toluol/Essigester 1:1) = 0.04 (Edukt = 0.3).

3.6 g (13.03 mmol) (5R)-5-(Hydroxymethyl)-3-[4-(2-oxo-1-pyrrolidinyl)phenyl]-1,3-oxazolidin-2-on und 2.9 g (28.67 mmol) Triethylamin werden in 160 ml Dichlormethan bei 0°C unter Rühren vorgelegt. Man gibt 1.79 g (15.64 mmol) Methansulfonsäurechlorid unter Rühren hinzu und rührt 1.5 Stunden bei 0°C sowie 3 h bei Raumtemperatur.

Das Reaktionsgemisch wird mit Wasser gewaschen und die wässrige Phase nochmals mit Methylenchlorid extrahiert. Die vereinigten organischen Extrakte werden mit MgSO₄ getrocknet und eingedampft. Anschließend wird der Rückstand (1.67 g) in 70 ml Acetonitril gelöst, mit 2.62 g (14.16 mmol) Phthalimidkalium versetzt und in einem geschlossenen Gefäß in einem Mikrowellenofen 45 Minuten lang bei 180°C gerührt.

Der Ansatz wird von unlöslichem Rückstand abfiltriert, das Filtrat im Vakuum eingedampft, der Rückstand (1.9 g) in Methanol gelöst und mit 0.47 g (9.37 mmol) Hydrazinhydrat versetzt. Man kocht 2 Stunden, kühlt ab, versetzt mit gesättigter Natriumbicarbonatlösung und extrahiert sechsmal mit insgesamt 2 1 Methylenchlorid. Die vereinigten organischen Extrakte des rohen (5S)-5-(Aminomethyl)-3-[4-(2-oxo-1-pyrrolidinyl)phenyl]-1,3-oxazolidin-2-on werden mit MgSO₄ getrocknet und im Vakuum eingedampft.

Die Endstufe, das 5-Chloro-N-({(5S)-2-oxo-3-[4-(2-oxo-1-pyrrolidinyl)phenyl]-1,3-oxazolidin-5-yl}methyl)-2-thiophencarboxamid, wird hergestellt, indem 0.32 g (1.16 mmol) des oben dargestellten (5S)-5-(Aminomethyl)-3-[4-(2-oxo-1-pyrrolidinyl)phenyl]-1,3-oxazolidin-2-ons, 5-Chlorthiophen-2-carbonsäure (0.19 g; 1.16 mmol) und 1-Hydroxy-1H-benzotriazol-Hydrat (HOBT) (0.23 g, 1.51 mmol) in 7.6 ml DMF gelöst werden. Man gibt 0.29 g (1.51 mmol) N'-(3-Dimethylaminopropyl)-N-ethylcarbodiimid (EDCI) hinzu und tropft bei Raumtemperatur 0.3 g (0.4 ml; 2.32 mmol, 2 Äquivalente) Diisopropylethylamin (DIEA) hinzu. Man rührt über Nacht bei Raumtemperatur.

Man dampft den Ansatz im Vakuum zur Trockene ein, löst den Rückstand in 3 ml DMSO und chromatographiert auf einer RP-MPLC mit Acetonitril/Wasser/0.5 % TFA-Gradienten. Aus den passenden Fraktionen dampft man den Acetonitrilanteil ab und saugt die ausgefallene Verbindung ab. Man erhält 0.19 g (39 % d. Th.) der Zielverbindung.

Auf analoge Weise wurden hergestellt:

### Beispiel 18

### 5-Chloro-N-({(5S)-2-oxo-3-[4-(1-pyrrolidinyl)phenyl]-1,3-oxazolidin-5-yl}methyl)-2-thiophencarboxamid

Analog zu Beispiel 17 erhält man aus 4-Pyrrolidin-1-yl-anilin (Reppe et al., Justus Liebigs Ann. Chem.; 596; 1955; 151) die Verbindung 5-Chloro-N-({(5S)-2-oxo-3-[4-(1-pyrrolidinyl)phenyl]-1,3-oxazolidin-5-yl}methyl)-2-thiophencarboxamid. IC₅₀=40 nM;
Smp.: 216°C;
R_{f}-Wert (SiO₂, Toluol/Essigester 1: 1) = 0.31 [Edukt: = 0.0].

### Beispiel 19

### 5-Chloro-N-({(5S)-2-oxo-3-[4-(diethylamino)phenyl]-1,3-oxazolidin-5-yl}methyl)-2-thiophencarboxamid

Analog erhält man aus N,N-Diethylphenyl-1,4-diamin (US-A-2 811 555; 1955) die Verbindung 5-Chloro-N-({(5S)-2-oxo-3-[4-(diethylamino)phenyl]-1,3-oxazolidin-5-yl} methyl)-2-thiophencarboxamid.
IC₅₀=270 nM;
Smp.: 181 °C;
R_{f}-Wert (SiO₂, Toluol/Essigester 1:1) = 0.25 [Edukt: = 0.0].

### Beispiel 36

### 5-Chloro-N-({(5S)-3-[2-methyl-4-(4-morpholinyl)phenyl]-2-oxo-1,3-oxazolidin-5-yl}methyl)-2-thiophencarboxamid

ausgehend von 2-Methyl-4-(4-morpholinyl)anilin (J.E.LuValle et al. J.Am.Chem.Soc. 1948, 70, 2223):
MS (ESI): m/z (%) = 436 ([M+H]⁺, 100), Cl-Muster;
HPLC (Methode 1): rt (%) = 3.77 (98).
IC₅₀: 1.26 µM

### Beispiel 37

### 5-Chloro-N-{[(5S)-3-(3-chloro-4-morpholinophenyl)-2-oxo-1,3-oxazolidin-5-yl]methyl}-2-thiophencarboxamid

ausgehend von 3-Chloro-4-(4-morpholinyl)anilin (H.R.Snyder et al. J.Pharm.Sci. 1977, 66, 1204):
MS (ESI): m/z (%) = 456 ([M+H]⁺, 100), Cl₂-Muster;
HPLC (Methode 2): rt (%) = 4.31 (100).
IC₅₀: 33 nM

### Beispiel 38

### 5-Chloro-N-({(5S)-3-[4-(4-morpholinylsulfonyl)phenyl]-2-oxo-1,3-oxazolidin-5-yl}methyl)-2-thiophencarboxamid

ausgehend von 4-(4-Morpholinylsulfonyl)anilin (Adams et al. J.Am.Chem.Soc. 1939, 61, 2342):
MS (ESI): m/z (%) = 486 ([M+H]⁺, 100), Cl-Muster;
HPLC (Methode 3): rt (%) = 4.07 (100).
IC₅₀: 2 µM

### Beispiel 39

### 5-Chloro-N-({(5S)-3-[4-(1-azetidinylsulfonyl)phenyl]-2-oxo-1,3-oxazolidin-5-yl}methyl)-2-thiophencarboxamid

ausgehend von 4-(1-Azetidinylsulfonyl)anilin:
MS (DCI, NH₃): m/z (%) = 473 ([M+NH₄]⁺, 100), Cl-Muster;
HPLC (Methode 3): rt (%) = 4.10 (100).
IC₅₀: 0.84 µM

### Beispiel 40

### 5-Chloro-N-[((5S)-3-{4-[(dimethylamino)sulfonyl]phenyl}-2-oxo-1,3-oxazolidin-5-yl)methyl]-2-thiophencarboxamid

ausgehend von 4-Amino-*N*,*N*-dimethylbenzolsulfonamid (LK.Khanna et al. J.Med.Chem. 1997, 40, 1619):
MS (ESI): m/z (%) = 444 ([M+H]⁺, 100), Cl-Muster;
HPLC (Methode 3): rt (%) = 4.22 (100).
IC₅₀: 90 nM

### Allgemeine Methode zur Acylierung von 5-(Aminomethyl)-3-[4-(2-oxo-1-pyrrolidinyl)phenyl]-1,3-oxazolidin-2-on mit Carbonsäurechloriden.

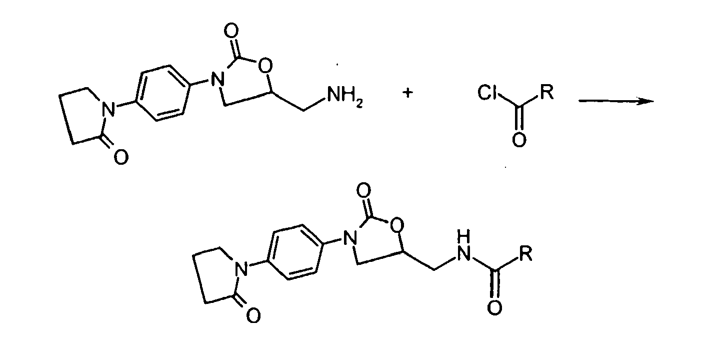

Zu dem entsprechendem Säurechlorid (2.5 eq.) wird unter Argon bei Raumtemperatur eine ca. 0.1 molare Lösung von 5-(Aminomethyl)-3-[4-(2-oxo-1-pyrrolidinyl)phenyl]-1,3-oxazolidin-2-on (aus Beispiel 45) (1.0 eq.) und absolutem Pyridin (ca. 6 eq) in absolutem Dichlormethan getropft. Die Mischung wird ca. 4 h bei Raumtemperatur gerührt, bevor ca. 5.5 eq PS-Trisamine (Argonaut Technologies) zugesetzt werden. Die Suspension wird 2 h leicht gerührt, nach Verdünnen mit Dichlormethan/DMF (3:1) filtriert (das Harz wird mit Dichlormethan/DMF gewaschen) und das Filtrat eingeengt. Das erhaltene Produkt wird gegebenenfalls durch präparative RP-HPLC gereinigt.

Auf analoge Weise wurde hergestellt:

### Beispiel 41

### N-({2-oxo-3-[4-(2-oxo-1-pyrrolidinyl)phenyl]-1,3-oxazolidin-5-yl}methyl)-2-thiophen-carboxamid

LC-MS (Methode 6): m/z (%) = 386 (M+H, 100);
LC-MS: rt (%) = 3.04 (100).
IC₅₀: 1.3 µM

### Allgemeine Methode zur Darstellung von Acylderivaten ausgehend von 5-(Aminomethyl)-3-[4-(2-oxo-1-pyrrolidinyl)phenyl]-1,3-oxazolidin-2-on und Carbonsäuren

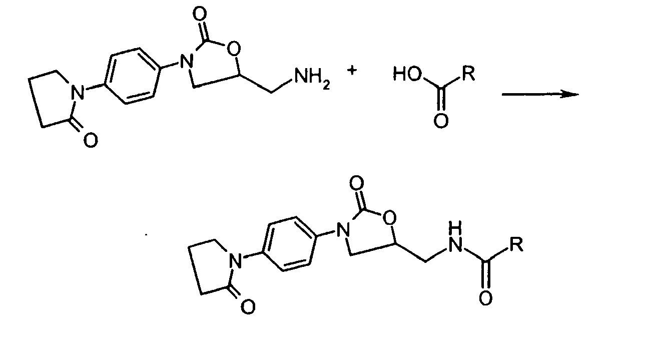

Zu 2.9 eq. harzgebundenem Carbodiimid (PS-Carbodiimid, Argonaut Technologies) werden entsprechende Carbonsäure (ca. 2 eq) und eine Mischung aus absolutem Dichlormethan/DMF (ca. 9:1) gegeben. Nach ca. 15 min leichtem Schütteln bei Raumtemperatur wird 5-(Aminomethyl)-3-[4-(2-oxo-1-pyrrolidinyl)phenyl]-1,3-oxazolidin-2-on (aus Beispiel 45) (1.0 eq.) hinzugesetzt und die Mischung über Nacht geschüttelt, bevor vom Harz abfiltriert (nachgewaschen mit Dichlormethan) und das Filtrat eingeengt wird. Das erhaltene Produkt wird gegebenenfalls durch präparative RP-HPLC gereinigt.

Auf analoge Weise wurden hergestellt:

### Beispiel 42

### 5-Methyl-N-({2-oxo-3-[4-(2-oxo-1-pyrrolidinyl)phenyl]-1,3-oxazolidin-5-yl}methyl)-2-thiophencarboxamid

LC-MS: m/z (%) = 400 (M+H, 100);
LC-MS (Methode 6): rt (%) = 3.23 (100).
IC₅₀: 0.16 µM

### Beispiel 43

### 5-Bromo-N-({2-oxo-3-[4-(2-oxo-1-pyrrolidinyl)phenyl]-1,3-oxazolidin-5-yl}methyl)-2-thiophencarboxamid

LC-MS : m/z (%) = 466 (M+H, 100);
LC-MS (Methode 5): rt (%) = 3.48 (78).
IC₅₀: 0.014 µM

### Beispiel 44

### 5-Chloro-N-({(5S)-2-oxo-3-[4-(3-oxo-4-morpholinyl)phenyl]-1,3-oxazolidin-5-yl}methyl)-2-thiophencarboxamid

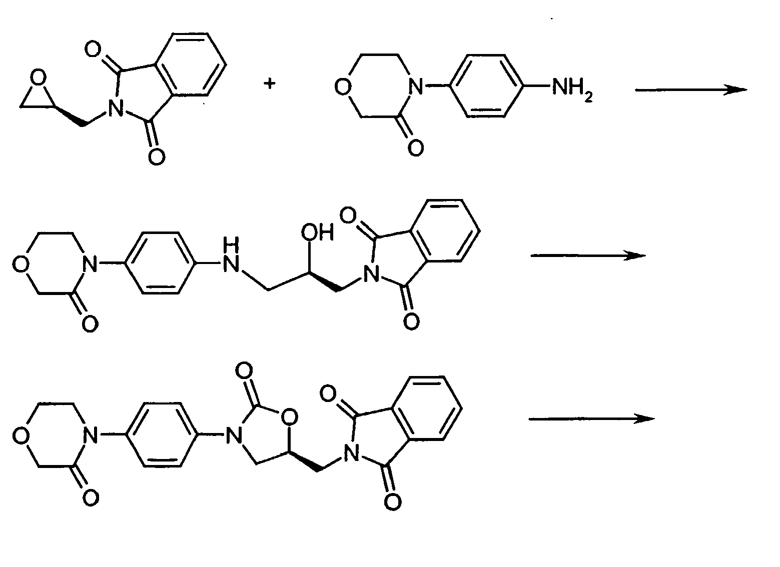

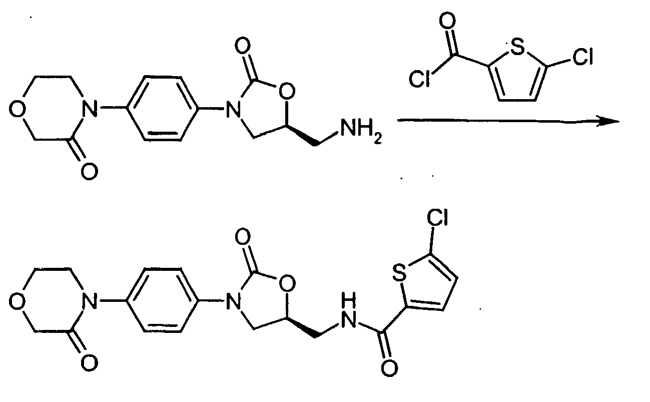

### a) 2-((2R)-2-Hydroxy-3-{[4-(3-oxo-4-morpholinyl)phenyl]amino}propyl)-1H-iso-indol-1,3(2H)-dion:

Eine Suspension von 2-[(2*S*)-2-Oxiranylmethyl]-1*H*-isoindol-1,3(2*H*)-dion (A. Gutcait et al. Tetrahedron Asym. 1996, 7, 1641) (5.68 g, 27.9 mmol) und 4-(4-Aminophenyl)-3-morpholinon (5.37 g, 27.9 mmol) in Ethanol-Wasser (9:1, 140 ml) wird für 14 h refluxiert (der Niederschlag geht in Lösung, nach einiger Zeit erneute Bildung eines Niederschlages). Der Niederschlag (gewünschtes Produkt) wird abfiltriert, dreimal mit Diethylether gewaschen und getrocknet. Die vereinigten Mutterlaugen werden im Vakuum eingeengt und nach Zugabe einer zweiten Portion 2-[(2*S*)-2-Oxiranylmethyl]-1*H*-isoindol-1,3(2*H*)-dion (2.84 g, 14.0 mmol) in Ethanol-Wasser (9:1, 70 ml) suspendiert und für 13 h refluxiert (der Niederschlag geht in Lösung, nach einiger Zeit erneute Bildung eines Niederschlages). Der Niederschlag (gewünschtes Produkt) wird abfiltriert, dreimal mit Diethylether gewaschen und getrocknet. Gesamtausbeute : 10.14 g, 92 % der Theorie.
MS (ESI): m/z (%) = 418 ([M+Na]⁺, 84), 396 ([M+H]⁺, 93);
HPLC (Methode 3): rt (%) = 3.34 (100).

### b) 2-({(5S)-2-Oxo-3-[4-(3-oxo-4-morpholinyl)phenyl]-1,3-oxazolidin-5-yl}methyl)-1H-isoindol-1,3(2H)-dion:

Zu einer Suspension des Aminoalkohols (3.58 g, 9.05 mmol) in Tetrahydrofuran (90 ml) wird unter Argon bei Raumtemperatur *N*,*N*'-Carbonyldiimidazol (2.94 g, 18.1 mmol) und Dimethylaminopyridin (katalytische Menge) gegeben. Die Reaktionssuspension wird bei 60°C für 12 h gerührt (der Niederschlag geht in Lösung, nach einiger Zeit erneute Bildung eines Niederschlages), mit einer zweiten Portion *N*,*N*'-Carbonyldiimidazol (2.94 g, 18.1 mmol) versetzt und weitere 12 h bei 60°C gerührt. Der Niederschlag (gewünschtes Produkt) wird abfiltriert, mit Tetrahydrofuran gewaschen und getrocknet. Das Filtrat wird im Vakuum eingeengt und weiteres Produkt mittels Flash-Chromatographie (Dichlormethan-Methanol-Gemische) gereinigt. Gesamtausbeute: 3.32 g, 87 % der Theorie.
MS (ESI): m/z (%) = 422 ([M+H]⁺, 100);
HPLC (Methode 4): rt (%) = 3.37 (100).

### c) 5-Chloro-N-({(5S)-2-oxo-3-[4-(3-oxo-4-morpholinyl)phenyl]-1,3-oxazolidin-5-yl}methyl)-2-thiophencarboxamid:

Zu einer Suspension des Oxazolidinons (4.45 g, 10.6 mmol) in Ethanol (102 ml) wird bei Raumtemperatur tropfenweise Methylamin (40%ig in Wasser, 10.2 ml, 0.142 mol) gegeben. Die Reaktionsmischung wird für 1 h refluxiert und im Vakuum eingeengt. Das Rohprodukt wird ohne weitere Reinigung in die nächste Reaktion eingesetzt.

Zu einer Lösung des Amins in Pyridin (90 ml) wird unter Argon bei 0°C 5-Chlorthiophen-2-carbonsäurechlorid (2.29 g, 12.7 mmol) getropft. Die Eiskühlung wird entfernt und das Reaktionsgemisch 1 h bei Raumtemperatur gerührt und mit Wasser versetzt. Nach Zugabe von Dichlormethan und Phasentrennung wird die wässrige Phase mit Dichlormethan extrahiert. Die vereinigten organischen Phasen werden getrocknet (Natriumsulfat), filtriert und im Vakuum eingeengt. Das gewünschte Produkt wird mittels Flash-Chromatographie (Dichlormethan-Methanol-Gemische) gereinigt. Gesamtausbeute: 3.92 g, 86 % der Theorie.
Smp: 232-233°C;
¹H NMR (DMSO-d⁶, 200 MHz): 9.05-8.90 (t, *J* = 5.8 Hz, 1H), 7.70 (d, *J* = 4.1 Hz, 1H), 7.56 (d, *J*= 9.0 Hz, 2H), 7.41 (d, *J=* 9.0 Hz, 2H), 7.20 (d, *J=* 4.1 Hz, 1H), 4.93-4.75 (m, 1H), 4.27-4.12 (m, 3H), 4.02-3.91 (m, 2H), 3.91-3.79 (dd, *J*= 6.1 Hz, 9.2 Hz, 1H), 3.76-3.66 (m, 2H), 3.66-3.54 (m, 2H);
MS (ESI): m/z (%) = 436 ([M+H]⁺, 100, Cl-Muster);
HPLC (Methode 2): rt (%) = 3.60 (100);
[α]²¹_{D} = -38° (c 0.2985, DMSO); ee: 99 %.
IC₅₀: 0.7 nM

Auf analoge Weise wurden hergestellt:

### Beispiel 45

### 5-Methyl-N-({(5S)-2-oxo-3-[4-(3-oxo-4-morpholinyl)phenyl]-1,3-oxazolidin-5-yl}methyl)-2-thiophencarboxamid

MS (ESI): m/z (%) = 831 ([2M+H]⁺, 100), 416 ([M+H]⁺, 66);
HPLC (Methode 3): rt (%) = 3.65 (100).
IC₅₀: 4.2 nM

### Beispiel 46

### 5-Bromo-N-({(5S)-2-oxo-3-[4-(3-oxo-4-morpholinyl)phenyl]-1,3-oxazolidin-5-yl}methyl)-2-thiophencarboxamid

MS (ESI): m/z (%) = 480 ([M+H]⁺, 100, Br-Muster);
HPLC (Methode 3): rt (%) = 3.87 (100).
IC₅₀: 0.3 nM

### Beispiel 47

### 5-Chloro-N-{[(5S)-3-(3-isopropyl-2-oxo-2,3-dihydro-1,3-benzoxazol-6-yl)-2-oxo-1,3-oxazolidin-5-yl]methyl}-2-thiophencarboxamid

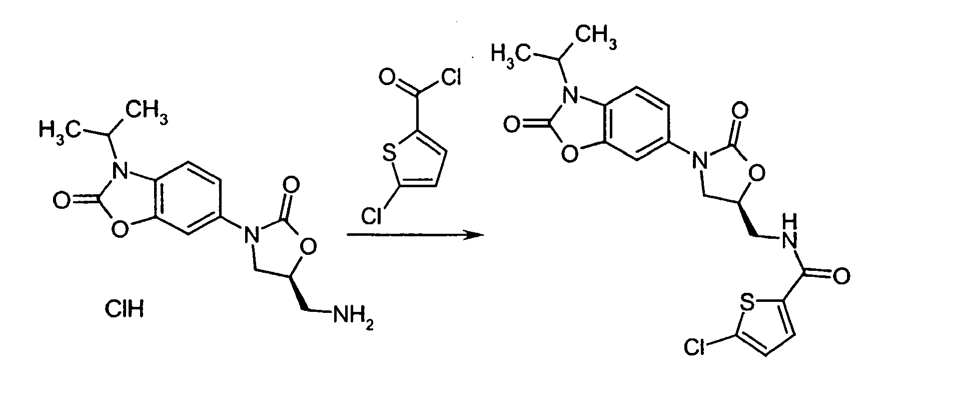

200 mg (0.61 mmol) 6-[(5S)-5-(Aminomethyl)-2-oxo-1,3-oxazolidin-3-yl]-3-isopropyl-1,3-benzoxazo1-2(3H)-on Hydrochlorid (EP 738726) werden in 5 ml Tetrahydrofuran suspendiert und mit 0.26 ml (1.83 mmol) Triethylamin und 132 mg (0.73 mmol) 5-Chlorthiophen-2-carbonsäurechlorid versetzt. Das Reaktionsgemisch wird über Nacht bei Raumtemperatur gerührt und anschließend eingeengt. Das Produkt wird durch Säulenchromatographie (Kieselgel, Methylenchlorid/Ethanol = 50/1 bis 20/1) isoliert. Es werden 115 mg (43% d. Th.) der gewünschten Verbindung erhalten. MS (ESI): m/z (%) = 436 (M+H, 100);
HPLC (Methode 4): rt = 3.78 min.

In analoger Weise wurden die folgenden Verbindungen hergestellt:

| Beispiel-Nr. | Struktur | Smp. [°C] | IC₅₀ [µM] |
|---|---|---|---|
| 48 | 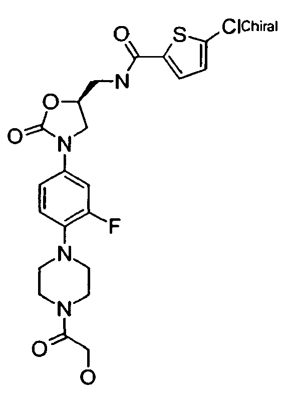 | 210 | 0,12 |
| 49 | 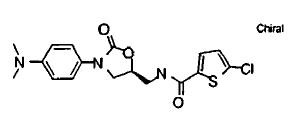 | 234 | 0,074 |
| 50 | 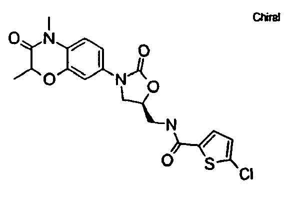 | 195 | 1,15 |
| 51 | 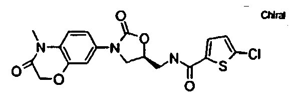 | 212 | 1,19 |
| 52 | 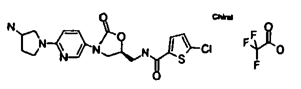 | 160 | 0,19 |
| 53 | 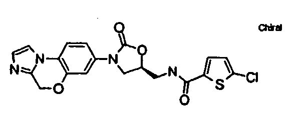 | MS (ESI): m/z (%) = 431 | 0,74 |
| | | ([M+H]⁺, 100), Cl-Muster | |
| 54 | 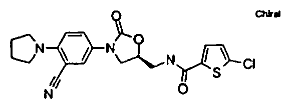 | 221 | 0,13 |
| | aus 5-Amino-2-pyrrolidino-benzonitril (Grell, W.,Hurnaus, R.; Griss, G.,Sauter, R.; Rupprecht, E. et al.; J.Med.Chem.1998, 41; 5219) | | |
| 55 | 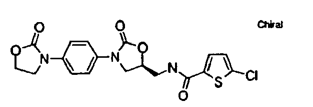 | 256 | 0,04 |
| | aus 3-(4-Amino-phenyl)-oxazolidin-2-on (Artico,M. et al.; Farmaco Ed.Sci. 1969, 24; 179) | | |
| 56 | 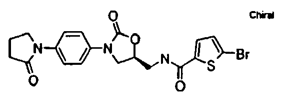 | 218 | 0,004 |
| 57 | 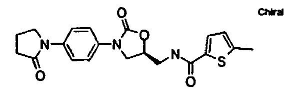 | 226 | 0,58 |
| 58 | 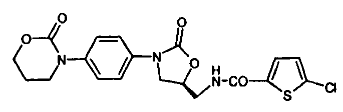 | 228-230 | |

Die folgenden Beispiele 20 bis 30 und 58 bis 139 beziehen sich auf die Verfahrensvariante [B], wobei die Beispiele 20 und 21 die Darstellung von Vorstufen beschreiben.

### Beispiel 20

### Darstellung von N-Allyl-5-chloro-2-thiophencarboxamid

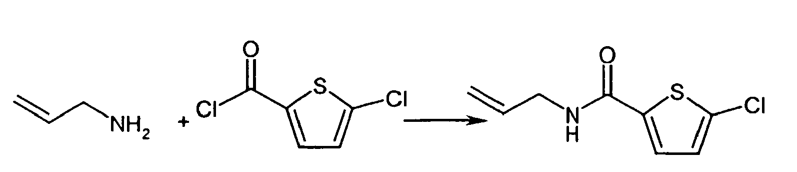

Zu einer eisgekühlten Lösung von 2.63 ml (35 mmol) Allylamin in 14.2 ml absolutem Pyridin und 14.2 ml absolutem THF wird 5-Chlor-thiophen-2-carbonsäurechlorid (7.61 g , 42 mmol) getropft. Die Eiskühlung wird entfernt und die Mischung 3 h bei Raumtemperatur gerührt, bevor im Vakuum eingeengt wird. Der Rückstand wird mit Wasser versetzt und der Feststoff abfiltriert. Das Rohprodukt wird durch Flashchromatographie an Silicagel (Dichlormethan) gereinigt.
Ausbeute: 7.20 g (99 % der Theorie);
MS (DCI, NH₄): m/z (%) = 219 (M+NH₄, 100), 202 (M+H, 32);
HPLC (Methode 1): rt (%) = 3.96 min (98.9).

### Beispiel 21

### Darstellung von 5-Chloro-N-(2-oxiranylmethyl)-2-thiophencarboxamid

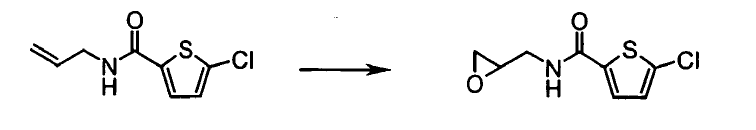

Eine eisgekühlte Lösung von 2.0 g (9.92 mmol) *N*-Allyl-5-chloro-2-thiophencarboxamid in 10 ml Dichlormethan wird mit meta-Chlorperbenzoesäure (3.83 g, ca. 60 %ig) versetzt. Die Mischung wird über Nacht gerührt, dabei Erwärmung auf Raumtemperatur, und anschließend mit 10% Natriumhydrogensulfat-Lösung gewaschen (dreimal). Die organische Phase wird mit gesättigter Natriumhydrogencarbonat-Lösung (zweimal) und mit gesättigter Natriumchlorid-Lösung gewaschen, über Magnesiumsulfat getrocknet und eingeengt. Das Produkt wird mittels Chromatographie an Silicagel (Cyclohexan/Essigester 1:1) gereinigt.
Ausbeute: 837 mg (39 % der Theorie);
MS (DCI, NH₄): m/z (%) =253 (M+NH₄, 100), 218 (M+H, 80);
HPLC (Methode 1): rt (%) = 3.69 min (ca. 80).

### Allgemeine Methode zu Darstellung von substituierten N-(3-Amino-2-hydroxy-propyl)-5-chloro-2-thiophencarboxamid-Derivaten ausgehend von 5-Chloro-N-(2-oxiranylmethyl)-2-thiophencarboxamid

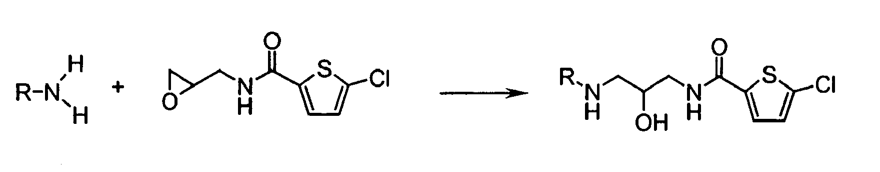

Zu einer Lösung von primärem Amin- oder Anilin-Derivat (1.5 bis 2.5 eq.) in 1,4-Dioxan, 1,4-Dioxan-Wasser Gemischen oder Ethanol, Ethanol-Wasser Gemischen (ca. 0.3 bis 1.0 mol/l) wird bei Raumtemperatur oder bei Temperaturen bis zu 80°C portionsweise 5-Chloro-*N*-(2-oxiranylmethyl)-2-thiophencarboxamid (1.0 eq.) gegeben. Die Mischung wird 2 bis 6 Stunden gerührt, bevor eingeengt wird. Aus dem Reaktionsgemisch kann das Produkt durch Chromatographie an Silicagel (Cyclohexan-Essigester-Gemische, Dichlormethan-Methanol-Gemische oder Dichlormethan-Methanol-Triethylamin-Gemische) isoliert werden.

Auf analoge Weise wurden hergestellt:

### Beispiel 22

### N-[3-(Benzylamino)-2-hydroxypropyl]-5-chloro-2-thiophencarboxamid

MS (ESI): m/z (%) = 325 (M+H, 100);
HPLC (Methode 1): rt (%) = 3.87 min (97.9).

### Beispiel 23

### 5-Chloro-N-[3-(3-cyanoanilino)-2-hydroxypropyl]-2-thiophencarboxamid

MS (ESI): m/z (%) = 336 (M+H, 100);
HPLC (Methode 2): rt (%) = 4.04 min (100).

### Beispiel 24

### 5-Chloro-N-[3-(4-cyanoanilino)-2-hydroxypropyl]-2-thiopheacarboxamid

MS (ESI): m/z (%) = 336 (M+H, 100);
HPLC (Methode 1): rt (%) = 4.12 min (100).

### Beispiel 25

### 5-Chloro-N-{3-[4-(cyanomethyl)anilino]-2-hydroxypropyl}-2-thiophencarboxamid

MS (ESI): m/z (%) = 350 (M+H, 100);
HPLC (Methode 4): rt (%) = 3.60 min (95.4).

### Beispiel 26

### 5-Chloro-N-{3-[3-(cyanomethyl)anilino]-2-hydroxypropyl}-2-thiophencarboxamid

MS (ESI): m/z (%) = 350 (M+H, 100);
HPLC (Methode 4): rt (%) = 3.76 min (94.2).

### Beispiel 58

### tert-Butyl-4-[(3-{[(5-chloro-2-thienyl)carbonyl]amino}-2-hydroxypropyl)amino]-benzylcarbamat

Ausgehend von *tert*-Butyl-4-aminobenzylcarbamat *(*Bioorg. Med. Chem. Lett.; 1997; 1921-1926):
MS (ES-pos): m/z (%) = 440 (M+H, 100), (ES-neg): m/z (%) = 438 (M-H, 100);
HPLC (Methode 1): rt (%) = 4.08 (100).

### Beispiel 59

### tert-Butyl-4-[(3-{[(5-chloro-2-thienyl)carbonyl]amino}-2-hydroxypropyl)amino]-phenyl-carbamat

Ausgehend von *N-tert-*Butyloxycarbonyl-1,4-phenylendiamin:
MS (ESI): m/z (%) = 426 (M+H, 45), 370 (100);
HPLC (Methode 1): rt (%) = 4.06 (100).

### Beispiel 60

### tert-Butyl-2-hydroxy-3-{[4-(2-oxo-1-pyrrolidinyl)phenyl]amino}propyl-carbamat

Ausgehend von 1-(4-Aminophenyl)-2-pyrrolidinon *(*Justus Liebigs Ann. Chem.; 1955; 596; 204):
MS (DCI, NH₃): m/z (%) = 350 (M+H, 100);
HPLC (Methode 1): rt (%) = 3.57 (97).

### Beispiel 61

### 5-Chloro-N-(3-{[3-fluoro-4-(3-oxo-4-morpholinyl)phenyl]amino}-2-hydroxypropyl)-2-thiophencarboxamid

800 mg (3.8 mmol) 4-(4-amino-2-fluorophenyl)-3-morpholinon und 700 mg (3.22 mmol) 5-chloro-N-(2-oxiranylmethyl)-2-thiophencarboxamid werden in 15 ml Ethanol und 1 ml Wasser 6 Stunden lang unter Rückfluss erhitzt. Man dampft im Vakuum ein, saugt von ausgefallenen Kristallen nach Behandeln mit Essigester ab und erhält durch Chromatographie der Mutterlauge 276 mg (17 % d. Th.) der Zielverbindung.
R_{f} (Essigester): 0.25.

### Beispiel 62

### (N-(3-Anilino-2-hydroxypropyl)-5-chloro-2-thiophencarboxamid

ausgehend von Anilin:
MS (DCI, NH₃): m/z (%) = 311 ([M+H]⁺, 100), Cl-Muster;
HPLC (Methode 3): rt (%) = 3.79 (100).

### Beispiel 63

### 5-Chloro-N-(2-hydroxy-3-{[4-(3-oxo-4-morpholinyl)phenyl]amino}propyl)-2-thiophencarboxamid

ausgehend von 4-(4-Aminophenyl)-3-morpholinon:
MS (ESI): m/z (%) = 410 ([M+H]⁺, 50), Cl-Muster;
HPLC (Methode 3): rt (%) = 3.58 (100).

### Beispiel 64

### N-[3-({4-[Acetyl(cyclopropyl)amino]phenyl}amino)-2-hydroxypropyl]-5-chloro-2-thiophencarboxamid

ausgehend von *N*-(4-Aminophenyl)-*N*-cyclopropylacetamid:
MS (ESI): m/z (%) = 408 ([M+H]⁺, 100), Cl-Muster;
HPLC (Methode 3): rt (%) = 3.77 (100).

### Beispiel 65

### N-[3-({4-[Acetyl(methyl)amino]phenyl}amino)-2-hydroxypropyl]-5-chloro-2-thiophencarboxamid

ausgehend von N-(4-Aminophenyl)-N-methylacetamid:
MS (ESI): m/z (%) = 382 (M+H, 100);
HPLC (Methode 4): rt = 3.31 min.

### Beispiel 66

### 5-Chloro-N-(2-hydroxy-3-{[4-(1H-1,2,3-triazol-1-yl)phenyl]amino}propyl)-2-thiophencarboxamid

ausgehend von 4-(1H-1,2,3-Triazol-1-yl)anilin (Bouchet et al.; J.Chem.Soc.Perkin Trans.2; 1974; 449):
MS (ESI): m/z (%) = 378 (M+H, 100);
HPLC (Methode 4): rt = 3.55 min.

### Beispiel 67

### Tert: butyl 1-{4-[(3-{[(5-chloro-2-thienyl)carbonyl]amino}-2-hydroxypropyl)-amino]phenyl}-L-prolinat

MS (ESI): m/z (%) = 480 (M+H, 100);
HPLC (Methode 4): rt = 3.40 min.

### Beispiel 68

### 1-{4-[(3-{[(5-Chloro-2-thienyl)carbonyl]amino}-2-hydroxypropyl)amino]phenyl}-4-piperidincarboxamid

MS (ESI): m/z (%) = 437 (M+H, 100);
HPLC (Methode 4): rt = 2.39 min.

### Beispiel 69

### 1-{4-[(3-{[(5-Chloro-2-thienyl)carbonyl]amino}-2-hydroxypropyl)-amino]phenyl}-3-piperidincarboxamid

MS (ESI): m/z (%) = 437 (M+H, 100);
HPLC (Methode 4): rt = 2.43 min.

### Beispiel 70

### 5-Chloro-N-(2-hydroxy-3-{[4-(4-oxo-1-piperidinyl)phenyl]amino}propyl)-2-thiophencarboxamid

MS (ESI): m/z (%) = 408 (M+H, 100);
HPLC (Methode 4): rt = 2.43 min.

### Beispiel 71

### 1-{4-[(3-{[(5-Chloro-2-thienyl)carbonyl]amino}-2-hydroxypropyl)amino]phenyl}-L-prolinamid

MS (ESI): m/z (%) = 423 (M+H, 100);
HPLC (Methode 4): rt = 2.51 min.

### Beispiel 72

### 5-Chloro-N-[2-hydroxy-3-({4-[3-(hydroxymethyl)-1-piperidinyl]phenyl}-amino)propyl]-2-thiophencarboxamid

MS (ESI): m/z (%) = 424 (M+H, 100);
HPLC (Methode 4): rt = 2.43 min.

### Beispiel 73

### 5-Chloro-N-[2-hydroxy-3-({4-[2-(hydroxymethyl)-1-piperidinyl]phenyl}-amino)propyl]-2-thiophencarboxamid

MS (ESI): m/z (%) = 424 (M+H, 100);
HPLC (Methode 4): rt = 2.49 min.

### Beispiel 74

### Ethyl-1-{4-[(3-{[(5-chloro-2-thienyl)carbonyl]amino}-2-hydroxypropyl)-amino]phenyl}-2-piperidincarboxylat

MS (ESI): m/z (%) = 466 (M+H, 100);
HPLC (Methode 4): rt = 3.02 min.

### Beispiel 75

### 5-Chloro-N-[2-hydroxy-3-({4-[2-(hydroxymethyl)-1-pyrrolidinyl]phenyl}amino)-propyl]-2-thiophencarboxamid

MS (ESI): m/z (%) = 410 (M+H, 100);
HPLC (Methode 4): rt = 2.48 min.

### Beispiel 76

### 5-Chloro-N-(2-hydroxy-3-{[4-(2-methylhexahydro-5H-pyrrolo[3,4-d]isoxazol-5-yl)phenyl]amino}propyl)-2-thiophencarboxamid

MS (ESI): m/z (%) = 437 (M+H, 100).
HPLC (Methode 5): rt = 1.74 min.

### Beispiel 77

### 5-Chloro-N-(2-hydroxy-3-{[4-(1-pyrrolidinyl)-3-(trifluoromethyl)phenyl]-amino}propyl)-2-thiophencarboxamid

MS (ESI): m/z (%) = 448 (M+H, 100);
HPLC (Methode 4): rt = 3.30 min.

### Beispiel 78

### 5-Chloro-N-(2-hydroxy-3-{[4-(2-oxo-1-pyrrolidinyl)-3-(trifluoromethyl)phenyl]-amino}propyl)-2-thiophencarboxamid

MS (ESI): m/z (%) = 462 (M+H, 100);
HPLC (Methode 4): rt = 3.50 min.

### Beispiel 79

### 5-Chloro-N-(3-{[3-chloro-4-(3-oxo-4-morpholinyl)phenyl]amino}-2-hydroxy-propyl)-2-thiophencarboxamid

MS (ESI): m/z (%) = 444 (M+H, 100);
HPLC (Methode 4): rt = 3.26 min.

### Beispiel 80

### 5-Chloro-N-(2-hydroxy-3-{[4-(3-oxo-4-morpholinyl)-3-(trifluoromethyl)phenyl]-amino}propyl)-2-thiophencarboxamid

MS (ESI): m/z (%) = 478 (M+H, 100);
HPLC (Methode 4): rt = 3.37 min.

### Beispiel 81

### 5-Chloro-N-(2-hydroxy-3-{[3-methyl-4-(3-oxo-4-morpholinyl)phenyl]amino}-propyl)-2-thiophencarboxamid

MS (ESI): m/z (%) = 424 (M+H, 100);
HPLC (Methode 4): rt = 2.86 min.

### Beispiel 82

### 5-Chloro-N-(3-{[3-cyano-4-(3-oxo-4-morpholinyl)phenyl]amino}-2-hydroxypropyl)-2-thiophencarboxamid

MS (ESI): m/z (%) = 435 (M+H, 100);
HPLC (Methode 4): rt = 3.10 min.

### Beispiel 83

### 5-Chloro-N-(3-{[3-chloro-4-(1-pyrrolidinyl)phenyl]amino}-2-hydroxypropyl)-2-thiophencarboxamid

MS (ESI): m/z (%) = 414 (M+H, 100);
HPLC (Methode 4): rt = 2.49 min.

### Beispiel 84

### 5-Chloro-N-(3-{[3-chloro-4-(2-oxo-1-pyrrolidinyl)phenyl]amino}-2-hydroxypropyl)-2-thiophencarboxamid

MS (ESI): m/z (%) = 428 (M+H, 100);
HPLC (Methode 4): rt = 3.39 min.

### Beispiel 85

### 5-Chloro-N-(3-{[3,5-dimethyl-4-(3-oxo-4-morpholinyl)phenyl]amino}-2-hydroxypropyl)-2-thiophencarboxamid

MS (ESI): m/z (%) = 438 (M+H, 100);
HPLC (Methode 4): rt = 2.84 min.

### Beispiel 86

### N-(3-{[3-(Aminocarbonyl)-4-(4-morpholinyl)phenyl]amino}-2-hydroxypropyl)-5-chloro-2-thiophencarboxamid

MS (ESI): m/z (%) = 439 (M+H, 100);
HPLC (Methode 4): rt = 2.32 min.

### Beispiel 87

### 5-Chloro-N-(2-hydroxy-3-{[3-methoxy-4-(4-morpholinyl)phenyl]amino}propyl)-2-thiophencarboxamid

MS (ESI): m/z (%) = 426 (M+H, 100);
HPLC (Methode 4): rt = 2.32 min.

### Beispiel 88

### N-(3-{[3-Acetyl-4-(4-morpholinyl)phenyl]amino}-2-hydroxypropyl)-5-chloro-2-thiophencarboxamid

MS (ESI): m/z (%) = 438 (M+H, 100);
HPLC (Methode 4): rt = 2.46 min.

### Beispiel 89

### N-(3-{[3-Amino-4-(3-oxo-4-morpholinyl)phenyl]amino}-2-hydroxypropyl)-5-chloro-2-thiophencarboxamid

MS (ESI): m/z (%) = 425 (M+H, 100);
HPLC (Methode 4): rt = 2.45 min.

### Beispiel 90

### 5-Chloro-N-(3-{[3-chloro-4-(2-methyl-3-oxo-4-morpholinyl)phenyl]amino}-2-hydroxypropyl)-2-thiophencarboxamid

MS (ESI): m/z (%) = 458 (M+H, 100);
HPLC (Methode 4): rt = 3.44 min.

### Beispiel 91

### 5-Chloro-N-(3-{[3-chloro-4-(2-methyl-5-oxo-4-morpholinyl)phenyl]amino}-2-hydroxypropyl)-2-thiophencarboxamid

MS (ESI): m/z (%) = 458 (M+H, 100);
HPLC (Methode 4): rt = 3.48 min.

### Beispiel 91a

### 5-Chloro-N-[2-hydroxy-3-({4-[(3-oxo-4-morpholinyl)methyl]phenyl}amino)-propyl]-2-thiophencarboxamid

Ausgehend von 4-(4-Amino-benzyl)-3-morpholinon (Surrey et al.; J. Amer. Chem. Soc. ; 77; 1955; 633):
MS (ESI): m/z (%) = 424 (M+H, 100);
HPLC (Methode 4): rt = 2.66 min.

### Allgemeine Methode zu Darstellung von 3-substituierten 5-Chloro-N-[(2-oxo-1,3-oxazolidin-5-yl)methyl]-2-thiophenearboxamid-Derivaten ausgehend von substituierten N-(3-Amino-2-hydroxypropyl)-5-chloro-2-thiophencarboxamid-Derivaten

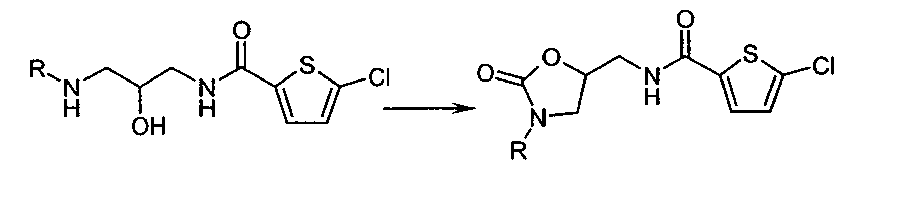

Zu einer Lösung von substituiertem *N*-(3-Amino-2-hydroxypropyl)-5-chloro-2-thiophencarboxamid-Derivat (1.0 eq.) in absolutem THF (ca. 0.1 mol/l) wird bei Raumtemperatur Carbodiimidazol (1.2 bis 1.8 eq.) oder ein vergleichbares Phosgenequivalent gegeben. Die Mischung wird bei Raumtemperatur oder gegebenenfalls bei erhöhter Temperatur (bis zu 70°C) für 2 bis 18 h gerührt, bevor im Vakuum eingeengt wird. Das Produkt kann durch Chromatographie an Silicagel (Dichlormethan-Methanol-Gemische oder Cyclohexan-Essigester-Gemische) gereinigt werden.

Auf analoge Weise wurden hergestellt:

### Beispiel 27

### N-[(3-Benzyl-2-oxo-1,3-oxazolidin-5-yl)methyl]-5-ehloro-2-thiophencarboxamid

MS (DCI, NH₄): m/z (%) = 372 (M+Na, 100), 351 (M+H, 45);
HPLC (Methode 1): rt (%) = 4.33 min (100).

### Beispiel 28

### 5-Chloro-N-{[3-(3-cyanophenyl)-2-oxo-1,3-oxazolidin-5-yl]methyl)-2-thiophen-carboxamid

MS (DCI, NH₄): m/z (%) = 362 (M+H, 42), 145 (100);
HPLC (Methode 2): rt (%) = 4.13 min (100).

### Beispiel 29

### 5-Chloro-N-({3-[4-(cyanomethyl)phenyl]-2-oxo-1,3-oxazolidin-5-yl}methyl)-2-thiophencarboxamid

MS (ESI): m/z (%) = 376 (M+H, 100);
HPLC (Methode 4): rt = 4.12 min

### Beispiel 30

### 5-Chloro-N-({3-[3-(cyanomethyl)phenyl]-2-oxo-1,3-oxazolidin-5-yl}methyl)-2-thiophencarboxamid

MS (ESI): m/z (%) = 376 (M+H, 100);
HPLC (Methode 4): rt = 4.17 min

### Beispiel 92

### tert-Butyl-4-[5-({[(5-chloro-2-thienyl)carbonyl]amino}methyl)-2-oxo-1,3-oxazolidin-3-yl]benzylcarbamat

ausgehend von Beispiel 58:
MS (ESI): m/z (%) = 488 (M+Na, 23), 349 (100);
HPLC (Methode 1): rt (%) = 4.51 (98.5).

### Beispiel 93

### tert-Butyl 4-[5-({[(5-chloro-2-thienyl)carbonyl]amino}methyl)-2-oxo-1,3-oxazolidin-3-yl]phenylcarbamat

ausgehend von Beispiel 59:
MS (ESI): m/z (%) = 493 (M+Na, 70), 452 (M+H, 10), 395 (100);
HPLC (Methode 1): rt (%) = 4.41 (100).

### Beispiel 94

### tert-Butyl-2-oxo-3-[4-(2-oxo-1-pyrrolidinyl)phenyl]-1,3-oxazolidin-5-yl}methylcarbamat

ausgehend von Beispiel 60:
MS (DCI, NH₃): m/z (%) = 393 (M+NH₄, 100);
HPLC (Methode 3): rt (%) = 3.97 (100).

### Beispiel 95

### 5-Chloro-N-({3-[3-fluoro-4-(3-oxo-4-morpholinyl)phenyl]-2-oxo-1,3-oxazolidin-5-yl}methyl)-2-thiophencarboxamid

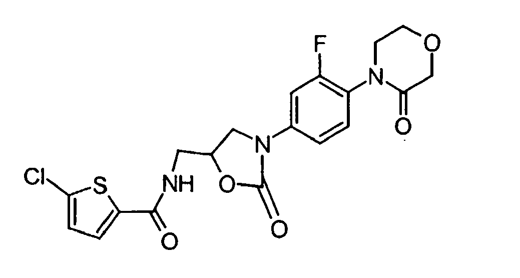

260 mg (0.608 mmol) 5-Chloro-N-(3-{[3-fluoro-4-(3-oxo-4-morpholinyl)phenyl]-amino}-2-hydroxypropyl)-2-thiophencarboxamid (aus Beispiel 61), 197 mg (1.22 mmol) Carbonylimidazol und 7 mg Dimethylaminopyridin werden in 20 ml Dioxan 5 Stunden lang unter Rückfluss gekocht. Anschließend gibt man 20 ml Acetonitril hinzu und rührt in einem Mikrowellenofen in einem geschlossenen Behälter 30 Minuten lang bei 180°C. Die Lösung wird einrotiert und auf einer RP-HPLC Säule chromatographiert. Man erhält 53 mg (19% d.Th.) der Zielverbindung.

*NMR (300 MHz, d₆-DMSO):* δ= 3.6-3.7 (m,4H), 3.85 (dd,1H), 3.95 (m,2H), 4.2 (m,1H), 4.21 (s,2H), 4.85 (m,1H), 4.18 (s,2H), 7.19(d,1H,thiophen), 7.35 (dd,1H), 7.45 (t,1H), 7.55 (dd,1H), 7.67 (d,1H,thiophen), 8.95(t,1H,CONH).

### Beispiel 96

### 5-Chloro-N-[(2-oxo-3-phenyl-1,3-oxazolidin-5-yl)methyl]-2-thiophencarboxamid

ausgehend von Beispiel 62:
MS (ESI): m/z (%) = 359 ([M+Na]⁺, 71), 337 ([M+H]⁺, 100), Cl-Muster;
HPLC (Methode 3): rt (%) = 4.39 (100).
IC₅₀: 2 µM

### Beispiel 97

### 5-Chloro-N-({2-oxo-3-[4-(3-oxo-4-morpholinyl)phenyl]-1,3-oxazolidin-5-yl}-methyl)-2-thiophencarboxamid

ausgehend von Beispiel 63:
MS (ESI): m/z (%) = 458 ([M+Na]⁺, 66), 436 ([M+H]⁺, 100), Cl-Muster;
HPLC (Methode 3): rt (%) = 3.89 (100).
IC₅₀: 1.4 nM

### Beispiel 98

### N-[(3-{4-[Acetyl(cyclopropyl)amino]phenyl}-2-oxo-1,3-oxazolidin-5-yl)methyl]-5-chloro-2-thiophencarboxamid

ausgehend von Beispiel 64:
MS (ESI): m/z (%) = 456 ([M+Na]⁺, 55), 434 ([M+H]⁺, 100), Cl-Muster;
HPLC (Methode 3): rt (%) = 4.05 (100).
IC₅₀: 50 nM

### Beispiel 99

### N-[(3-{4-[Acetyl(methyl)amino]phenyl}-2-oxo-1,3-oxazolidin-5-yl)methyl]-5-chloro-2-thiophencarboxamid

MS (ESI): m/z (%) = 408 (M+H, 30), 449 (M+H+MeCN, 100);
HPLC (Methode 4): rt = 3.66 min.

### Beispiel 100

### 5-Chloro-N-({2-oxo-3-[4-(1H-1,2,3-triazol-1-yl)phenyl]-1,3-oxazolidin-5-yl}-methyl)-2-thiophencarboxamid

MS (ESI): m/z (%) = 404 (M+H, 45), 445 (M+H+MeCN, 100);
HPLC (Methode 4): rt = 3.77 min.

### Beispiel 101

### Tert.-butyl-1-{4-[5-({[(5-chloro-2-thienyl)carbonyl]amino}methyl)-2-oxo-1,3-oxazolidin-3-yl] phenyl}-L-prolinat

MS (ESI): m/z (%) = 450 (M+H-56, 25), 506 (M+H, 100);
HPLC (Methode 4): rt = 5.13 min.

### Beispiel 102

### 1-{4-[5-({[(5-Chloro-2-thienyl)carbonyl]amino}methyl)-2-oxo-1,3-oxazolidin-3-yl]phenyl}-4-piperidincarboxamid

MS (ESI): m/z (%) = 463 (M+H, 100);
HPLC (Methode 4): rt = 2.51 min.

### Beispiel 103

### 1-{4-[5-({[(5-Chloro-2-thienyl)carbonyl]amino}methyl)-2-oxo-1,3-oxazolidin-3-yl]phenyl}-3-piperidincarboxamid

MS (ESI): m/z (%) = 463 (M+H, 100);
HPLC (Methode 4): rt = 2.67 min.

### Beispiel 104

### 5-Chloro-N-({2-oxo-3-[4-(4-oxo-1-piperidinyl)phenyl]-1,3-oxazolidin-5-yl}methyl)-2-thiophencarboxamid

MS (ESI): m/z (%) = 434 (M+H, 40), 452 (M+H+H₂O, 100), 475 (M+H+MeCN, 60);
HPLC (Methode 4): rt = 3.44 min.

### Beispiel 105

### 1-{4-[5-({[(5-Chloro-2-thienyl)carbonyl]amino}methyl)-2-oxo-1,3-oxazolidin-3-yl]phenyl}-L-prolinamid

MS (ESI): m/z (%) = 449 (M+H, 100);
HPLC (Methode 4): rt = 3.54 min.

### Beispiel 106

### 5-Chloro-N-[(3-{4-[3-(hydroxymethyl)-1-piperidinyl]phenyl}-2-oxo-1,3-oxazolidin-5-yl)methyl]-2-thiopheucarboxamid

MS (ESI): m/z (%) = 450 (M+H, 100);
HPLC (Methode 5): rt = 2.53 min.

### Beispiel 107

### 5-Chloro-N-[(3-{4-[2-(hydroxymethyl)-1-piperidinyl]phenyl}-2-oxo-1,3-oxazolidin-5-yl)methyl]-2-thiophencarboxamid

MS (ESI): m/z (%) = 450 (M+H, 100);
HPLC (Methode 5): rt = 2.32 min.

### Beispiel 108

### Ethyl 1-{4-[5-({[(5-chloro-2-thienyl)carbonyl]amino}methyl)-2-oxo-1,3-oxazolidin-3-yl]phenyl}-2-piperidincarboxylat

MS (ESI): m/z (%) = 492 (M+H, 100);
HPLC (Methode 5): rt = 4.35 min.

### Beispiel 109

### 5-Chloro-N-[(3-{4-[2-(hydroxymethyl)-1-pyrrolidinyl]phenyl}-2-oxo-1,3-oxazolidin-5-yl)methyl]-2-thiophencarboxamid

MS (ESI): m/z (%) = 436 (M+H, 100);
HPLC (Methode 4): rt = 2.98 min.

### Beispiel 110

### 5-Chloro-N-({2-oxo-3-[4-(1-pyrrolidinyl)-3-(trifluoromethyl)phenyl]-1,3-oxazolidin-5-yl}methyl)-2-thiophencarboxamid

MS (ESI): m/z (%) = 474 (M+H, 100);
HPLC (Methode 4): rt = 4.63 min.

### Beispiel 111

### 5-Chloro-N-({3-[4-(2-methylhexahydro-5H-pyrrolo[3,4-d]isoxazol-5-yl)phenyl]-2-oxo-1,3-oxazolidin-5-yl}methyl)-2-thiophencarboxamid

MS (ESI): m/z (%) = 463 (M+H, 100);
HPLC (Methode 4): rt = 2.56 min.

### Beispiel 112

### 5-Chloro-N-({2-oxo-3-[4-(2-oxo-1-pyrrolidinyl)-3-(trifluoromethyl)phenyl]-1,3-oxazolidin-5-yl}methyl)-2-thiophencarboxamid

MS (ESI): m/z (%) = 488 (M+H, 100);
HPLC (Methode 4): rt = 3.64 min.

### Beispiel 113

### 5-Chloro-N-({3-[3-chloro-4-(3-oxo-4-morpholinyl)phenyl]-2-oxo-1,3-oxazolidin-5-yl}methyl)-2-thiophencarboxamid

MS (ESI): m/z (%) = 470 (M+H, 100);
HPLC (Methode 4): rt = 3.41 min.

### Beispiel 114

### 5-Chloro-N-({2-oxo-3-[4-(3-oxo-4-morpholinyl)-3-(trifluoromethyl)phenyl]-1,3-oxazolidin-5-yl}methyl)-2-thiophencarboxamid

MS (ESI): m/z (%) = 504 (M+H, 100);
HPLC (Methode 4): rt = 3.55 min.

### Beispiel 115

### 5-Chloro-N-({3-[3-methyl-4-(3-oxo-4-morpholinyl)phenyl]-2-oxo-1,3-oxazolidin-5-yl}methyl)-2-thiophencarboxamid

MS (ESI): m/z (%) = 450 (M+H, 100);
HPLC (Methode 4): rt = 3.23 min.

### Beispiel 116

### 5-Chloro-N-({3-[3-cyano-4-(3-oxo-4-morpholinyl)phenyl]-2-oxo-1,3-oxazolidin-5-yl}methyl)-2-thiophencarboxamid

MS (ESI): m/z (%) = 461 (M+H, 100);
HPLC (Methode 4): rt = 3.27 min.

### Beispiel 117

### 5-Chloro-N-({3-[3-chloro-4-(1-pyrrolidinyl)phenyl]-2-oxo-1,3-oxazolidin-5-yl}methyl)-2-thiophencarboxamid

MS (ESI): m/z (%) = 440 (M+H, 100);
HPLC (Methode 4): rt = 3.72 min.

### Beispiel 118

### 5-Chloro-N-({3-[3-chloro-4-(2-oxo-1-pyrrolidinyl)phenyl]-2-oxo-1,3-oxazolidin-5-yl}methyl)-2-thiophencarboxamid

MS (ESI): m/z (%) = 454 (M+H, 100);
HPLC (Methode 4): rt = 3.49 min.

### Beispiel 119

### 5-Chloro-N-({3-[3,5-dimethyl-4-(3-oxo-4-morpholinyl)phenyl]-2-oxo-1,3-oxazolidin-5-yl}methyl)-2-thiophencarboxamid

MS (ESI): m/z (%) = 464 (M+H, 100);
HPLC (Methode 4): rt = 3.39 min.

### Beispiel 120

### N-({3-[3-(Aminocarbonyl)-4-(4-morpholinyl)phenyl]-2-oxo-1,3-oxazolidin-5-yl}methyl)-5-chloro-2-thiophencarboxamid

MS (ESI): m/z (%) = 465 (M+H, 100);
HPLC (Methode 4): rt = 3.07 min.

### Beispiel 121

### 5-Chloro-N-({3-[3-methoxy-4-(4-morpholinyl)phenyl]-2-oxo-1,3-oxazolidin-5-yl}methyl)-2-thiophencarboxamid

MS (ESI): m/z (%) = 452 (M+H, 100);
HPLC (Methode 4): rt = 2.86 min.

### Beispiel 122

### N-({3-[3-Acetyl-4-(4-morpholinyl)phenyl]-2-oxo-1,3-oxazolidin-5-yl}methyl)-5-chloro-2-thiophencarboxamid

MS (ESI): m/z (%) = 464 (M+H, 100);
HPLC (Methode 4): rt = 3.52 min.

### Beispiel 123

### N-({3-[3-Amino-4-(3-oxo-4-morpholinyl)phenyl]-2-oxo-1,3-oxazolidin-5-yl}-methyl)-5-chloro-2-thiophencarboxamid

MS (ESI): m/z (%) = 451 (M+H, 100);
HPLC (Methode 6): rt = 3.16 min.

### Beispiel 124

### 5-Chloro-N-({3-[3-chloro-4-(2-methyl-3-oxo-4-morpholinyl)phenyl]-2-oxo-1,3. oxazolidin-5-yl}methyl)-2-thiophencarboxamid

MS (ESI): m/z (%) = 484 (M+H, 100);
HPLC (Methode 4): rt = 3.59 min.

### Beispiel 125

### 5-Chloro-N-({3-[3-chloro-4-(2-methyl-5-oxo-4-morpholinyl)phenyl]-2-oxo-1,3-oxazolidin-5-yl}methyl)-2-thiophencarboxamid

MS (ESI): m/z (%) = 484 (M+H, 100);
HPLC (Methode 4): rt = 3.63 min.

### Beispiel 125a

### 5-Chloro-N-[(2-oxo-3-{4-[(3-oxo-4-morpholinyl)methyl]phenyl}-1,3-oxazolidin-5-yl)methyl]-2-thiophencarboxamid

MS (ESI): m/z (%) = 450 (M+H, 100);
HPLC (Methode 4): rt = 3.25 min.

Über den Weg der Epoxidöffnung mit einem Amin und anschließende Cyclisierung zum entsprechenden Oxazolidinon wurden darüber hinaus die folgenden Verbindungen hergestellt:

| Beispiel-Nr. | Struktur | Smp. [°C] | IC₅₀ [µM] |
|---|---|---|---|
| 126 | 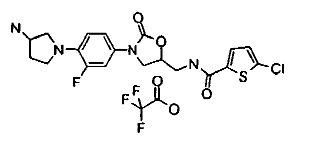 | 229Z | 0,013 |
| 127 | 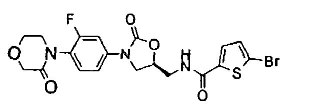 | 159 | 0,0007 |
| 128 | 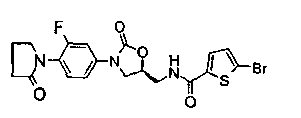 | 198 | 0,002 |
| 129 | 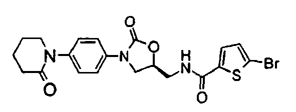 | 196 | 0,001 |
| 130 | 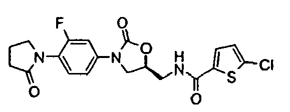 | 206 | 0,0033 |
| 130a | 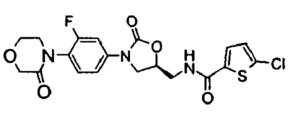 | 194 | |
| 131 | 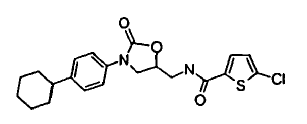 | 195 | 0,85 |
| 132 | 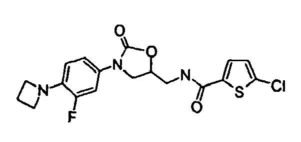 | 206 | 0,12 |
| 133 | 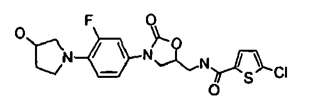 | 217 | 0,062 |
| 134 | 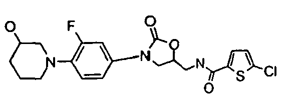 | 207 | 0,48 |
| | aus 1-(4-Amino-phenyl)-piperidin-3-ol (Tong,L.K.J. et al.; J.Amer.Chem.Soc 1960; 82,1988). | | |
| 135 | 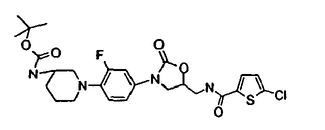 | 202 | 1,1 |
| 136 | 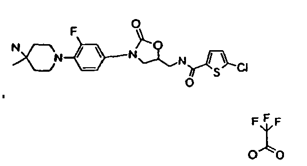 | 239 | 1,2 |
| 137 | 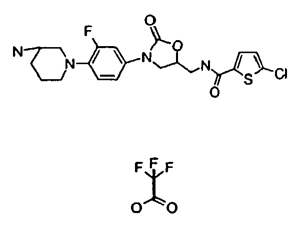 | 219 | 0,044 |
| 138 | 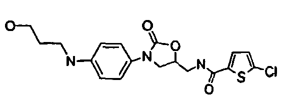 | 95 | 0,42 |
| 139 | 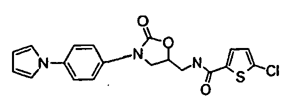 | 217 | 1,7 |

Die folgenden Beispiele 14 bis 16 sind Ausführungsbeispiele für den fakultativen, d.h. gegebenenfalls stattfindenden Oxidationsverfahrensschritt.

### Beispiel 14

### 5-Chloro-N-({(5S)-3-[3-fluoro-4-(1-oxo-1[lambda]⁴,4-thiazinan-4-yl)phenyl]-2-oxo-1,3-oxazolidin-5-yl}methyl)-2-thiophencarboxamid

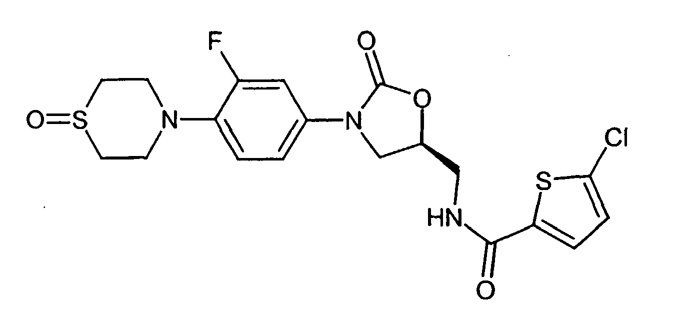

5-Chloro-N-({(5S)-3-[3-fluoro-4-(1,4-thiazinan-4-yl)phenyl]-2-oxo-1,3-oxazolidin-5-yl}methyl)-2-thiophencarboxamid (0.1 g, 0.22 mmol) aus Beispiel 3 in Methanol (0.77 ml) wird bei 0°C zu einer Lösung von Natriumperiodat (0.05 g, 0.23 mmol) in Wasser (0.54 ml) gegeben und 3 h bei 0°C gerührt. Anschließend gibt man 1 ml DMF hinzu und rührt 8 h bei RT. Nach Zugabe von weiteren 50 mg Natriumperiodat wird nochmals über Nacht bei RT gerührt. Man versetzt anschließend den Ansatz mit 50 ml Wasser und saugt das unlösliche Produkt ab. Man erhält nach Waschen mit Wasser und Trocknen 60 mg (58 % d. Th.) Kristalle.
Smp.: 257°C;
R_{f} (Kieselgel, Toluol/Essigester 1:1) = 0.54 (Edukt = 0.46);
IC₅₀-Wert = 1.1 µM;
MS (DCI) 489 (M+NH₄), Cl-Muster.

### Beispiel 15

### Darstellung von 5-Chloro-N-({(5S)-3-[4-(1,1-dioxo-1[lambda]⁶,4-thiazinan-4-yl)-3-fluorophenyl]-2-oxo-1,3-oxazolidin-5-yl}methyl)-2-thiophencarboxamid

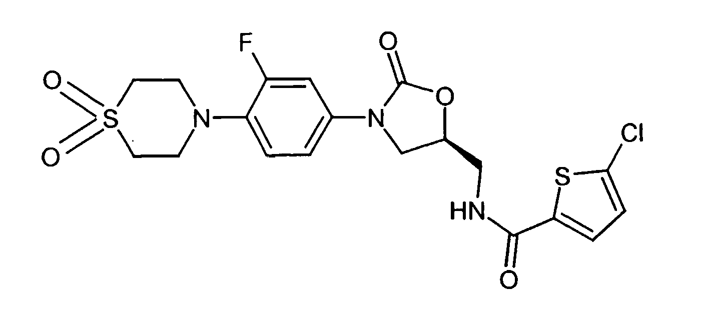

Man versetzt 5-Chloro-N-({(5S)-3-[3-fluoro-4-(1,4-thiazinan-4-yl)phenyl]-2-oxo-1,3-oxazolidin-5-yl}methyl)-2-thiophencarboxamid aus Beispiel 3 (0.1 g, 0.22 mmol) in 3.32 ml einer Mischung von 1 Teil Wasser und 3 Teilen Aceton mit 80 mg (0.66 mmol) N-Methylmorpholin-N-oxid (NMO) und 0.1 ml einer 2.5 %igen Lösung von Osmiumtetroxid in 2-Methyl-2-propanol. Man rührt über Nacht bei Raumtemperatur und gibt nochmals 40 mg NMO hinzu. Nachdem eine weitere Nacht gerührt wurde, gibt man den Ansatz in 50 ml Wasser und extrahiert dreimal mit Essigester. Aus der organischen Phase erhält man nach Trocknen und Eindampfen 23 mg und aus der wässrigen Phase nach Absaugen des unlöslichen Feststoffs 19 mg (insges. 39% d. Th.) der Zielverbindung.
Smp.: 23 8°C;
R_{f} (Toluol/Essigester 1:1) = 0.14 (Edukt = 0.46);
IC₅₀-Wert = 210 nM;
MS (DCI): 505 (M+NH₄), Cl-Muster.

### Beispiel 16

### 5-Chloro-N-{[(5S)-3-(3-fluoro-4-morpholinophenyl)-2-oxo-1,3-oxazolidin-5-yl]methyl}-2-thiophencarboxamid N-oxid

wird durch Behandeln von 5-Chloro-N-{[(5S)-3-(3-fluoro-4-morpholinophenyl)-2-oxo-1,3-oxazolidin-5-yl]methyl}-2-thiophencarboxamid aus Beispiel 1 mit Monoperoxyphthalsäure-Magnesiumsalz erhalten.
MS (ESI): 456 (M+H, 21 %, Cl-Muster), 439 (100%).

Die folgenden Beispiele 31 bis 35 und 140 bis 147 beziehen sich auf den fakultativen, d.h. gegebenenfalls stattfindenden Amidinierungsverfahrensschritt.

### Allgemeine Methode zur Darstellung von Amidinen und Amidinderivaten ausgehend von cyanomethylphenylsubstituierten 5-Chloro-N-[(2-oxo-1,3-oxazolidin-5-yl)methyl]-2-thiophencarboxamid Derivaten

Das jeweilige cyanomethylphenylsubstituierte 5-Chloro-N-[(2-oxo-1,3-oxazolidin-5-yl)methyl]-2-thiophencarboxamid-Derivat (1.0 eq.) wird zusammen mit Triethylamin (8.0 eq.) für ein bis zwei Tage bei RT in einer gesättigten Lösung von Schwefelwasserstoff in Pyridin gerührt (ca. 0.05 - 0.1 mol/l). Das Reaktionsgemisch wird mit Ethylacetat (EtOAc) verdünnt und mit 2 N Salzsäure gewaschen. Die organische Phase wird mit MgSO₄ getrocknet, filtriert und im Vakuum eingedampft.

Das Rohprodukt wird in Aceton gelöst (0.01-0.1 mol/l) und mit Methyliodid (40 eq.) versetzt. Das Reaktionsgemisch wird 2 bis 5 h bei Raumtemperatur (RT) gerührt und dann im Vakuum eingeengt.

Der Rückstand wird in Methanol gelöst (0.01-0.1 mol/l) und zur Darstellung der unsubstituierten Amidine mit Ammoniumacetat (3 eq.) und Ammoniumchlorid (2 eq.) versetzt. Zur Darstellung der substituierten Amidinderivate werden primäre oder sekundäre Amine (1.5 eq.) und Essigsäure (2 eq.) zu der methanolischen Lösung gegeben. Nach 5-30 h wird das Lösungsmittel im Vakuum entfernt und der Rückstand durch Chromatographie an einer RP8-Kieselgel-Säule gereinigt (Wasser/Acetonitril 9/1-1/1 + 0.1 % Trifluoressigsäure).

Auf analoge Weise wurden hergestellt:

### Beispiel 31:

### N-({3-[4-(2-Amino-2-iminoethyl)phenyl]-2-oxo-1,3-oxazolidin-5-yl}methyl)-5-chloro-2-thiophencarboxamid

MS (ESI): m/z (%) = 393 (M+H, 100);
HPLC (Methode 4): rt = 2.63 min

### Beispiel 32:

### 5-Chloro-N-({3-[3-(4,5-dihydro-1H-imidazol-2-ylmethyl)phenyl]-2-oxo-1,3-oxazolidin-5-yl}methyl)-2-thiophencarboxamid

MS (ESI): m/z (%) = 419 (M+H, 100);
HPLC (Methode 4): rt = 2.61 min

### Beispiel 33:

### 5-Chloro-N-[(3-{3-[2-imino-2-(4-morpholinyl)ethyl]phenyl}-2-oxo-1,3-oxazolidin-5-yl)methyl]-2-thiophencarboxamid

MS (ESI): m/z (%) = 463 (M+H, 100);
HPLC (Methode 4): rt = 2.70 min

### Beispiel 34:

### 5-Chloro-N-[(3-{3-[2-imino-2-(1-pyrrolidinyl)ethyl]phenyl}-2-oxo-1,3-oxazolidin-5-yl)methyl]-2-thiophencarboxamid

MS (ESI): m/z (%) = 447 (M+H, 100);
HPLC (Methode 4): rt = 2.82 min

### Beispiel 35:

### N-({3-[3-(2-Amino-2-iminoethyl)phenyl]-2-oxo-1,3-oxazolidin-5-yl}methyl)-5-chloro-2-thiophencarboxamid

MS (ESI): m/z (%) = 393 (M+H, 100);
HPLC (Methode 4): rt = 2.60 min

### Beispiel 140

### 5-Chloro-N-({3-[4-(4,5-dihydro-1H-imidazol-2-ylmethyl)phenyl]-2-oxo-1,3-oxazolidin-5-yl}methyl)-2-thiophencarboxamid

MS (ESI): m/z (%) = 419 (M+H, 100);
HPLC (Methode 4): rt = 2.65 min

### Beispiel 141

### 5-Chloro-N-[(3-{4-[2-imino-2-(4-morpholinyl)ethyl]phenyl}-2-oxo-1,3-oxazolidin-5-yl)methyl]-2-thiophencarboxamid

MS (ESI): m/z (%) = 463 (M+H, 100);
HPLC (Methode 4): rt = 2.65 min

### Beispiel 142

### 5-Chloro-N-[(3-{4-[2-imino-2-(1-piperidinyl)ethyl]phenyl}-2-oxo-1,3-oxazolidin-5-yl)methyl]-2-thiophencarboxamid

MS (ESI): m/z (%) = 461 (M+H, 100);
HPLC (Methode 4): rt = 2.83 min

### Beispiel 143

### 5-Chloro-N-[(3-{4-[2-imino-2-(1-pyrrolidinyl)ethyl]phenyl}-2-oxo-1,3-oxazolidin-5-yl)methyl]-2-thiophencarboxamid

MS (ESI): m/z (%) = 447 (M+H, 100);
HPLC (Methode 4): rt = 2.76 min

### Beispiel 144

### 5-Chloro-N-[(3-{4-[2-(cyclopentylamino)-2-iminoethyl]phenyl}-2-oxo-1,3-oxazolidin-5-yl)methyl]-2-thiophencarboxamid

MS (ESI): m/z (%) = 461 (M+H, 100);
HPLC (Methode 4): rt = 2.89 min

### Beispiel 145

### 5-Chloro-N-{[3-(4-{2-imino-2-[(2,2,2-trifluoroethyl)amino]ethyl}phenyl)-2-oxo1,3-oxazolidin-5-yl]methyl}-2-thiophencarboxamid

MS (ESI): m/z (%) = 475 (M+H, 100);
HPLC (Methode 4): rt = 2.79 min

### Beispiel 146

### N-({3-[4-(2-Anilino-2-iminoethyl)phenyl]-2-oxo-1,3-oxazolidin-5-yl}methyl)-5-chloro-2-thiophencarboxamid

MS (ESI): m/z (%) = 469 (M+H, 100);
HPLC (Methode 4): rt = 2.83 min

### Beispiel 147

### 5-Chloro-N-[(3-{4-[2-imino-2-(2-pyridinylamino)ethyl]phenyl}-2-oxo-1,3-oxazolidin-5-yl)methyl]-2-thiophencarboxamid

MS (ESI): m/z (%) = 470 (M+H, 100);
HPLC (Methode 4): rt = 2.84 min

Die folgenden Beispiele 148 bis 151 beziehen sich auf die Abspaltung von BOC-Aminoschutzgruppen:

### Allgemeine Methode zur Abspaltung von Boc-Schutzgruppen (tert-Butyloxy-carbonyl):

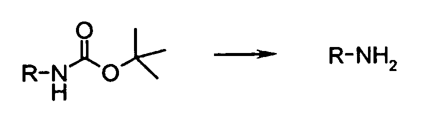

Zu einer eisgekühlten Lösung einer *tert*.-Butyloxycarbonyl- (Boc) geschützten Verbindung in Chloroform oder Dichlormethan (ca.0.1 bis 0.3 mol/l) wird wässrige Trifluoressigsäure (TFA, ca. 90 %) getropft. Nach ca. 15 min wird die Eiskühlung entfernt und die Mischung ca. 2-3 h bei Raumtemperatur gerührt, bevor die Lösung eingeengt und am Hochvakuum getrocknet wird. Der Rückstand wird in Dichlormethan oder Dichlormethan/Methanol aufgenommen und mit gesättigter Natriumhydrogencarbonat- oder IN Natriumhydroxid-Lösung gewaschen. Die organische Phase wird mit gesättigter Natriumchlorid-Lösung gewaschen, über wenig Magnesiumsulfat getrocknet und konzentriert. Gegebenenfalls erfolgt eine Reinigung durch Kristallisation aus Ether oder Ether/Dichlormethan-Gemischen.

Auf analoge Weise wurden aus den entsprechen Boc-geschützten Vorläufern hergestellt:

### Beispiel 148

### N-({3-[4-(Aminomethyl)phenyl]-2-oxo-1,3-oxazolidin-5-yl}methyl)-5-chloro-2-thiophen-carboxamid

ausgehend von Beispiel 92:
MS (ESI): m/z (%) = 349 (M-NH₂, 25), 305 (100);
HPLC (Methode 1): rt (%) = 3.68 (98).
IC₅₀: 2.2 µM

### Beispiel 149

### N-{[3-(4-Aminophenyl)-2-oxo-1,3-oxazolidin-5-yl]methyl}-5-chloro-2-thiophen-carboxamid

ausgehend von Beispiel 93:
MS (ESI): m/z (%) = 352 (M+H, 25);
HPLC (Methode 1): rt (%) = 3.50 (100).
IC₅₀: 2 µM

Eine enantiomerenreine Alternativsynthese dieser Verbindung ist im folgenden Schema dargestellt (vgl. auch Delalande S.A., DE 2836305,1979; Chem.Abstr. 90, 186926):

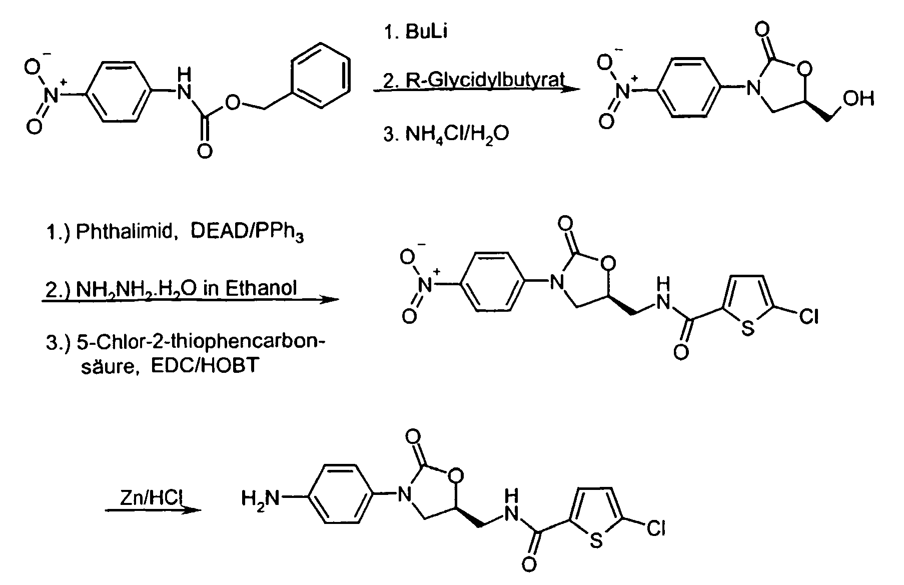

### Beispiel 150

### 5-Chloro-N-({3-[4-(glycylamino)phenyl]-2-oxo-1,3-oxazolidin-5-yl}methyl)-2-thiophencarboxamid

ausgehend von Beispiel 152:
MS (ES-pos): m/z (%) = 408 (100);
HPLC (Methode 3): rt (%) = 3.56 (97).
IC₅₀: 2 µM

### Beispiel 151

### 5-(Aminomethyl)-3-[4-(2-oxo-1-pyrrolidinyl)phenyl]-1,3-oxazolidin-2-on

ausgehend von Beispiel 60:
MS (ESI): m/z (%) = 276 (M+H, 100);
HPLC (Methode 3): rt (%) = 2.99 (100).
IC₅₀: 2 µM

Die folgenden Beispiele 152 bis 166 beziehen sich auf die Aminogruppenderivatisierung von Anilin- oder Benzylamin-substituierten Oxazolidinonen mit verschiedenen Reagenzien:

### Beispiel 152

### 5-Chloro-N-({3-[4-(N-tert.-butyloxycarbonyl-glycylamino)phenyl]-2-oxo-1,3-oxazolidin-5-yl} methyl)-2-thiophencarboxamid

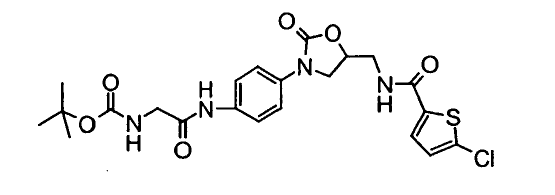

Zu einer Lösung von 751 mg (4.3 mmol) Boc-Glycin, 870 mg (6.4 mmol) HOBT (1-Hydroxy-1H-benzotriazol x H₂O), 1790 mg (4.7 mmol) HBTU [O-(Benzotriazol-1-yl)-N,N,N',N'-tetramethyluroniumhexafluorophosphat] und 1.41 ml (12.9 mmol) *N*-Methylmorpholin in 15 ml DMF/CH₂Cl₂ (1:1) werden bei 0°C 754 mg (2.1 mmol) *N*-{[3-(4-Aminophenyl)-2-oxo-1,3-oxazolidin-5-yl]methyl}-5-chloro-2-thiophencarboxamid (aus Beispiel 149) gegeben. Die Mischung wird über Nacht bei Raumtemperatur gerührt, bevor mit Wasser verdünnt wird. Der ausgefallene Feststoff wird abfiltriert und getrocknet. Ausbeute: 894 mg (79.7 % der Theorie);
MS (DCI, NH₃): m/z (%) = 526 (M+NH₄, 100);
HPLC (Methode 3): rt (%) = 4.17 (97).

### Beispiel 153

### N-[(3-{4-[(Acetylamino)methyl]phenyl}-2-oxo-1,3-oxazolidin-5-yl)methyl]-5-chloro-2-thiophencarboxamid

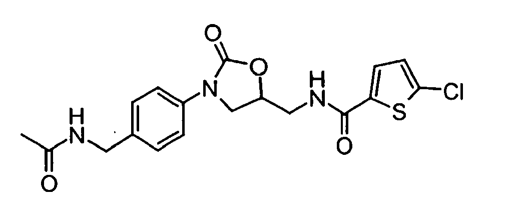

Eine Mischung von 30 mg (0.082 mmol) *N*-({3-[4-(Aminomethyl)phenyl]-2-oxo-1,3-oxazolidin-5-yl}methyl)-5-chloro-2-thiophen-carboxamid (aus Beispiel 148) in 1.5 ml absolutem THF und 1.0 ml absolutem Dichlormethan, 0.02 ml absolutem Pyridin wird bei 0°C mit Acetanhydrid (0.015 ml, 0.164 mmol) versetzt. Die Mischung wird über Nacht bei Raumtemperatur gerührt. Nach Zusetzen von Ether und Kristallisation wird das Produkt gewonnen. Ausbeute: 30 mg (87 % der Theorie),
MS (ESI): m/z (%) = 408 (M+H, 18), 305 (85);
HPLC (Methode 1): rt (%) = 3.78 (97).
IC₅₀: 0.6 µM

### Beispiel 154

### N-{[3-(4-{[(Aminocarbonyl)amino]methyl}phenyl)-2-oxo-1,3-oxazolidin-5-yl]-methyl}-5-chloro-2-thiophencarboxamid

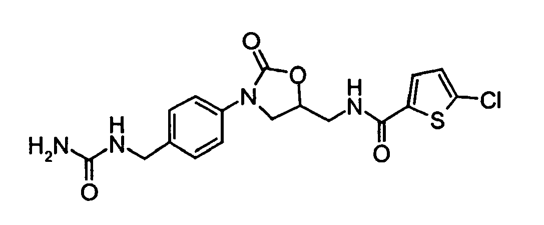

Zu einer Mischung von 30 mg (0.082 mmol) *N*-({3-[4-(Aminomethyl)phenyl]-2-oxo-1,3-oxazolidin-5-yl}methyl)-5-chloro-2,thiophen-carboxamid (aus Beispiel 148) in 1.0 ml Dichlormethan werden bei Raumtemperatur 0.19 ml (0.82 mmol) Trimethylsilylisocyanat getropft. Es wird über Nacht gerührt, bevor nach Zusatz von Ether das Produkt durch Filtration gewonnen wird. Ausbeute: 21.1 mg (52 % der Theorie), MS (ESI): m/z (%) = 409 (M+H, 5), 305 (72);
HPLC (Methode 1): rt (%) = 3.67 (83).
IC₅₀: 1.3 µM

### Allgemeine Methode zur Acylierung von N-{[3-(4-Aminophenyl)-2-oxo-1,3-oxazolidin-5-yl]methyl}-5-chloro-2-thiophenearboxamid mit Carbonsäurechloriden:

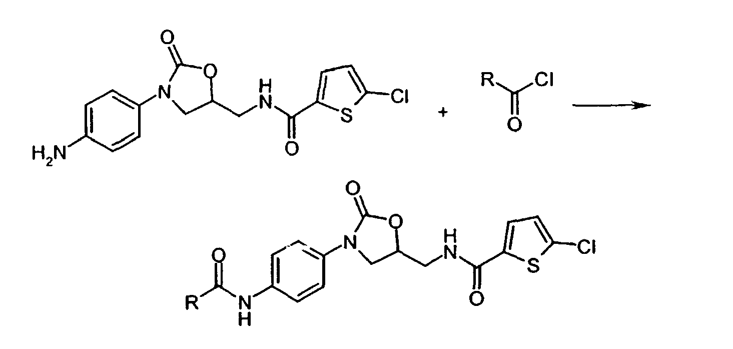

Unter Argon wird zu entsprechendem Säurechlorid (2.5 eq.) eine ca. 0.1 molare Lösung von *N*-{[3-(4-Aminophenyl)-2-oxo-1,3-oxazolidin-5-yl]methyl}-5-chloro-2-thiophencarboxamid (aus Beispiel 149) (1.0 eq.) in absolutem Dichlormethan/Pyridin (19:1) getropft. Die Mischung wird über Nacht gerührt, bevor mit ca. 5 eq PS-Trisamine (Argonaut Technologies) und 2 ml absolutem Dichlormethan versetzt wird. Nach 1 h leichtem Rühren, wird abfiltriert und das Filtrat konzentriert. Gegebenenfalls erfolgt eine Reinigung der Produkte durch präparative RP-HPLC.

Auf analoge Weise wurden hergestellt:

### Beispiel 155

### N-({3-[4-(Acetylamino)phenyl]-2-oxo-1,3-oxazolidin-5-yl}methyl)-5-chloro-2-thiophen-carboxamid

LC-MS: m/z (%) = 394 (M+H, 100);
LC-MS (Methode 6): rt (%) = 3.25 (100).
IC₅₀: 1.2 µM

### Beispiel 156

### 5-Chloro-N-[(2-oxo-3-{4-[(2-thienylcarbonyl)amino]phenyl}-1,3-oxazolidin-5-yl)methyl]-2-thiophencarboxamid

LC-MS: m/z (%) = 462 (M+H, 100);
LC-MS (Methode 6): rt (%) = 3.87 (100).
IC₅₀: 1.3 µM

### Beispiel 157

### 5-Chloro-N-[(3-{4-[(methoxyacetyl)amino]phenyl}-2-oxo-1,3-oxazolidin-5-yl)-methyl]-2-thiophencarboxamid

LC-MS: m/z (%) = 424 (M+H, 100);
LC-MS (Methode 6): rt (%) = 3.39 (100).
IC₅₀:0.73 µM

### Beispiel 158

### N-{4-[5-({[(5-Chloro-2-thienyl)carbonyl]amino}methyl)-2-oxo-1,3-oxazolidin-3-yl]phenyl}-3,5-dimethyl-4-isoxazolcarboxamid

LC-MS: m/z (%) = 475 (M+H, 100).
IC₅₀: 0.46 µM

### Beispiel 159

### 5-Chloro-N-{[3-(4-{[(3-chloropropyl)sulfonyl]amino}phenyl)-2-oxo-1,3-oxazolidin-5-yl]methyl}-2-thiophencarboxamid

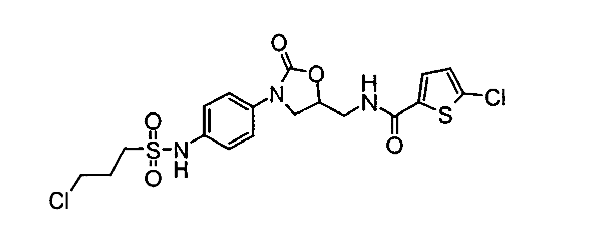

Zu einer eisgekühlten Lösung von 26.4 mg (0.15 mmol) 3-Chloro-1-propansulfonsäurechlorid und 0.03 ml (0.2 mmol) Triethylamin in 3.5 ml absolutem Dichlormethan werden 35 mg (0.1 mmol) *N*-{[3-(4-Aminophenyl)-2-oxo-1,3-oxazolidin-5-yl]-methyl}-5-chloro-2-thiophen-carboxamid (aus Beispiel 149) gegeben. Nach 30 min wird die Eiskühlung entfernt und die Mischung über Nacht bei Raumtemperatur gerührt, bevor 150 mg (ca. 5.5 eq) PS-Trisamine (Argonaut Technologies) und 0.5 ml Dichlormethan zugesetzt werden. Die Suspension wird 2 h leicht gerührt, filtriert (das Harz wird mit Dichlormethan/Methanol nachgewaschen) und das Filtrat eingeengt. Das Produkt wird durch präparative RP-HPLC gereinigt. Ausbeute: 19.6 mg (40 % der Theorie),
LC-MS: m/z (%) = 492 (M+H, 100);
LC-MS (Methode 5): rt (%) = 3.82 (91).
IC₅₀: 1.7 µM

### Beispiel 160

### 5-Chloro-N-({3-[4-(1,1-dioxido-2-isothiazolidinyl)phenyl]-2-oxo-1,3-oxaxolidin-5-yl}methyl)-2-thiophencarboxamid

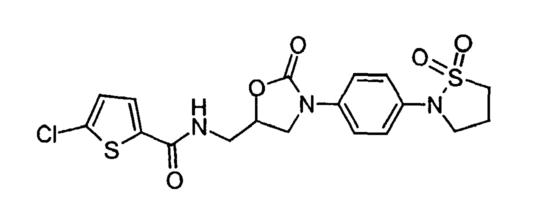

Eine Mischung aus 13.5 mg (0.027 mmol) 5-Chloro-*N*-{[3-(4-{[(3-chloropropyl)sulfonyl]amino} phenyl)-2-oxo-1,3-oxazolidin-5-yl]methyl} -2-thiophen-carboxamid (aus Beispiel 159) und 7.6 mg (0.055 mmol) Kaliumcarbonat in 0.2 ml DMF wird 2 h auf 100°C erhitzt. Nach Abkühlen wird mit Dichlormethan verdünnt und mit Wasser gewaschen. Die organische Phase wird getrocknet und eingeengt. Der Rückstand wird durch präparative Dünnschichtchromatographie (Silicagel, Dichlormethan/Methanol, 95:5) gereinigt. Ausbeute: 1.8 mg (14.4 % der Theorie),
MS (ESI): m/z (%) = 456 (M+H, 15), 412 (100);
LC-MS (Methode 4): rt (%) = 3.81 (90).
IC₅₀: 0.14 µM

### Beispiel 161

### 5-Chloro-N-[((5S)-3-{4-[(5-chloropentanoyl)amino]phenyl}-2-oxo-1,3-oxazolidin-5-yl)methyl]-2-thiophencarboxamid

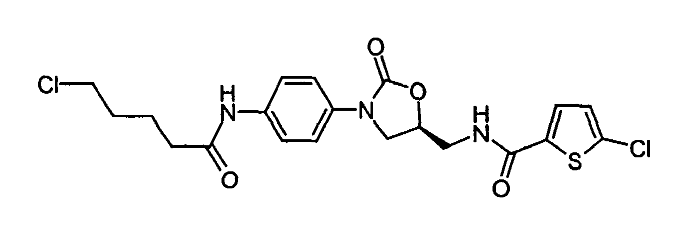

0.5 g (1.29 mmol) N-{[(SS)-3-(4-Aminophenyl)-2-oxo-1,3-oxazolidin-5-yl]methyl}-5-chloro-2-thiophencarboxamid (aus Beispiel 149) werden in 27 ml Tetrahydrofuran gelöst und mit 0.2 g (1,29 mmol) 5-Chlorvaleriansäurechlorid sowie 0.395 ml (2.83 mmol) Triethylamin versetzt. Man dampft den Ansatz im Vakuum ein und chromatographiert auf Kieselgel mit einem Toluol/Essigester=1:1 -> Essigester-Gradienten. Man erhält 315 mg (52% d.Th.) eines Feststoffs.
Smp.: 211 °C.

### Beispiel 162

### 5-Chloro-N-({(5S)-2-oxo-3-[4-(2-oxo-1-piperidinyl)phenyl]-1,3-oxazolidin-5-yl}-methyl)-2-thiophencarboxamid

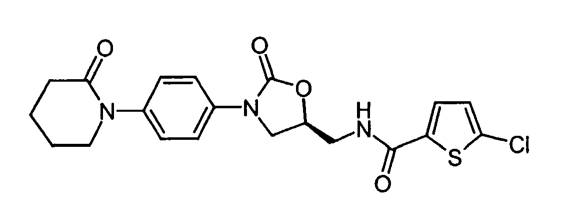

Man gibt unter inerten Bedingungen zu 5 ml DMSO 30 mg 60-proz. NaH in Paraffinöl und erwärmt 30 min lang auf 75°C bis zur Beendigung der Gasentwicklung. Anschließend tropft man eine Lösung von 290 mg (0.617 mmol) 5-Chloro-N-[((5S)-3-{4-[(5-chloropentanoyl)amino]phenyl}-2-oxo-1,3-oxazolidin-5-yl)methyl]-2-thiophencarboxamid (aus Beispiel 161) in 5 ml Methylenchlorid hinzu und rührt über Nacht bei Raumtemperatur. Die Reaktion wird abgebrochen und das Gemisch in 100 ml Wasser gegeben und mit Essigester extrahiert. Die eingedampfte organische Phase wird auf einer RP-8 Säule chromatographiert und mit Acetonitril/Wasser eluiert. Man erhält 20 mg (7.5% d.Th.) der Zielverbindung.
Smp.: 205°C;
*NMR (300 MHz, d₆-DMSO):* δ = 1.85 (m,4H), 2.35 (m,2H), 3.58 (m,4H), 3.85 (m,1H), 4.2 (t,1H), 4.82 (m,1H), 7.18 (d,1H,thiophen), 7.26 (d,2H), 7.5 (d,2H), 2.68 (d,1H,thiophen), 9.0 (t,1H,CONH).
IC₅₀: 2.8 nM

### Beispiel 163

### 5-Chloro-N-[((5S)-3-{4-[(3-bromopropionyl)amino]phenyl}-2-oxo-1,3-oxazolidin-5-yl)methyl]-2-thiophencarboxamid

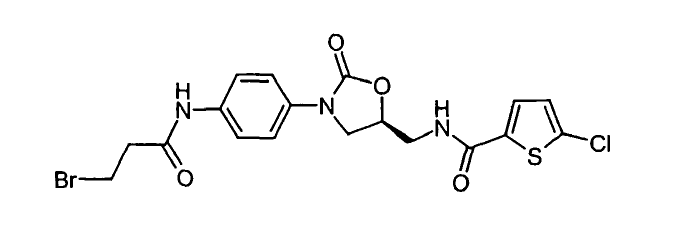

wird in analoger Weise aus Beispiel 149 erhalten.

### Beispiel 164

### 5-Chloro-N-({(5S)-2-oxo-3-[4-(2-oxo-1-azetidinyl)phenyl]-1,3-oxazolidin-5-yl}-methyl)-2-thiophencarboxamid

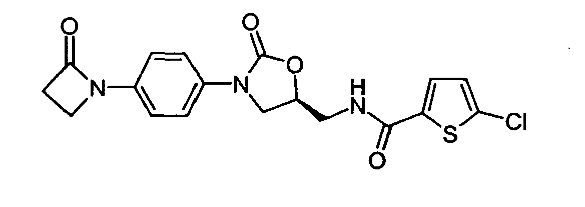

wird in analoger Weise durch Cyclisierung der offenkettigen Bromopropionylverbindung aus Beispiel 163 mittels NaH/DMSO erhalten.
MS (ESI): m/z (%) = 406 ([M+H]⁺, 100), Cl-Muster.
IC₅₀: 380 nM

### Beispiel 165

### tert-Butyl 4-{4-[5-({[(5-chloro-2-thienyl)carbonyl]amino}methyl)-2-oxo-1,3-oxazolidin-3-yl]phenyl}-3,5-dioxo-1-piperazincarboxylat

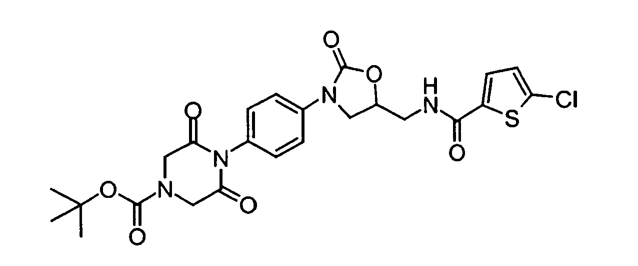

Zu einer Lösung von 199 mg (0.85 mmol) Boc-Iminodiessigsäure, 300 mg (2.2 mmol) HOBT, 0.66 ml (6 mmol) *N*-Methylmorpholin und 647 mg (1.7 mmol) HBTU werden 300 mg (0.85 mmol) *N*-{[3-(4-Aminophenyl)-2-oxo-1,3-oxazolidin-5-yl]-methyl}-5-chloro-2-thiophen-carboxamid in 6 ml einer Mischung aus DMF und Dichlormethan (1:1) gegeben. Die Mischung wird über Nacht gerührt, bevor nach Verdünnen mit Dichlormethan mit Wasser, gesättigter Ammoniumchlorid-Lösung, gesättigter Natriumhydrogencarbonat-Lösung, Wasser und gesättigter Natriumchlorid-Lösung gewaschen wird. Die organische Phase wird über Magnesiumsulfat getrocknet und eingeengt. Das Rohprodukt wird durch Chromatographie an Silicagel (Dichlormethan/Methanol 98:2) gereinigt. Ausbeute: 134 mg (29 % der Theorie);
MS (ESI): m/z (%) = 571 (M+Na, 82), 493 (100);
HPLC (Methode 3): rt (%) = 4.39 (90).
IC₅₀: 2 µM

### Beispiel 166

### N-[((5S)-3-{4-[(3R)-3-Amino-2-oxo-1-pyrrolidinyl]phenyl}-2-oxo-1,3-oxazolidin-5-yl)methyl]-5-chloro-2-thiophencarboxamid Trifluoracetat

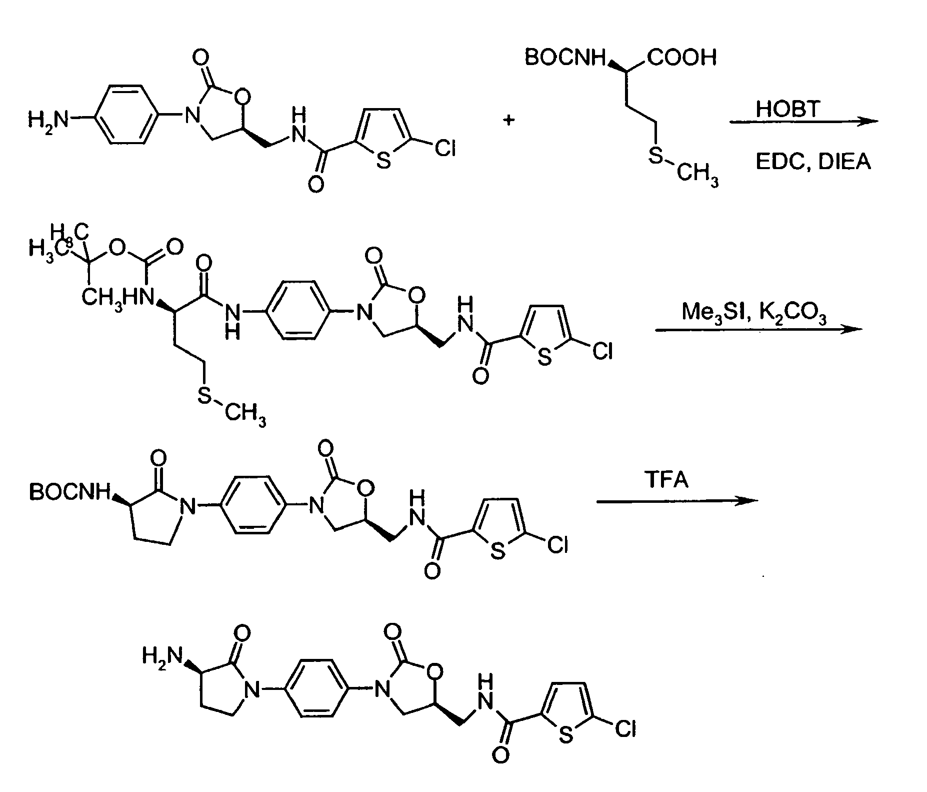

### N2-(tert-Butoxycarbonyl)-N1-{4-[(5S)-5-({[(5-chloro-2-thienyl)carbonyl]amino} methyl)-2-oxo-1,3-oxazolidin-3-yl]phenyl}-D-methioninamid

429 mg (1.72 mmol) N-BOC-D-Methionin, 605 mg (1.72 mmol) N-{[(5S)-3-(4-aminophenyl)-2-oxo-1,3-oxazolidin-5-yl]methyl}-5-chloro-2-thiophencarboxamid, und 527 mg (3.44 mmol) HOBT-Hydrat werden in 35 ml DMF gelöst, mit 660 mg (3.441 mmol) EDCI Hydrochlorid und anschließend tropfenweise mit 689 mg (5.334 mmol) N-Ethyl-diisopropylamin versetzt. Man rührt bei Raumtemperatur zwei Tage lang. Die erhaltene Suspension wird abgesaugt und der Rückstand mit DMF gewaschen. Die vereinigten Filtrate werden mit etwas Kieselgel versetzt, im Vakuum eingedampft und auf Kieselgel mit einem Toluol -> T10EE7 - Gradienten chromatographiert. Man erhält 170 mg (17% d.Th.) der Zielverbindung mit einem Schmelzpunkt von 183°C.
R_{f} (SiO₂, Toluol/Essigester=1:1):0.2. *¹H-NMR (300 MHz, d₆-DMSO):* δ=1.4 (s,1H,BOC), 1.88-1.95 (m,2H), 2.08 (s,3H,SMe), 2.4-2.5 (m,2H, teilweise verdeckt durch DMSO), 3.6 (m,2H), 3.8 (m,1H), 4.15 (m,2H), 4.8 (m,1H), 7.2 (1H, thiophen), 7.42 (d, Teil eines AB-Systems, 2H), 7.6 (d, Teil eines AB-Systems, 2H), 7.7 (d, 1H, thiophen), 8.95 (t,1H, CH₂NHCO), 9.93 (bs,1H,NH).

### tert-Butyl (3R)-1-{4-[(5S)-5-({[(5-chloro-2-thienyl)carbonyl]amino}methyl)-2-oxo-1,3-oxazolidin-3-yl] phenyl}-2-oxo-3-pyrrolidinylcarbamat

170 mg (0.292 mmol) N2-(tert-butoxycarbonyl)-N1-{4-[(5S)-5-({[(5-chloro-2-thienyl)carbonyl]amino}methyl)-2-oxo-1,3-oxazolidin-3-yl]phenyl}-D-methionin-amid werden in 2 ml DMSO gelöst und mit 178.5 mg (0.875 mmol) Trimethylsulfoniumiodid sowie 60.4 mg (0.437 mmol) Kaliumcarbonat versetzt und 3.5 Stunden bei 80°C gerührt. Anschließend wird im Hochvakuum eingedampft und der Rückstand mit Ethanol gewaschen. Es verbleiben 99 mg der Zielverbindung.
*¹H-NMR (300 MHz, d₆-DMSO):* δ =1.4 (s,1H,BOC), 1.88-2.05 (m,1H), 2.3-2.4 (m,1H), 3.7-3.8 (m,3H), 3.8-3.9 (m,1H), 4.1-4.25 (m,1H), 4.25-4.45 (m,1H), 4.75-4.95 (m,1H), 7.15 (1H, thiophen), 7.25 (d,1H), 7.52 (d, Teil eines AB-Systems, 2H), 7.65 (d, Teil eines AB-Systems, 2H), 7.65 (d, 1H, thiophen), 9.0 (breites s,1H).

### N-[((5S)-3-{4-[(3R)-3-Amino-2-oxo-1-pyrrolidinyl]phenyl}-2-oxo-1,3-oxazolidin-5-yl)methyl]-5-chloro-2-thiophencarboxamid Trifluoracetat

Man suspendiert 97 mg (0.181 mmol) tert-butyl (3R)-1-{4-[(5S)-S-({[(5-Chloro-2-thienyl)carbonyl]amino}methyl)-2-oxo-1,3-oxazolidin-3-yl]phenyl}-2-oxo-3-pyrrolidinylcarbamat in 4 ml Methylenchlorid, gibt 1.5 ml Trifluoressigsäure hinzu und rührt 1 Stunde bei Raumtemperatur. Anschließend wird im Vakuum eingedampft und auf einer RP-HPLC gereinigt (Acetonitril/Wasser/0.1%TFA-Gradient). Man erhält nach Eindampfen der betreffenden Fraktion 29 mg (37% d.Th.) der Zielverbindung mit einem Schmelzpunkt von 241°C (Zers.).
R_{f} (SiO₂,EtOH/TEA=17:1) 0.19.
*¹H-NMR (300 MHz, d₆-DMSO): δ* =1.92-2.2 (m,1H), 2.4-2.55 (m,1H, teilweise verdeckt durch DMSO-peak), 3.55-3.65 (m,2H), 3.75-3.95 (m,3H), 4.1-4.3 (m,2H), 4.75-4.9 (m,1H), 7.2 (1H, thiophen), 7.58 (d, Teil eines AB-Systems, 2H), 7.7 (d, Teil eines AB-Systems, 2H), 7.68 (d, 1H, thiophen), 8.4 (breites s,3H, NH3), 8.9 (t,1H,NHCO).

Die folgenden Beispiele 167 bis 170 beziehen sich auf die Einführung von Sulfonamidgruppen in Phenyl-substituierten Oxazolidinonen:

### Allgemeine Methode zur Darstellung von substituierten Sulfonamiden ausgehend von 5-Chloro-N-[(2-oxo-3-phenyl-1,3-oxazolidin-5-yl)methyl]-2-thiophencarboxamid

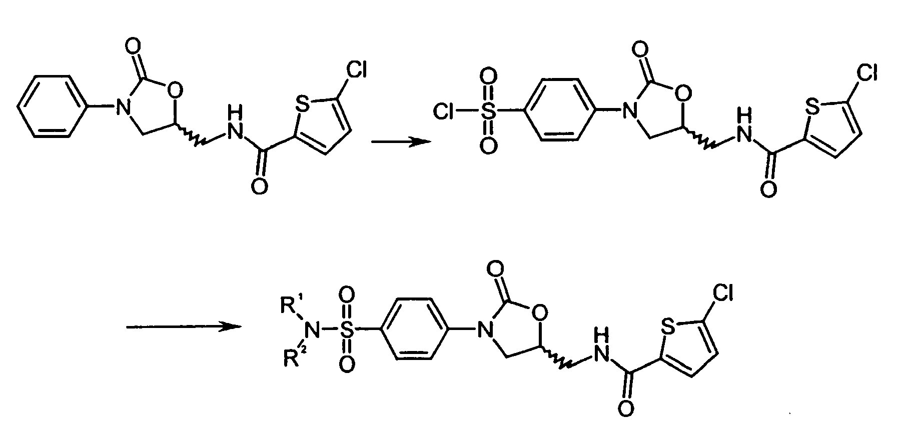

Zu Chlorsulfonsäure (12 eq.) wird unter Argon bei 5°C 5-Chloro-*N*-[(2-oxo-3-phenyl-1,3-oxazolidin-5-yl)methyl]-2-thiophencarboxamid (aus Beispiel 96) gegeben. Das Reaktionsgemisch wird bei Raumtemperatur für 2 h gerührt und anschließend auf Eiswasser gegeben. Der ausfallende Niederschlag wird filtriert, mit Wasser gewaschen und getrocknet.

Anschließend wird unter Argon bei Raumtemperatur in Tetrahydrofuran (0.1 mol/l) gelöst und mit dem entsprechenden Amin (3 eq.), Triethylamin (1.1 eq.) und Dimethylaminopyridin (0.1 eq.) versetzt. Das Reaktionsgemisch wird 1-2 h gerührt und anschließend im Vakuum eingeengt. Das gewünschte Produkt wird mittels Flash-Chromatographie (Dichlormethan-Methanol-Gemische) gereinigt.

Auf analoge Weise wurden hergestellt:

### Beispiel 167

### 5-Chloro-N-({2-oxo-3-[4-(1-pyrrolidinylsulfonyl)phenyl]-1,3-oxazolidin-5-yl}-methyl)-2-thiophencarboxamid

MS (ESI): m/z (%) = 492 ([M+Na]⁺, 100), 470 ([M+H]⁺, 68), Cl-Muster;
HPLC (Methode 3): rt (%) = 4.34 (100).
IC₅₀: 0.5 µm

### Beispiel 168

### 5-Chloro-N-[(3-{4-[(4-methyl-1-piperazinyl)sulfonyl]phenyl}-2-oxo-1,3-oxazolidin-5-yl)methyl]-2-thiophencarboxamid

MS (ESI): m/z (%) = 499 ([M+H]⁺, 100), Cl-Muster;
HPLC (Methode 2): rt (%) = 3.3 (100).

### Beispiel 169

### 5-Chloro-N-({2-oxo-3-[4-(1-piperidinylsulfonyl)phenyl]-1,3-oxazolidin-5-yl}-methyl)-2-thiophencarboxamid

MS (ESI): m/z (%) = 484 ([M+H]⁺, 100), Cl-Muster;
HPLC (Methode 2): rt (%) = 4.4 (100).

### Beispiel 170

### 5-Chloro-N-[(3-{4-[(4-hydroxy-1-piperidinyl)sulfonyl]phenyl}-2-oxo-1,3-oxazolidin-5-yl)methyl]-2-thiophencarboxamid

MS (ESI): m/z (%) = 500 ([M+H]⁺, 100), Cl-Muster;
HPLC (Methode 3): rt (%) = 3.9 (100).

### Beispiel 171

### 5-Chloro-N-({2-oxo-3-[4-(1-pyrrolidinyl)phenyl]-1,3-oxazolidin-5-yl}methyl)-2-thiophencarboxamid

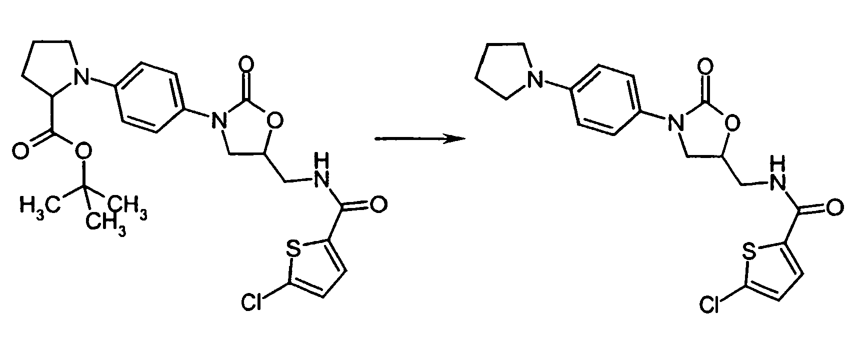

780 mg (1.54 mmol) tert.-Butyl-1-{4-[5-({[(5-chloro-2-thienyl)carbonyl]amino}-methyl)-2-oxo-1,3-oxazolidin-3-yl]phenyl}prolinat werden in 6 ml Dichlormethan und 9 ml Trifluoressigsäure gelöst und das Gemisch wird zwei Tage lang bei 40°C gerührt. Dann wird das Reaktionsgemisch eingeengt und mit Ether und 2 N Natronlauge verrührt. Die wässrige Phase wird eingeengt und mit Ether und 2 N Salzsäure verrührt. Die organische Phase dieser Extraktion wird über MgSO₄ getrocknet, filtriert und eingeengt. Das Rohprodukt wird an Kieselgel chromatographiert (CH₂Cl₂/EtOH/konz. wässr. NH₃-Lsg. = 100/1/0.1 bis 20/1/0.1).
Es werden 280 mg (40 % d. Th.) des Produkts erhalten.
MS (ESI): m/z (%) = 406 (M+H, 100);
HPLC (Methode 4): rt = 3.81 min.

HPLC-Parameter und LC-MS Parameter der in den vorrangegangenen Beispielen angegebenen HPLC- und LC-MS-Daten (die Einheit der Retentionszeit (rt) ist Minuten):
[1] Säule: Kromasil C18, L-R Temperatur: 30°C, Fluss = 0.75 mlmin⁻¹, Eluent: A = 0.01 M HCLO₄, B = CH₃CN, Gradient: -> 0.5 min 98%A -> 4.5 min 10%A ->6.5 min 10%A
[2] Säule: Kromasil C18 60*2, L-R Temperatur: 30°C, Fluss = 0.75 mlmin⁻¹, Eluent: A = 0.01 M H₃PO₄, B = CH₃CN, Gradient: -> 0.5 min 90%A -> 4.5 min 10%A ->6.5 min 10%A
[3] Säule: Kromasil C18 60*2, L-R Temperatur: 30°C, Fluss = 0.75 mlmin⁻¹, Eluent: A = 0.005 M HCIO₄, B = CH₃CN, Gradient: -> 0.5 min 98%A -> 4.5 min 10%A ->6.5 min 10%A
[4] Säule: Symmetry C18 2.1x150 mm, Säulenofen: 50°C, Fluss = 0.6 mlmin⁻¹, Eluent: A = 0.6 g 30%ige HCl/l Wasser, B = CH₃CN, Gradient: 0.0 min 90%A -> 4.0 min 10%A ->9 min 10%A
[5] MHZ-2Q, Instrument Micromass Quattro LCZ
   Säule Symmetry C18, 50 mm x 2.1 mm, 3.5 µm, Temperatur: 40°C, Fluss = 0.5 ml min⁻¹, Eluent A = CH₃CN + 0.1% Ameisensäure, Eluent B = Wasser + 0.1% Ameisensäure, Gradient: 0.0 min 10% A -> 4 min 90% A -> 6 min 90% A
[6] MHZ-2P, Instrument Micromass Platform LCZ
   Säule Symmetry C18, 50 mm x 2.1 mm, 3.5 µm, Temperatur: 40°C, Fluss = 0.5 mlmin⁻¹, Eluent A = CH₃CN +0.1% Ameisensäure, Eluent B = Wasser +0.1% Ameisensäure, Gradient: 0.0 min 10% A -> 4 min 90% A -> 6 min 90% A
[7] MHZ-7Q, Instrument Micromass Quattro LCZ
   Säule Symmetry C18, 50 mm x 2.1 mm, 3.5 µm, Temperatur: 40°C, Fluss = 0.5 mlmin⁻¹, Eluent A = CH₃CN + 0.1% Ameisensäure, Eluent B = Wasser + 0.1% Ameisensäure, Gradient: 0.0 min 5% A -> 1 min 5% A -> 5 min 90% A -> 6 min 90% A

### Allgemeine Methode zu Darstellung von Oxazolidinonen der allgemeinen Formel B durch festphasenunterstützte Synthese

Umsetzungen mit unterschiedlichen harzgebundenen Produkten fanden in einem Satz von getrennten Reaktionsgefäßen statt.

5-(Brommethyl)-3-(4-fluor-3-nitrophenyl)-1,3-oxazolidin-2-on A (dargestellt aus Epibromhydrin und 4-Fluor-3-nitrophenylisocyanat mit LiBr/Bu₃PO in Xylol analog US 4128654, Bsp.2) (1,20 g, 3,75 mmol) und Ethyldiisoproylamin (DIEA, 1,91 ml, 4,13 mmol) wurden in DMSO (70 ml) gelöst, mit einem sekundären Amin (1,1 eq, Aminkomponente 1) versetzt und 5 h bei 55°C umgesetzt. Zu dieser Lösung wurde TentaGel SAM Harz (5,00 g, 0,25 mmol/g) gegeben und 48 h bei 75°C reagiert. Das Harz wurde filtriert und wiederholt mit Methanol (MeOH), Dimethylformamid (DMF), MeOH, Dichlormethan (DCM) und Diethylether gewaschen und getrocknet. Das Harz (5,00 g) wurde in Dichlormethan (80 ml) suspendiert, mit DIEA (10 eq) und 5-Chlorthiophen-2-carbonsäurechlorid [hergestellt durch Reaktion von 5-Chlorthiophen-2-carbonsäure (5 eq) und 1-Chlor-1-Dimethylamino-2-methylpropen (5 eq) in DCM (20 ml) bei Raumtemperatur für 15 Minuten] versetzt und 5 h bei Raumtemperatur reagiert. Das erhaltene Harz wurde filtriert und wiederholt mit MeOH, DCM und Diethylether gewaschen und getrocknet. Anschließend wurde das Harz in DMF/Wasser (v/v 9:2, 80 ml) suspendiert, mit SnCl₂*2H₂O (5 eq) versetzt und 18 h bei Raumtemperatur umgesetzt. Das Harz wurde wiederum wiederholt mit MeOH, DMF, Wasser, MeOH, DCM und Diethylether gewaschen und getrocknet. Dieses Harz wurde in DCM suspendiert, mit DIEA (10 eq) und bei 0°C mit einem Säurechlorid (5 eq Säurederivat 1) versetzt und bei Raumtemperatur über Nacht reagiert. Carbonsäuren wurden vor der Umsetzung durch Reaktion mit 1-Dimethylamino-1-chlor-2-methylpropen (1 eq, bezogen auf die Carbonsäure) in DCM bei Raumtemperatur für 15 min in die korrespondierenden Säurechloride überführt. Das Harz wurde wiederholt mit DMF, Wasser, DMF, MeOH, DCM und Diethylether gewaschen und getrocknet. Im Falle der Verwendung von Fmoc-geschützten Aminosäuren als Säurederivat 1 wurde die Fmoc-Schutzgruppe im letzten Reaktionsschritt durch Umsetzung mit Piperidin/DMF (v/v, 1/4) bei Raumtemperatur für 15 Minuten abgespalten und das Harz mit DMF, MeOH, DCM und Diethylether gewaschen und getrocknet. Die Produkte wurden anschließend mit Trifluoressigsäure (TFA)/DCM (v/v, 1/1) von der festen Phase gespalten, das Harz wurde abfiltriert und die Reaktionslösungen wurden eingedampft. Die Rohprodukte wurden über Kieselgel filtriert (DCM/MeOH, 9:1) und eingedampft um einen Satz von Produkten **B** zu erhalten.

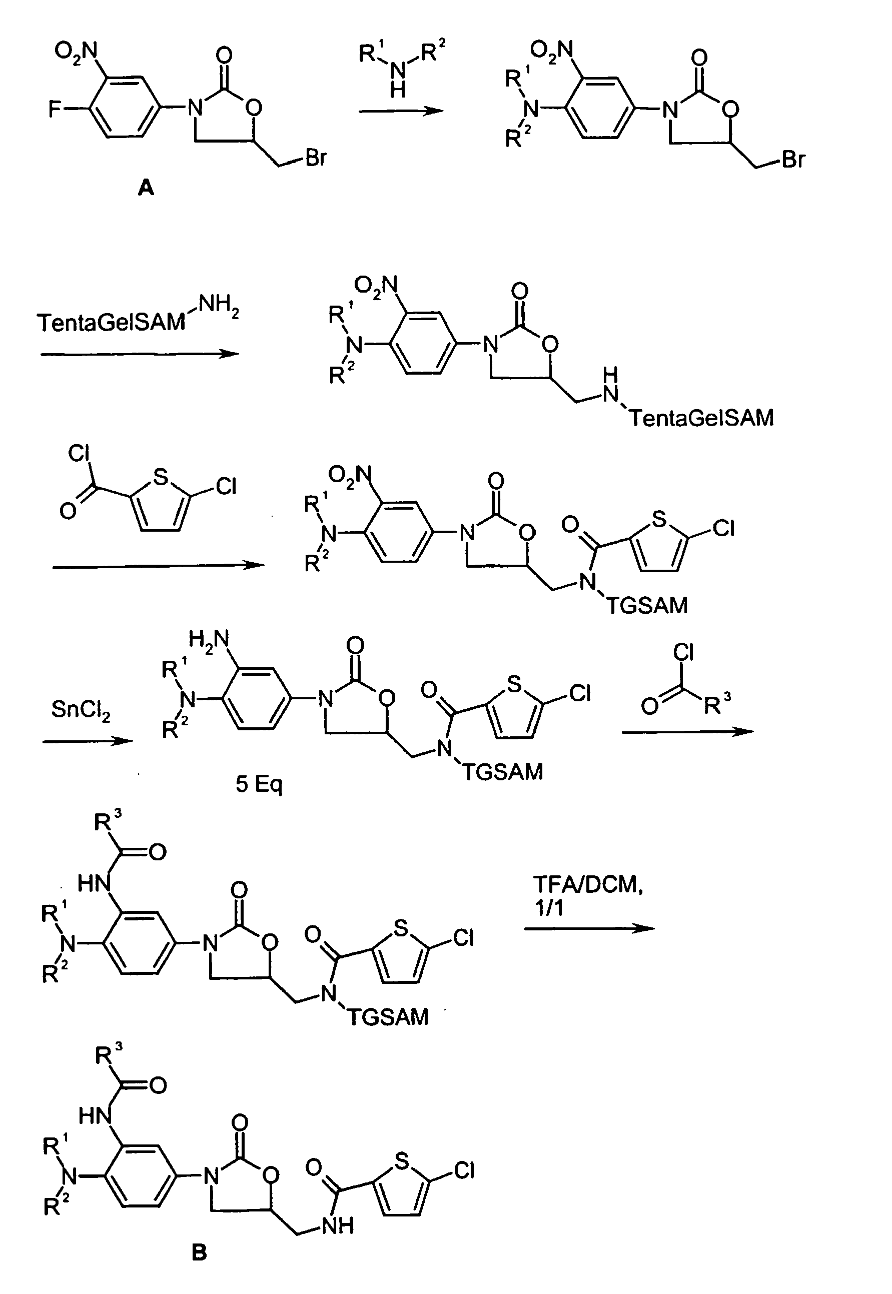

Durch festphasenunterstützte Synthese hergestellte Verbindungen:

### Beispiel 172

### N-({3-[3-Amino-4-(1-pyrrolidinyl)phenyl]-2-oxo-1,3-oxazolidin-5-yl}methyl)-5-chlor-2-thiophencarboxamid

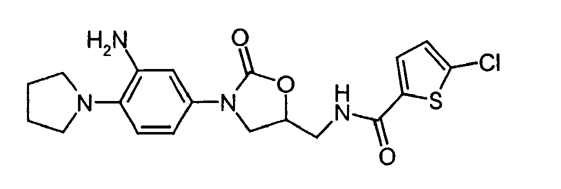

Analog der allgemeinen Arbeitsvorschrift zur Herstellung der Derivate **B** wurden 5 g (1,25 mmol) TentaGel SAM Harz mit Pyrrolidin als Aminderivat 1 umgesetzt. Das nach der Reduktion mit SnCl₂*2H₂O erhaltene Anilin wurde ohne weiteren Acylierungsschritt von der festen Phase abgespalten und eingedampft. Das Rohprodukt wurde zwischen Ethylacetat und NaHCO₃-Lösung verteilt, die organische Phase wurde mit NaCl ausgesalzen, dekantiert und zur Trockene eingedampft. Dieses Rohprodukt wurde durch Vakuum-Flashchromatographie an Kieselgel (Dichlormethan/Ethylacetat, 3:1 - 1:2) gereinigt.
¹H-NMR (300 MHz, CDCl₃): 1.95 - 2.08, br, 4 H; 3.15-3.30, br, 4 H; 3.65-3.81, m, 2 H; 3.89, ddd, 1H; 4.05, dd, 1 H; 4.81, dddd, 1 H; 6.46, dd, 1 H; 6.72, dd, 1 H; 6.90, dd, 1H; 6.99, dd, 1 H; 7.03, dd, 1 H; 7.29, d, 1 H.

### Beispiel 173

### N-[(3-{3-(β-Alanylamino)-4-[(3-hydroxypropyl)amino]phenyl}-2-oxo-1,3-oxazolidin-5-yl)methyl]-5-chlor-2-thiophencarboxamid

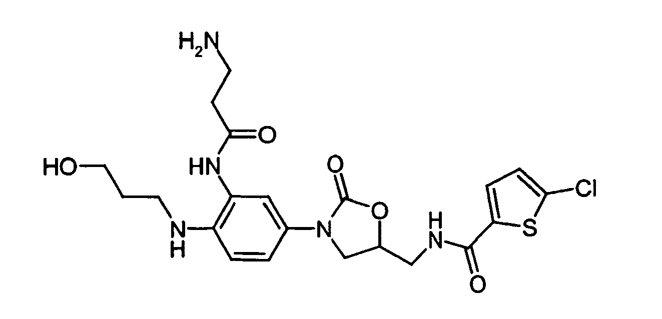

Analog der allgemeinen Arbeitsvorschrift zur Herstellung der Derivate **B** wurden 5 g (1,25 mmol) TentaGel SAM Harz mit Azetidin als Aminderivat 1 und Fmoc-ß-Alanin als Säurederivat 1 umgesetzt. Das nach der Abspaltung erhaltene Rohprodukt wurde 48 h in Methanol bei Raumtemperatur gerührt und zur Trockene eingedampft. Dieses Rohprodukt wurde durch Reversed Phase HPLC mit einem Wasser/TFA/Acetonitril-Gradienten gereinigt.
¹H-NMR (400 MHz, CD₃OD): 2.31, tt, 2 H; 3.36, t, 2 H; 3.54, t, 2 H; 3.62, t, 2 H; 3.72, dd, 1 H; 3.79, dd, 1 H; 4.01, dd, 1 H; 4.29, dd, 2 H; 4.43, t, 2 H; 4.85-4.95, m, 1 H; 7.01, d, 1 H; 4.48 - 7.55, m, 2 H; 7.61, d, 1 H; 7.84, d, 1 H.

### Beispiel 174

### N-({3-[4-(3-Amino-1-pyrrolidinyl)-3-nitrophenyl]-2-oxo-1,3-oxazolidin-5-yl}-methyl)-5-chlor-2-thiophencarboxamid

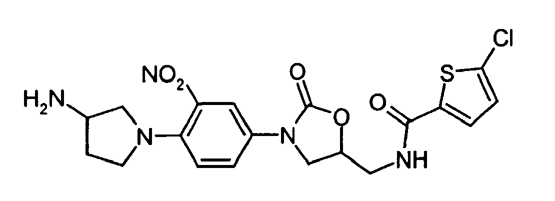

Analog der allgemeinen Arbeitsvorschrift zur Herstellung der Derivate **B** wurden 130 mg (32,5 µmol) TentaGel SAM Harz mit *tert*-Butyl 3-pyrrolidinylcarbamate als Aminderivat 1 umgesetzt. Das nach der Acylierung mit 5-Chlorthiophencarbonsäure erhaltene Nitrobenzolderivat wurde von der festen Phase abgespalten und eingedampft. Dieses Rohprodukt wurde durch Reversed Phase HPLC mit einem Wasser/TFA/Acetorutril-Gradienten gereinigt.
¹H-NMR (400 MHz, CD₃OH): 2.07-2.17, m, 1 H; 2.39-2.49, m, 1 H; 3.21-3.40, m, 2 H; 3.45, dd, 1 H; 3.50-3.60, m, 1 H; 3.67, dd, 1 H; 3.76, dd, 1 H; 3.88-4.00, m, 2 H; 4.14 - 4.21, t, 1 H; 4.85 - 4.95, m, 1 H; 7.01, d, 1 H; 7.11, d, 1 H; 7.52, d, 1 H; 7.66, dd, 1 H; 7.93, d, 1 H.

### Beispiel 175

### N-({3-[3-amino-4-(1-piperidinyl)phenyl]-2-oxo-1,3-oxazolidin-5-yl}methyl)-5-chloro-2-thiophencarboxamid

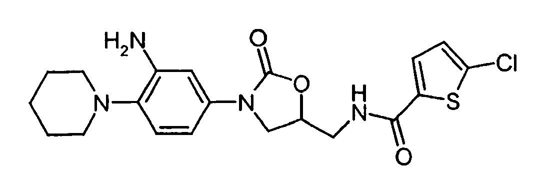

Analog der allgemeinen Arbeitsvorschrift zur Herstellung der Derivate B wurden 130 mg (32,5 µmol) TentaGel SAM Harz mit Piperidin als Aminderivat 1 umgesetzt. Das nach der Reduktion erhaltene Anilin wurde ohne weiteren Acylierungsschritt von der festen Phase abgespalten und eingedampft. Dieses Rohprodukt wurde durch Reversed Phase HPLC mit einem Wasser/TFA/Acetonitril-Gradienten gereinigt.
¹H-NMR (400 MHz, CD₃OH): 1.65-1.75, m, 2 H; 1.84-1.95, m, 4 H; 3.20-3.28, m, 4 H; 3.68, dd, 1 H; 3.73, dd, 1H; 3.90, dd, 1 H; 4.17, dd, 1 H; 4.80-4.90, m, 1 H; 7.00, d, 1 H; 7.05, dd, 1 H; 7.30-7.38, m, 2H; 7.50, d, 1 H.

### Beispiel 176

### N-({3-[3-(Acetylamino)-4-(1-pyrrolidinyl)phenyl]-2-oxo-1,3-oxazolidin-5-yl}-methyl)-5-chlor-2-thiophencarboxamid

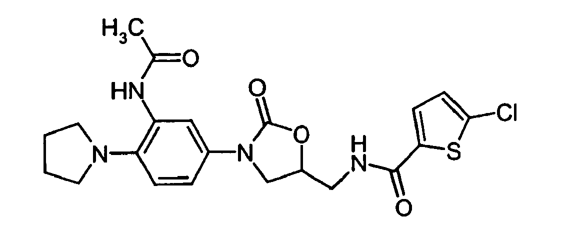

Analog der allgemeinen Arbeitsvorschrift zur Herstellung der Derivate B wurden 130 mg (32.5 µmol) TentaGel SAM Harz mit Pyrrolidin als Aminderivat 1 und Acetylchlorid als Säurederivat 1 umgesetzt. Das Rohprodukt wurde zwischen Ethylacetat und NaHCO₃-Lösung verteilt, die organische Phase wurde mit NaCl ausgesalzen, dekantiert und zur Trockene eingedampft. Dieses Rohprodukt wurde durch Vakuum-Flashchromatographie an Kieselgel (Dichlormethan/Ethylacetat, 1:1-0:1) gereinigt.
¹H-NMR (400 MHz, CD₃OH): 1.93 - 2.03, br, 4 H; 2.16, s, 3 H; 3.20-3.30, br, 4 H; 3.70, d, 2 H; 3.86, dd, 1H; 4.10, dd, 1 H; 4.14, dd, 1 H; 4.80-4.90, m, 1 H; 7.00, d, 1 H; 7.07, d, 1 H; 7.31, dd, 1 H; 7.51, d, 1 H; 7.60, d, 1 H.

Analog zu der allgemeinen Arbeitsvorschrift wurden die folgenden Verbindungen hergestellt.

| **Beispiel** | **Struktur** | **Ret.-Zeit** | **HPLC [%]** |
|---|---|---|---|
| 177 | 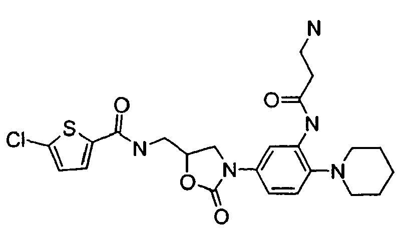 | 2,62 | 79,7 |
| 178 | 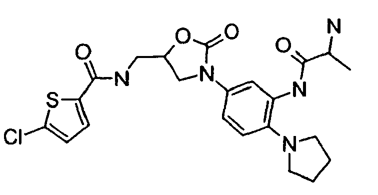 | 2,49 | 33,7 |
| 179 | 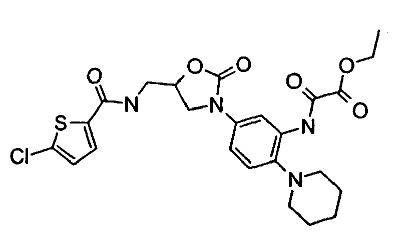 | 4,63 | 46,7 |
| 180 | 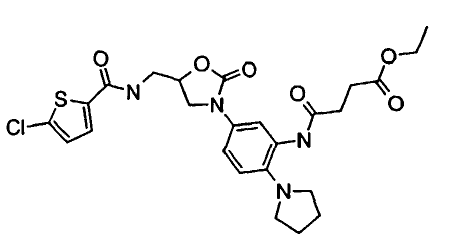 | 3,37 | 44,8 |
| 181 | 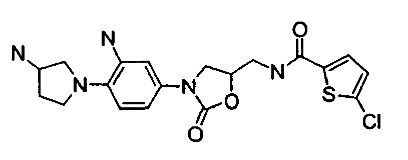 | 2,16 | 83 |
| 182 | 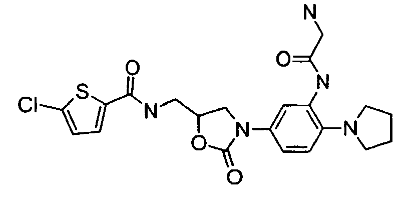 | 2,31 | 93,3 |
| 183 | 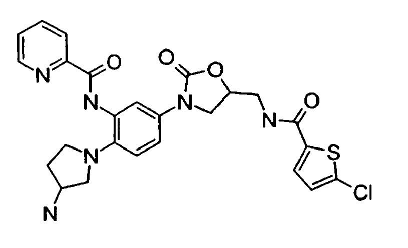 | 2,7 | 100 |
| 184 | 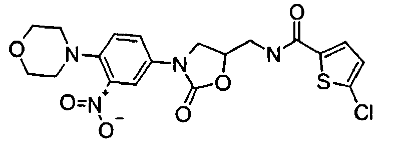 | 3,91 | 51 |
| 185 | 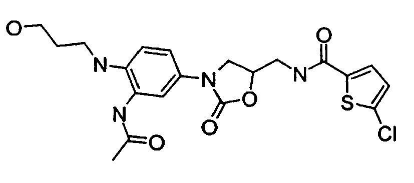 | 2,72 | 75,2 |
| 186 | 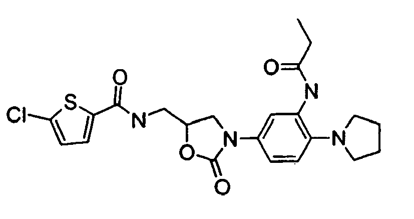 | 3,17 | 46 |
| 187 | 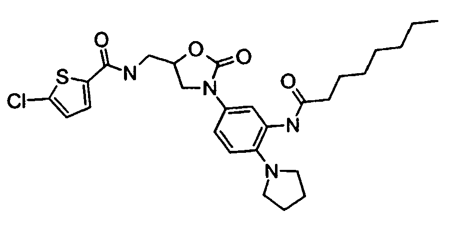 | 4,61 | 50,2 |
| 188 | 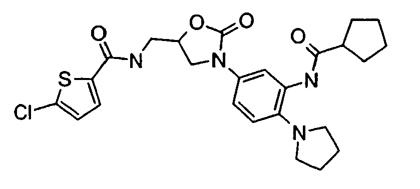 | 3,89 | 56,6 |
| 189 | 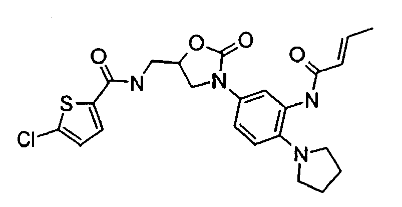 | 3,37 | 52,9 |
| 190 | 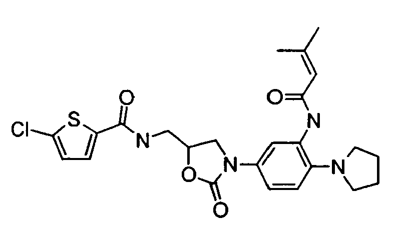 | 3,6 | 53,9 |
| 191 | 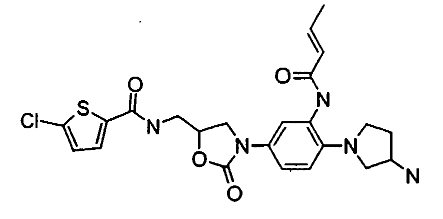 | 2,52 | 70,1 |
| 192 | 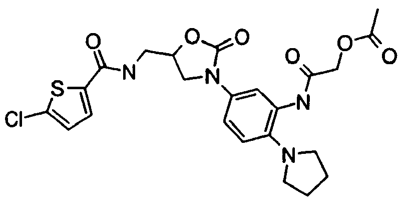 | 3,52 | 46,6 |
| 193 | 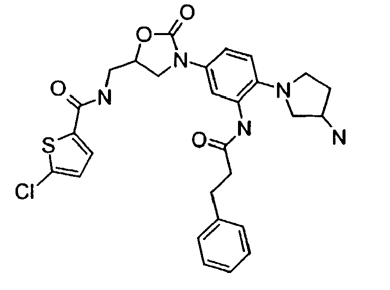 | 2,87 | 50,1 |
| 194 | 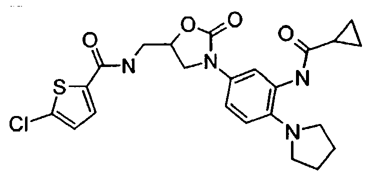 | 3,25 | 71,1 |
| 195 | 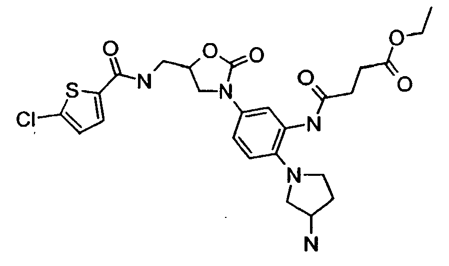 | 2,66 | 67 |
| 196 | 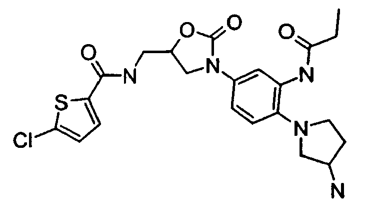 | 2,4 | 52,1 |
| 197 | 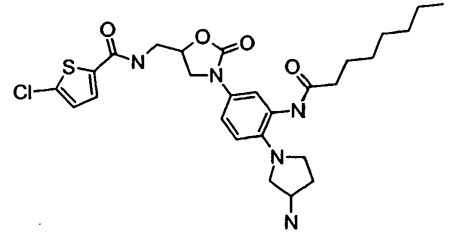 | 3,13 | 48,9 |
| 198 | 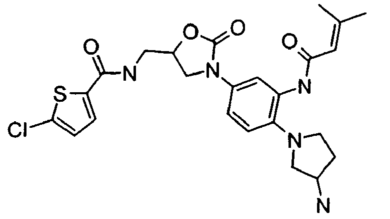 | 2,67 | 75,5 |
| 199 | 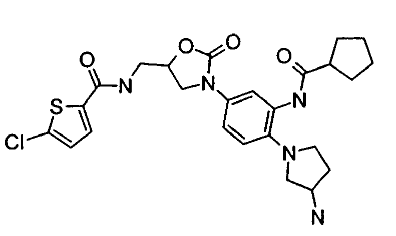 | 2,72 | 65,7 |
| 200 | 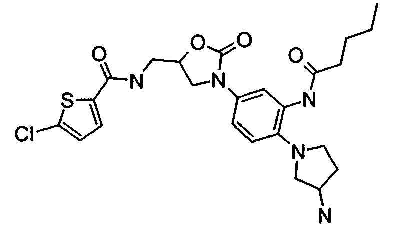 | 2,71 | 57,3 |
| 201 | 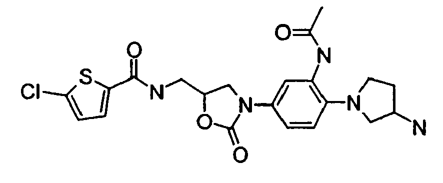 | 2,22 | 100 |
| 202 | 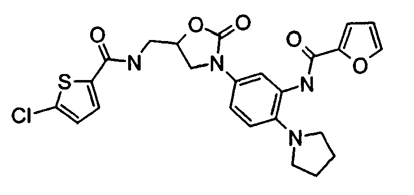 | 3,89 | 75,7 |
| 203 | 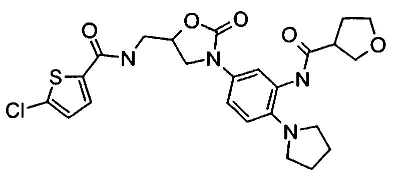 | 3,19 | 49,6 |
| 204 | 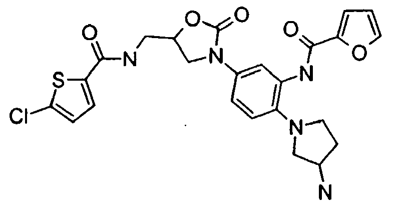 | 2,55 | 88,2 |
| 205 | 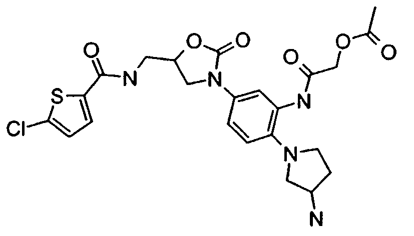 | 2,44 | 68,6 |
| 206 | 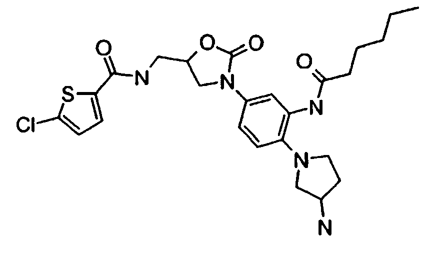 | 2,86 | 71,8 |
| 207 | 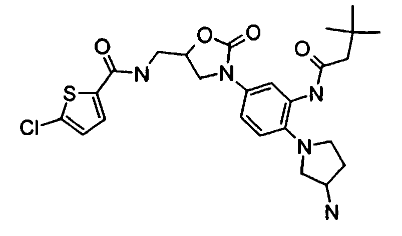 | 2,8 | 63,6 |
| 208 | 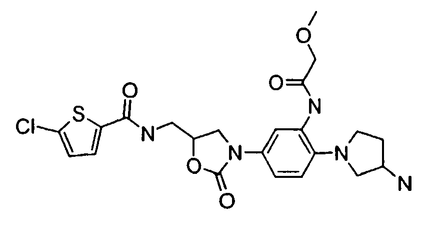 | 2,41 | 77 |
| 209 | 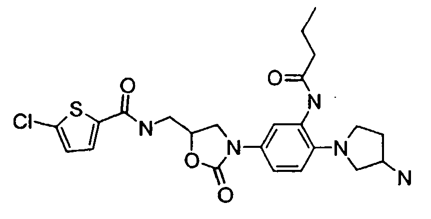 | 2,56 | 67,9 |
| 210 | 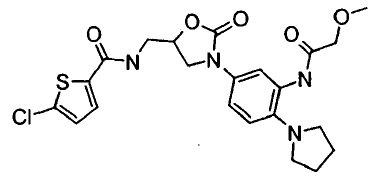 | 3,67 | 78,4 |
| 211 | 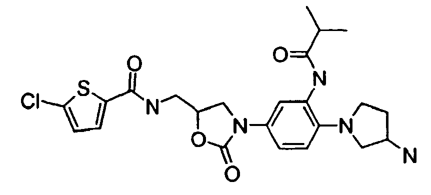 | 2,54 | 59,8 |
| 212 | 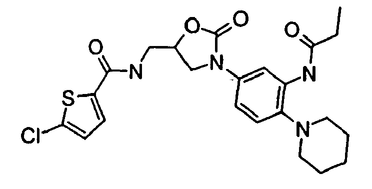 | 3,84 | 59,2 |
| 213 | 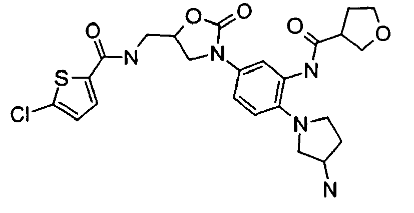 | 2,41 | 67,8 |
| 214 | 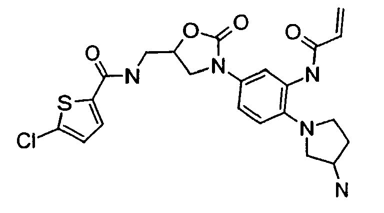 | 2,41 | 75,4 |
| 215 | 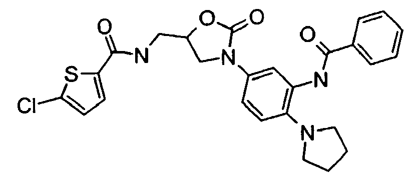 | 4,01 | 81,3 |
| 216 | 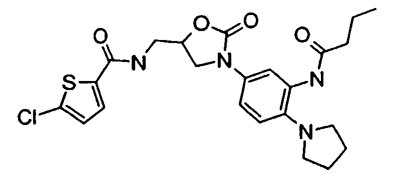 | 3,46 | 49,5 |
| 217 | 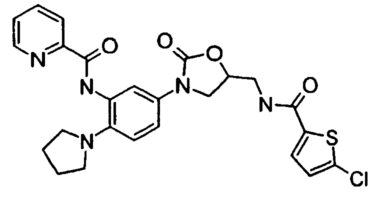 | 4,4 | 60,2 |
| 218 | 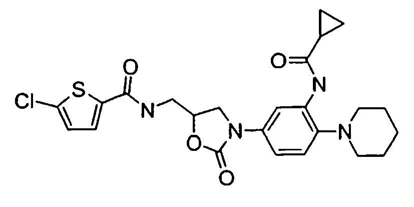 | 3,79 | 70,9 |
| 219 | 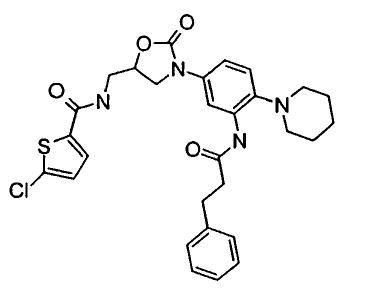 | 4,57 | 51,5 |
| 220 | 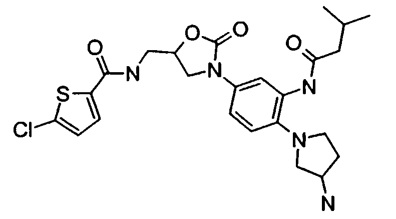 | 2,68 | 100 |
| 221 | 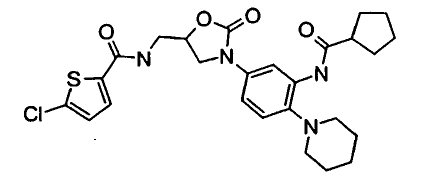 | 4,53 | 63,5 |
| 222 | 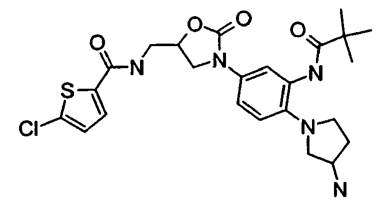 | 2,66 | 89,2 |
| 223 | 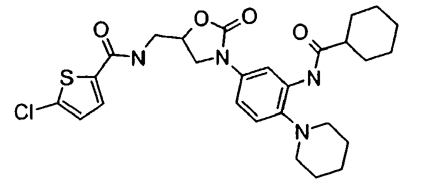 | 4,76 | 69,3 |
| 224 | 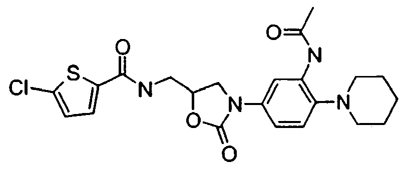 | 3,45 | 77,4 |
| 225 | 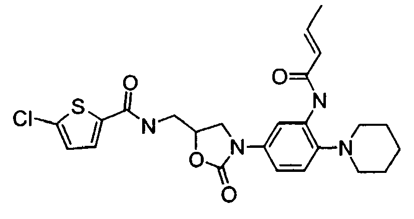 | 3,97 | 63,2 |
| 226 | 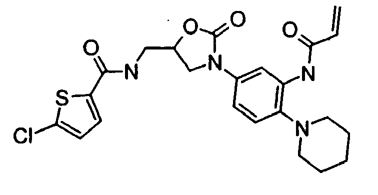 | 3,94 | 61,4 |
| 227 | 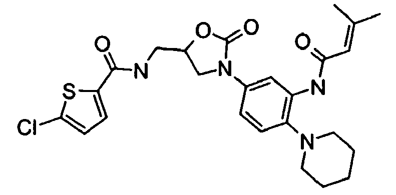 | 4,15 | 66,3 |
| 228 | 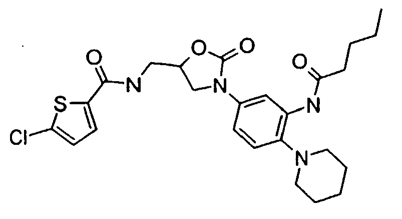 | 4,41 | 55,1 |
| 229 | 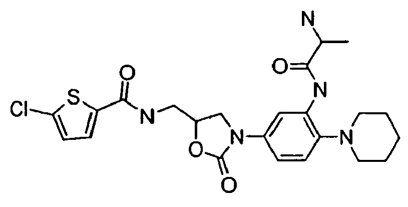 | 2,83 | 41,1 |
| 230 | 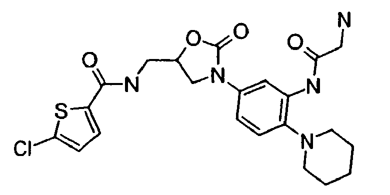 | 2,7 | 83 |
| 231 | 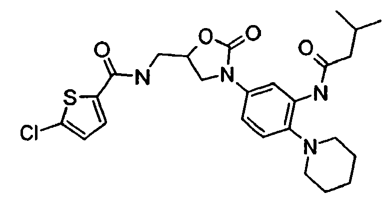 | 4,39 | 64,2 |
| 232 | 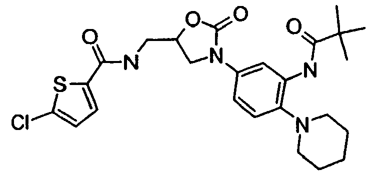 | 4,85 | 74,9 |
| 233 | 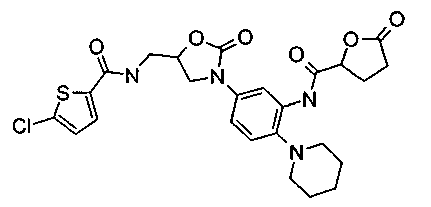 | 4,17 | 41 |
| 234 | 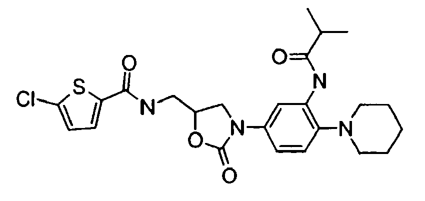 | 4,21 | 61,8 |
| 235 | 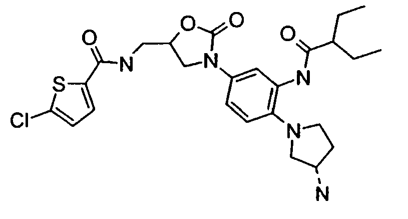 | 2,75 | 100 |
| 236 | 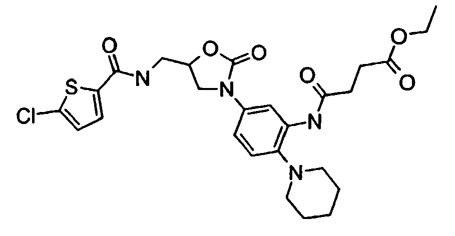 | 3,94 | 50 |
| 237 | 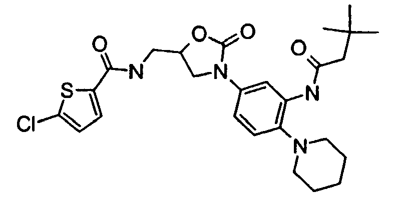 | 4,65 | 75,8 |
| 238 | 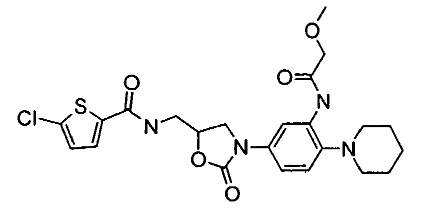 | 4,4 | 75,3 |
| 239 | 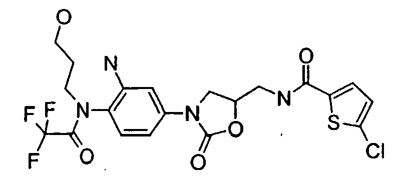 | 4,24 | 62,2 |
| 240 | 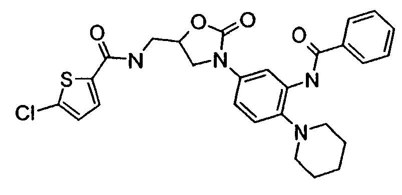 | 4,76 | 75,1 |
| 241 | 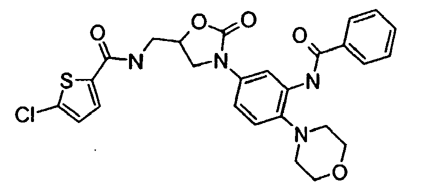 | 4,17 | 72,5 |
| 242 | 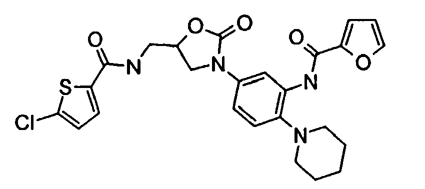 | 4,6 | 74,8 |
| 243 | 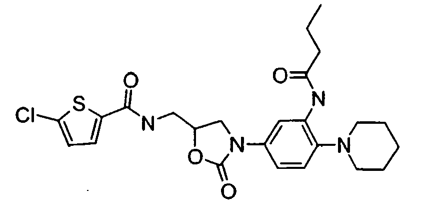 | 4,12 | 51,6 |
| 244 | 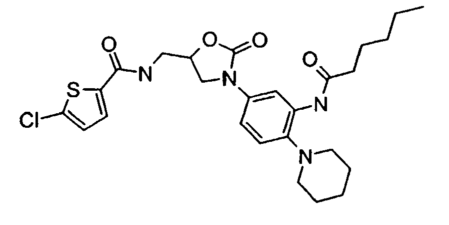 | 4,71 | 66,2 |
| 245 | 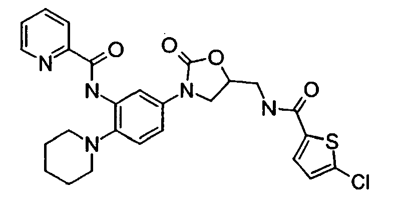 | 4,86 | 62 |
| 246 | 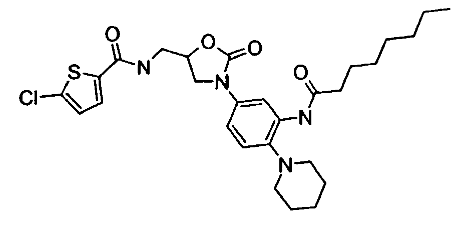 | 5,23 | 58,3 |
| 247 | 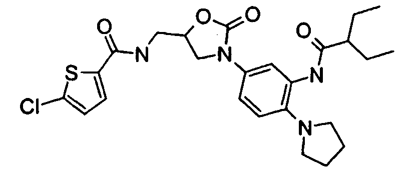 | 4,17 | 72,4 |
| 248 | 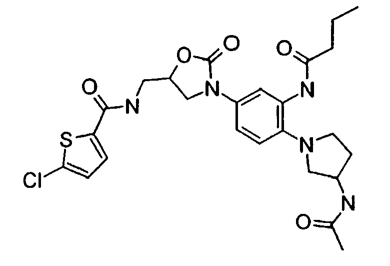 | 3,35 | 59,6 |
| 249 | 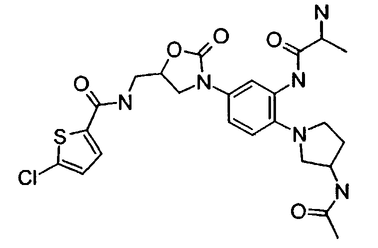 | 2,41 | 60,3 |
| 250 | 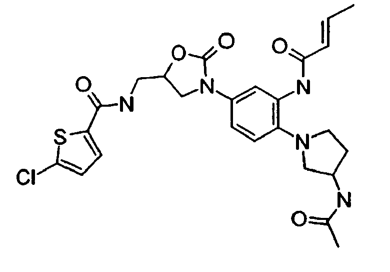 | 3,31 | 65,2 |
| 251 | 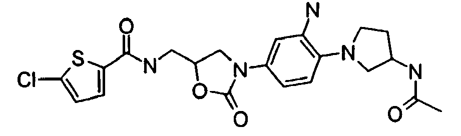 | 2,86 | 36,5 |
| 252 | 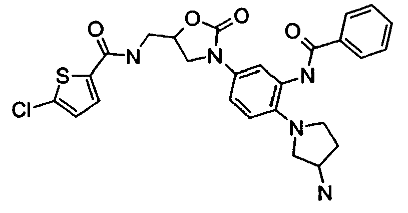 | 2,69 | 89,8 |
| 253 | 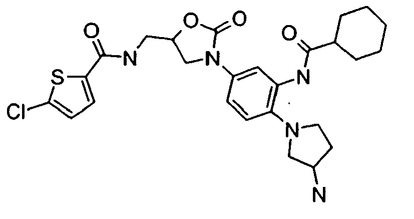 | 2,81 | 67,4 |
| 254 | 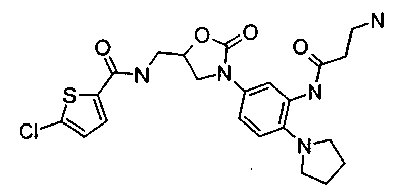 | 2,19 | 75,4 |

Alle Produkte der festphasenunterstützten Synthese wurden mittels LC-MS charakterisiert. Dazu wurde standardmäßig folgendes Trennsystem verwendet: HP 1100 mit UV-Detektor (208 - 400 nm), 40°C Ofentemperatur, Waters-Symmetry C18 Säule (50 mm x 2.1 mm, 3,5 µm). Laufmittel A: 99.9 % Acetonitril/0.1 % Ameisensäure, Laufmittel B: 99.9 % Wasser/0,1 % Ameisensäure; Gradient:

| Zeit | A:% | B:% | Fluss |
|---|---|---|---|
| 0,00 | 10,0 | 90,0 | 0,50 |
| 4,00 | 90,0 | 10,0 | 0,50 |
| 6,00 | 90,0 | 10,0 | 0,50 |
| 6,10 | 10,0 | 90,0 | 1,00 |
| 7,50 | 10,0 | 90,0 | 0,50 |

Der Nachweis der Substanzen erfolgte mittels eines Micromass Quattro LCZ MS, Ionisierung: ESI positiv/negativ.

Bei den oben aufgeführten Strukturen, die den oder die Reste 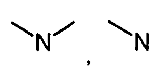 oder -O beinhalten, ist stets eine 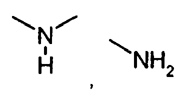 oder -OH-Funktion gemeint.

## Patentansprüche

1. Kombinationen enthaltend
A) mindestens eine Verbindung der Formel (I) 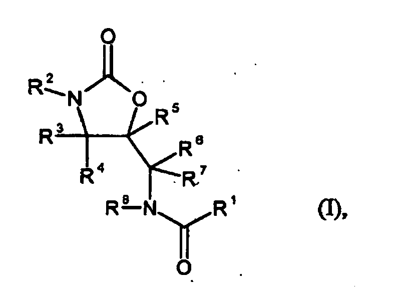 in welcher
R¹ für 2-Thiophen, steht, das in der 5-Position substituiert ist durch einen Rest aus der Gruppen Chlor, Brom, Methyl oder Trifluormethyl,
R² für D-A- steht:
wobei:
der Rest "A" für Phenylen steht;
der Rest "D" für einen gesättigten 5- oder 6-gliedrigen Hetero- cyclus steht,
der über ein Stickstoffatom mit "A" verknüpft ist,
der in direkter Nachbarschaft zum verknüpfenden Stickstoff atom eine Carbonylgruppe besitzt und
in dem ein Ring-Kohlenstoffglied durch ein Heteroatom aus der Reihe S, N und O ersetzt sein kann;
wobei
die zuvor definierten Gruppe "A" in der meta-Position bezüg- lich der Verknüpfung zum Oxazolidinon gegebenenfalls ein- oder zweifach substituiert sein kann mit einem Rest aus der von Fluor, Chlor, Nitro, Amino, Trifluormethyl, Methyl oder Cyano,
R³, R⁴, R⁵, R⁶, R⁷ und R⁸ für Wasserstoff stehen,
deren pharmazeutisch verträglichen Salze, Hydrate oder deren Mischungen
und
B) Cerivastatin (Rivastatin, Baycol), Lovastatin (Mevacor), Simvastatin (Zocor), Pravastatin (Pravachol), Fluvastatin (Lescol), Atorvastatin (Lipitor), Captopril, Lisinopril, Enalapril, Ramipril, Cilazapril, Benazepril, Fosinopril, Quinapril, Perindopril, Embusartan, Losartan, Valsartan, Irbesartan, Candesartan, Eprosartan, Temisartan, Carvedilol, Alprenolol, Bisoprolol, Acebutolol, Atenolol, Betaxolol, Carteolol, Metoprolol, Nadolol, Penbutolol, Pindolol, Propanolol, Timolol, Prazosin, Bunazosin, Doxazosin, Terazosin, Hydrochlorothiazid, Furosemid, Bumetanid, Piretanid, Torasemid, Amilorid, Dihydralazin, Verapamil, Diltiazem, Nifedipin (Adalat), Nitrendipin (Bayotensin), Isosorbid-5-mononitrat, Isosorbid-dinitrat, Glycerolbinitrat, Gewebsplasminogen-Aktivator (t-PA), Streptokinase, Reteplase, Urokinase, Heparin (UFH), Tinzaparin, Certoparin, Parnaparin, Nadioparin, Ardeparin, Enoxaparin, Reviparin, Dalteparin, Hirudin, Aspirin, Ticlopidin (Ticlid), Clopidogrel (Plavix), Abciximab, Eptifibatide, Tirofiban, Lamifiban oder Lefradafiban

2. Kombinationen nach Anspruch 1, **dadurch gekennzeichnet, dass** die Verbindung A) 5-Chloro-*N*-({(5S)-2-oxo-3-[4-(3-oxo-4-morpholinyl)phenyl]-1,3-oxazolidin-5-yl}methyl)-2-thiophencarboxamid der Formel 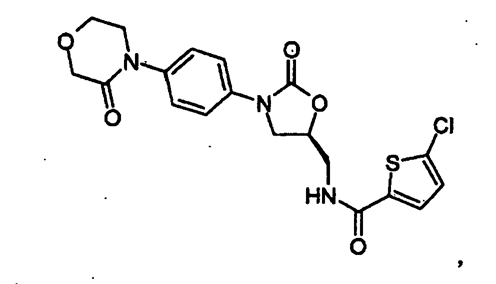 seine pharmazeutisch verträglichen Salze, Hydrate oder deren Mischungen ist.

3. Verfahren zur Herstellung der Kombinationen nach Anspruch 1 oder 2, **dadurch gekennzeichnet, dass** man Komponente A) und Komponete B) in geeigneter Weise kombiniert oder herrichtet.

4. Kombinationen nach Anspruch 1 oder 2 zur Prophylaxe und/oder Behandlung von Erkrankungen.

5. Arzneimittel, enthaltend mindestens eine Kombination gemäß Anspruch 1 oder 2 und gegebenenfalls weitere pharmazeutische Wirkstoffe

6. Arzneimittel enthaltend mindestens eine Kombination gemäß Anspruch 1 oder 2 sowie ein oder mehrere pharmakologisch unbedenkliche Hilfs-und/oder Trügerstoffe

7. Verwendung von Kombinationen des Anspruchs 1 oder 2 zur Herstellung eines Arzneimittel zur Prophylaxe und/oder Behandlung von thromboembolischen Erkrankungen.

8. Verwendung von Kombinationen des Anspruch 1 oder 2 zur Herstellung eines Arzneimittels zur Prophylaxe und/oder Behandlung von Herzinfarkt, Angina Pectoris (eingeschlossen instabile Angina), plötzlichem Herztod, Reokklusionen und Restenosen nach einer Angioplastie oder aortokoronarem Bypass, Hirnschlag, transitorischen ischämischen Attacken, peripheren arteriellen Verschlusskrankheiten, Lungenembolien oder tiefen venösen Thrombosen.

## Claims

1. Combinations comprising
A) at least one compound of the formula (I) 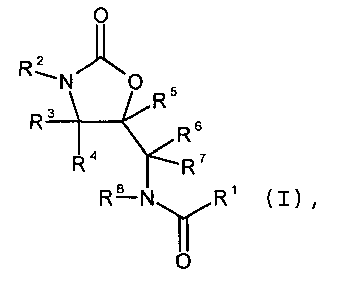 in which
R¹ is 2-thiophene which is substituted in position 5 by a radical from the group of chlorine, bromine, methyl or trifluoromethyl,
R² is D-A-:
where:
the radical "A" is phenylene;
the radical "D" is a saturated 5- or 6-membered heterocycle which is linked via a nitrogen atom to "A",
which has a carbonyl group in direct vicinity to the linking nitrogen atom, and in which a ring carbon member may be replaced by a heteroatom from the series S, N and O;
where
the group "A" defined above may optionally be substituted once or twice in the meta position relative to the linkage to the oxazolidinone by a radical from the group of fluorine, chlorine, nitro, amino, trifluoromethyl, methyl or cyano,
R³, R⁴, R⁵, R⁶, R⁷ and R⁸ are hydrogen,
the pharmaceutically acceptable salts, hydrates thereof or mixtures thereof
and
B) cerivastatin (Rivastatin, Baycol), lovastatin (Mevacor), simvastatin (Zocor), pravastatin (Pravachol), fluvastatin (Lescol), atorvastatin (Lipitor), captopril, lisinopril, enalapril, ramipril, cilazapril, benazepril, fosinopril, quinapril, perindopril, embusartan, losartan, valsartan, irbesartan, candesartan, eprosartan, temisartan, carvedilol, alprenolol, bisoprolol, acebutolol, atenolol, betaxolol, carteolol, metoprolol, nadolol, penbutolol, pindolol, propanolol, timolol, prazosin, bunazosin, doxazosin, terazosin, hydrochlorothiazide, furosemide, bumetanide, piretanide, torasemide, amiloride, dihydralazine, verapamil, diltiazem, nifedipine (Adalat), nitrendipine (Bayotensin), isosorbide 5-mononitrate, isosorbide dinitrate, glycerol trinitrate, tissue plasminogen activator (t-PA), streptokinase, reteplase, urokinase, heparin (UFH), tinzaparin, certoparin, parnaparin, nadroparin, ardeparin, enoxaparin, reviparin, dalteparin, hirudin, aspirin, ticlopidin (Ticlid), clopidogrel (Plavix), abciximab, eptifibatide, tirofiban, lamifiban or lefradafiban.

2. Combinations according to Claim 1, **characterized in that** the compound A) is 5-chloro-*N*-({(5*S*)-2-oxo-3-[4-(3-oxo-4-morpholinyl)phenyl]-1,3-oxazolidin-5-yl}methyl)-2-thiophenecarboxamide of the formula 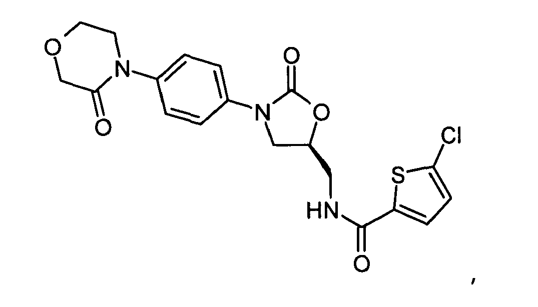 its pharmaceutically acceptable salts, hydrates or mixtures thereof.

3. Process for producing the combinations according to Claim 1 or 2, **characterized in that** component A) and component B) are combined or prepared in a suitable way.

4. Combinations according to Claim 1 or 2 for the prophylaxis and/or treatment of disorders.

5. Medicament comprising at least one combination according to Claim 1 or 2 and, where appropriate, further active pharmaceutical ingredients.

6. Medicament comprising at least one combination according to Claim 1 or 2 and one or more pharmacologically suitable excipients and/or carriers.

7. Use of combinations of Claim 1 or 2 in the manufacture of a medicament for the prophylaxis and/or treatment of thromboembolic disorders.

8. Use of combinations of Claim 1 or 2 in the manufacture of a medicament for the prophylaxis and/or treatment of myocardial infarction, angina pectoris (including unstable angina), sudden heart death, reocclusions and restenoses after angioplasty or aortocoronary bypass, stroke, transient ischemic attacks, peripheral arterial occlusive diseases, pulmonary embolisms or deep venous thromboses.

## Revendications

1. Combinaisons contenant
A) au moins un composé de formule (I) 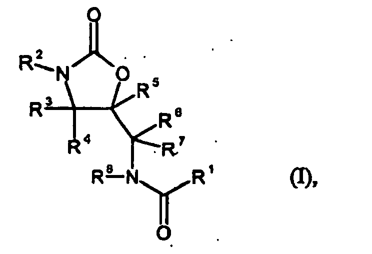 dans laquelle
R¹ représente un reste 2-thiophène, qui est substitué en position 5 par un reste du groupe chloro, bromo, méthyle ou trifluorométhyle,
R² représente un groupe D-A-
dans lequel
le reste "A" est un reste phénylène,
le reste "D" représente un hétérocycle pentagonal ou hexagonal saturé,
qui est lié à "A" par l'intermédiaire d'un atome d'azote,
qui porte au voisinage immédiat de l'atome d'azote de liaison un groupe carbonyle et
dans lequel un atome de carbone chaînon du noyau peut être remplacé par un hétéroatome de la série S, N et O ; le groupe "A" défini ci-dessus pouvant éventuellement être substitué une ou deux fois, en position méta par rapport à la liaison à l'oxazolidinone, avec un reste du groupe fluoro, chloro, nitro, amino, trifluorométhyle, méthyle ou cyano,
R³, R⁴, R⁵, R⁶, R⁷ et R⁸ représentent l'hydrogène,
ses sels, hydrates ou leurs mélanges pharmaceutiquement acceptables
et
B) de la cérivastatine (rivastatine, baycol), de la lovastatine (mévacor), de la simvastatine (zocor), de la pravastatine (Pravachol), de la fluvastatine (lescol), de l'atorvastatine (lipitor), du captopril, du lisinopril, de l'énalapril, du ramipril, du cilazapril, du benazépril, du fosinopril, du quinapril, du périndopril, de l'embusartan, du losartan, du valsartan, de l'irbesartan, du candésartan, de l'éprosartan, du témisartan, du carvédilol, de l'alprénolol, du bisoprolol, de l'acébutolol, de l'aténolol, du bétaxolol, du cartéolol, du métoprolol, du nadolol, du pentabutolol, du pindolol, du propanolol, du timolol, de la prazosine, de la bunazosine, de la doxazosine, de la térazosine, l'hydrochlorothiazide, du furoméside, du bumétanide, du pirétanide, du torasémide, de l'amiloride, de la dihydralazine, du vérapamil, du diltiazem, de la nifédipine (adalate), de la nitrendipine (bayotensine), du 5-mononitrate d'isosorbide, du dinitrate d'isosorbide, du trinitrate de glycérol, de l'activateur de plasminogène tissulaire (t-PA), de la streptokinase, de la rétéplase, de l'urokinase, de l'héparine (UFH), de la tinzaparine, de la certoparine, de la parnaparine, de la nadroparine, de l'ardéparine, de l'énoxaparine, de la réviparine, de la daltéparine, de l'hirudine, de l'aspirine, de la ticlopidine (ticlide), du clopidogrel (plavix), de l'abciximab, de l'eptifibatide, du tirofiban, du lamifiban ou du léfradafiban.

2. Combinaisons suivant la revendication 1, **caractérisées en ce que** le composé A) est le 5-chloro-*N*({(5S)2-oxo-3-[4-(3-oxo-4-morpholinyl)phényl]-1,3-oxazolidine-5-yl}méthyl)-2-thiophènecarboxamide de formule 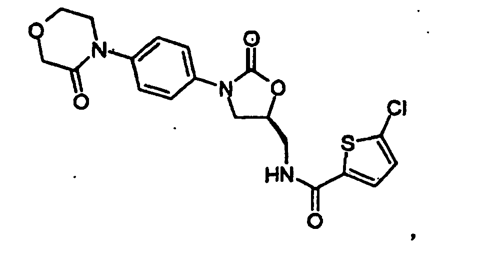 ses sels, hydrates ou leurs mélanges pharmaceutiquement acceptables.

3. Procédé de préparation des combinaisons suivant la revendication 1 ou 2, **caractérisé en ce qu'**on combine ou apprête le composant A) et le composant B) d'une manière convenable.

4. Combinaisons suivant la revendication 1 ou 2, destinées à la prophylaxie et/ou au traitement de maladies.

5. Médicament, contenant au moins une combinaison suivant la revendication 1 ou 2 et, le cas échéant, d'autres substances pharmaceutiques actives.

6. Médicament, contenant au moins une combinaison suivant la revendication 1 ou 2 ainsi qu'une ou plusieurs substances auxiliaires et/ou un ou plusieurs supports pharmacologiquement acceptables.

7. Utilisation de combinaisons suivant la revendication 1 ou 2 pour la préparation d'un médicament destiné à la prophylaxie et/ou au traitement de maladies thromboemboliques.

8. Utilisation de combinaisons suivant la revendication 1 ou 2 pour la préparation d'un médicament destiné à la prophylaxie et/ou au traitement de l'infarctus du myocarde, de l'angine de poitrine (y compris l'angor instable), de la mort soudaine par arrêt cardiaque, de réocclusions et de resténoses à la suite d'une angioplastie ou d'un pontage aorto-coronarien, d'une apoplexie cérébrale, d'attaques ischémiques transitoires, d'artériopathies périphériques oblitérantes, d'embolies pulmonaires et de thromboses veineuses profondes.
